# EUROPÄISCHE PATENTSCHRIFT

(11) **EP 1 928 905 B1**
(45) Veröffentlichungstag und Bekanntmachung des Hinweises auf die Patenterteilung: **15.04.2015**
(21) Anmeldenummer: 06805972.4
(22) Anmeldetag: 29.09.2006
(51) Int. Cl.: C07K 14/475, C07K 14/71, C07K 16/28, A61K 39/395, A61P 25/00

(54) **BINDUNGSDOMÄNEN VON PROTEINEN DER REPULSIVE GUIDANCE MOLECULE (RGM) PROTEINFAMILIE UND FUNKTIONALE FRAGMENTE DAVON SOWIE DEREN VERWENDUNG**
BINDING DOMAINS OF PROTEINS OF THE REPULSIVE GUIDANCE MOLECULE (RGM) PROTEIN FAMILY AND FUNCTIONAL FRAGMENTS THEREOF, AND THEIR USE
DOMAINES DE LIAISON DE PROTEINES DE LA FAMILLE PROTEINIQUE DES MOLECULES DE GUIDAGE REPULSIF (RGM), FRAGMENTS FONCTIONNELS DE CES DOMAINES ET LEUR UTILISATION

(30) Priorität: 30.09.2005 EP 05021451; 01.10.2005 US 722565 P
(43) Veröffentlichungstag der Anmeldung: 11.06.2008
(73) Patentinhaber: AbbVie Deutschland GmbH & Co KG, 65205 Wiesbaden (DE)
(72) Erfinder: MÜLLER, Bernhard, K., 67435 Neustadt (DE); SCHAFFAR, Gregor, 68165 Mannheim (DE); MUELLER, Reinhold, 67105 Schifferstadt (DE)
(74) Vertreter: Reitstötter Kinzebach
(86) Internationale Anmeldenummer: PCT/EP2006/009497
(87) Internationale Veröffentlichungsnummer: WO 2007/039256

(56) Entgegenhaltungen:
- EP-A- 1 347 046
- WO-A-00/73801
- WO-A-02/051438
- WO-A-03/031462
- WO-A-03/089608
- WO-A-2004/003150
- WO-A-2004/092405
- MATSUNAGA EIJI ET AL: "RGM and its receptor neogenin regulate neuronal survival." NATURE CELL BIOLOGY AUG 2004, Bd. 6, Nr. 8, August 2004 (2004-08), Seiten 749-755, XP002424997 ISSN: 1465-7392
- MATSUNAGA EIJI ET AL: "Repulsive guidance molecule/neogenin: a novel ligand-receptor system playing multiple roles in neural development." DEVELOPMENT, GROWTH & DIFFERENTIATION DEC 2004, Bd. 46, Nr. 6, Dezember 2004 (2004-12), Seiten 481-486, XP002424998 ISSN: 0012-1592
- RAJAGOPALAN SRIKANTH ET AL: "Neogenin mediates the action of repulsive guidance molecule." NATURE CELL BIOLOGY AUG 2004, Bd. 6, Nr. 8, August 2004 (2004-08), Seiten 756-762, XP002424999 ISSN: 1465-7392 in der Anmeldung erwähnt
- OLDEKAMP J ET AL: "Expression pattern of the repulsive guidance molecules RGM A, B and C during mouse development" GENE EXPRESSION PATTERNS, ELSEVIER, Bd. 4, Nr. 3, Mai 2004 (2004-05), Seiten 283-288, XP002345933 ISSN: 1567-133X

### SEQUENCE LISTING

<110> Abbott GmbH & Co. KG
<120> RGM Bindungsdomänen
<130> M/46268-PCT
<160> 34
<170> PatentIn version 3.3
<210> 1
   <211> 1353
   <212> DNA
   <213> Homo sapiens
<220>
   <221> CDS
   <222> (1)..(1353)
<400> 1
   atg cag ccg cca agg gag agg cta gtg gta aca ggc cga gct gga tgg       48
   Met Gln Pro Pro Arg Glu Arg Leu Val Val Thr Gly Arg Ala Gly Trp
   1               5                   10                  15
   atg ggt atg ggg aga ggg gca gga cgt tca gcc ctg gga ttc tgg ccg       96
   Met Gly Met Gly Arg Gly Ala Gly Arg Ser Ala Leu Gly Phe Trp Pro
               20                  25                  30
   acc ctc gcc ttc ctt ctc tgc agc ttc ccc gca gcc acc tcc ccg tgc      144
   Thr Leu Ala Phe Leu Leu Cys Ser Phe Pro Ala Ala Thr Ser Pro Cys
           35                  40                  45
   aag atc ctc aag tgc aac tct gag ttc tgg agc gcc acg tcg ggc agc      192
   Lys Ile Leu Lys Cys Asn Ser Glu Phe Trp Ser Ala Thr Ser Gly Ser
       50                  55                  60
   cac gcc cca gcc tca gac gac acc ccc gag ttc tgt gca gcc ttg cgc      240
   His Ala Pro Ala Ser Asp Asp Thr Pro Glu Phe Cys Ala Ala Leu Arg
   65                  70                  75                  80
   agc tac gcc ctg tgc acg cgg cgg acg gcc cgc acc tgc cgg ggt gac      288
   Ser Tyr Ala Leu Cys Thr Arg Arg Thr Ala Arg Thr Cys Arg Gly Asp
                   85                  90                  95
   ctg gcc tac cac tcg gcc gtc cat ggc ata gag gac ctc atg agc cag      336
   Leu Ala Tyr His Ser Ala Val His Gly Ile Glu Asp Leu Met Ser Gln
               100                 105                 110
   cac aac tgc tcc aag gat ggc ccc acc tcg cag cca cgc ctg cgc acg      384
   His Asn Cys Ser Lys Asp Gly Pro Thr Ser Gln Pro Arg Leu Arg Thr
           115                 120                 125
   ctc cca ccg gcc gga gac agc cag gag cgc tcg gac agc ccc gag atc      432
   Leu Pro Pro Ala Gly Asp Ser Gln Glu Arg Ser Asp Ser Pro Glu Ile
       130                 135                 140
   tgc cat tac gag aag agc ttt cac aag cac tcg gcc acc ccc aac tac      480
   Cys His Tyr Glu Lys Ser Phe His Lys His Ser Ala Thr Pro Asn Tyr
   145                 150                 155                 160
   acg cac tgt ggc ctc ttc ggg gac cca cac ctc agg act ttc acc gac      528
   Thr His Cys Gly Leu Phe Gly Asp Pro His Leu Arg Thr Phe Thr Asp
                   165                 170                 175
   cgc ttc cag acc tgc aag gtg cag ggc gcc tgg ccg ctc atc gac aat      576
   Arg Phe Gln Thr Cys Lys Val Gln Gly Ala Trp Pro Leu Ile Asp Asn
               180                 185                 190
   aat tac ctg aac gtg cag gcc acc aac acg cct gtg ctg ccc ggc tca      624
   Asn Tyr Leu Asn Val Gln Ala Thr Asn Thr Pro Val Leu Pro Gly Ser
           195                 200                 205
   gcg gcc act gcc acc agc aag ctc acc atc atc ttc aag aac ttc cag      672
   Ala Ala Thr Ala Thr Ser Lys Leu Thr Ile Ile Phe Lys Asn Phe Gln
       210                 215                 220
   gag tgt gtg gac cag aag gtg tac cag gct gag atg gac gag ctc ccg      720
   Glu Cys Val Asp Gln Lys Val Tyr Gln Ala Glu Met Asp Glu Leu Pro
   225                 230                 235                 240
   gcc gcc ttc gtg gat ggc tct aag aac ggt ggg gac aag cac ggg gcc      768
   Ala Ala Phe Val Asp Gly Ser Lys Asn Gly Gly Asp Lys His Gly Ala
                   245                 250                 255
   aac agc ctg aag atc act gag aag gtg tca ggc cag cac gtg gag atc      816
   Asn Ser Leu Lys Ile Thr Glu Lys Val Ser Gly Gln His Val Glu Ile
               260                 265                 270
   cag gcc aag tac atc ggc acc acc atc gtg gtg cgc cag gtg ggc cgc      864
   Gln Ala Lys Tyr Ile Gly Thr Thr Ile Val Val Arg Gln Val Gly Arg
           275                 280                 285
   tac ctg acc ttt gcc gtc cgc atg cca gag gaa gtg gtc aat gct gtg      912
   Tyr Leu Thr Phe Ala Val Arg Met Pro Glu Glu Val Val Asn Ala Val
       290                 295                 300
   gag gac tgg gac agc cag ggt ctc tac ctc tgc ctg cgg ggc tgc ccc      960
   Glu Asp Trp Asp Ser Gln Gly Leu Tyr Leu Cys Leu Arg Gly Cys Pro
   305                 310                 315                 320
   ctc aac cag cag atc gac ttc cag gcc ttc cac acc aat gct gag ggc      1008
   Leu Asn Gln Gln Ile Asp Phe Gln Ala Phe His Thr Asn Ala Glu Gly
                   325                 330                 335
   acc ggt gcc cgc agg ctg gca gcc gcc agc cct gca ccc aca gcc ccc      1056
   Thr Gly Ala Arg Arg Leu Ala Ala Ala Ser Pro Ala Pro Thr Ala Pro
               340                 345                 350
   gag acc ttc cca tac gag aca gcc gtg gcc aag tgc aag gag aag ctg      1104
   Glu Thr Phe Pro Tyr Glu Thr Ala Val Ala Lys Cys Lys Glu Lys Leu
           355                 360                 365
   ccg gtg gag gac ctg tac tac cag gcc tgc gtc ttc gac ctc ctc acc      1152
   Pro Val Glu Asp Leu Tyr Tyr Gln Ala Cys Val Phe Asp Leu Leu Thr
       370                 375                 380
   acg ggc gac gtg aac ttc aca ctg gcc gcc tac tac gcg ttg gag gat      1200
   Thr Gly Asp Val Asn Phe Thr Leu Ala Ala Tyr Tyr Ala Leu Glu Asp
   385                 390                 395                 400
   gtc aag atg ctc cac tcc aac aaa gac aaa ctg cac ctg tat gag agg      1248
   Val Lys Met Leu His Ser Asn Lys Asp Lys Leu His Leu Tyr Glu Arg
                   405                 410                 415
   act cgg gac ctg cca ggc agg gcg gct gcg ggg ctg ccc ctg gcc ccc      1296
   Thr Arg Asp Leu Pro Gly Arg Ala Ala Ala Gly Leu Pro Leu Ala Pro
               420                 425                 430
   cgg ccc ctc ctg ggc gcc ctc gtc ccg ctc ctg gcc ctg ctc cct gtg      1344
   Arg Pro Leu Leu Gly Ala Leu Val Pro Leu Leu Ala Leu Leu Pro Val
           435                 440                 445
   ttc tgc tag                                                          1353
   Phe Cys
       450
<210> 2
   <211> 450
   <212> PRT
   <213> Homo sapiens
<400> 2
   Met Gln Pro Pro Arg Glu Arg Leu Val Val Thr Gly Arg Ala Gly Trp
   1               5                   10                  15
   Met Gly Met Gly Arg Gly Ala Gly Arg Ser Ala Leu Gly Phe Trp Pro
   20 25 30
   Thr Leu Ala Phe Leu Leu Cys Ser Phe Pro Ala Ala Thr Ser Pro Cys
   35 40 45
   Lys Ile Leu Lys Cys Asn Ser Glu Phe Trp Ser Ala Thr Ser Gly Ser
   50 55 60
   His Ala Pro Ala Ser Asp Asp Thr Pro Glu Phe Cys Ala Ala Leu Arg
   65 70 75 80
   Ser Tyr Ala Leu Cys Thr Arg Arg Thr Ala Arg Thr Cys Arg Gly Asp
   85 90 95
   Leu Ala Tyr His Ser Ala Val His Gly Ile Glu Asp Leu Met Ser Gln
   100 105 110
   His Asn Cys Ser Lys Asp Gly Pro Thr Ser Gln Pro Arg Leu Arg Thr
   115 120 125
   Leu Pro Pro Ala Gly Asp Ser Gln Glu Arg Ser Asp Ser Pro Glu Ile
   130 135 140
   Cys His Tyr Glu Lys Ser Phe His Lys His Ser Ala Thr Pro Asn Tyr
   145 150 155 160
   Thr His Cys Gly Leu Phe Gly Asp Pro His Leu Arg Thr Phe Thr Asp
   165 170 175
   Arg Phe Gln Thr Cys Lys Val Gln Gly Ala Trp Pro Leu Ile Asp Asn
   180 185 190
   Asn Tyr Leu Asn Val Gln Ala Thr Asn Thr Pro Val Leu Pro Gly Ser
   195 200 205
   Ala Ala Thr Ala Thr Ser Lys Leu Thr Ile Ile Phe Lys Asn Phe Gln
   210 215 220
   Glu Cys Val Asp Gln Lys Val Tyr Gln Ala Glu Met Asp Glu Leu Pro
   225 230 235 240
   Ala Ala Phe Val Asp Gly Ser Lys Asn Gly Gly Asp Lys His Gly Ala
   245 250 255
   Asn Ser Leu Lys Ile Thr Glu Lys Val Ser Gly Gln His Val Glu Ile
   260 265 270
   Gln Ala Lys Tyr Ile Gly Thr Thr Ile Val Val Arg Gln Val Gly Arg
   275 280 285
   Tyr Leu Thr Phe Ala Val Arg Met Pro Glu Glu Val Val Asn Ala Val
   290 295 300
   Glu Asp Trp Asp Ser Gln Gly Leu Tyr Leu Cys Leu Arg Gly Cys Pro
   305 310 315 320
   Leu Asn Gln Gln Ile Asp Phe Gln Ala Phe His Thr Asn Ala Glu Gly
   325 330 335
   Thr Gly Ala Arg Arg Leu Ala Ala Ala Ser Pro Ala Pro Thr Ala Pro
   340 345 350
   Glu Thr Phe Pro Tyr Glu Thr Ala Val Ala Lys Cys Lys Glu Lys Leu
   355 360 365
   Pro Val Glu Asp Leu Tyr Tyr Gln Ala Cys Val Phe Asp Leu Leu Thr
   370 375 380
   Thr Gly Asp Val Asn Phe Thr Leu Ala Ala Tyr Tyr Ala Leu Glu Asp
   385 390 395 400
   Val Lys Met Leu His Ser Asn Lys Asp Lys Leu His Leu Tyr Glu Arg
   405 410 415
   Thr Arg Asp Leu Pro Gly Arg Ala Ala Ala Gly Leu Pro Leu Ala Pro
               420                 425                 430
   Arg Pro Leu Leu Gly Ala Leu Val Pro Leu Leu Ala Leu Leu Pro Val
           435                 440                 445
   Phe Cys
       450
<210> 3
   <211> 1437
   <212> DNA
   <213> Homo sapiens
<220>
   <221> CDS
   <222> (1)..(1437)
<400> 3
   atg ata agg aag aag agg aag cga agc gcg ccc ccc ggc cca tgc cgc       48
   Met Ile Arg Lys Lys Arg Lys Arg Ser Ala Pro Pro Gly Pro Cys Arg
   1               5                   10                  15
   agc cac ggg ccc aga ccc gcc acg gcg ccc gcg ccg ccg ccc tcg ccg       96
   Ser His Gly Pro Arg Pro Ala Thr Ala Pro Ala Pro Pro Pro Ser Pro
               20                  25                  30
   gag ccc acg aga cct gca tgg acg ggc atg ggc ttg aga gca gca cct      144
   Glu Pro Thr Arg Pro Ala Trp Thr Gly Met Gly Leu Arg Ala Ala Pro
           35                  40                  45
   tcc agc gcc gcc gct gcc gcc gcc gag gtt gag cag cgc cgc cgc ccc      192
   Ser Ser Ala Ala Ala Ala Ala Ala Glu Val Glu Gln Arg Arg Arg Pro
       50                  55                  60
   ggg ctc tgc ccc ccg ccg ctg gag ctg ctg ctg ctg ctg ctg ttc agc      240
   Gly Leu Cys Pro Pro Pro Leu Glu Leu Leu Leu Leu Leu Leu Phe Ser
   65                  70                  75                  80
   ctc ggg ctg ctc cac gca ggt gac tgc caa cag cca gcc caa tgt cga      288
   Leu Gly Leu Leu His Ala Gly Asp Cys Gln Gln Pro Ala Gln Cys Arg
                   85                  90                  95
   atc cag aaa tgc acc acg gac ttc gtg tcc ctg act tct cac ctg aac      336
   Ile Gln Lys Cys Thr Thr Asp Phe Val Ser Leu Thr Ser His Leu Asn
   100 105 110
   tct gcc gtt gac ggc ttt gac tct gag ttt tgc aag gcc ttg cgt gcc 384
   Ser Ala Val Asp Gly Phe Asp Ser Glu Phe Cys Lys Ala Leu Arg Ala
   115 120 125
   tat gct ggc tgc acc cag cga act tca aaa gcc tgc cgt ggc aac ctg 432
   Tyr Ala Gly Cys Thr Gln Arg Thr Ser Lys Ala Cys Arg Gly Asn Leu
   130 135 140
   gta tac cat tct gcc gtg ttg ggt atc agt gac ctc atg agc cag agg 480
   Val Tyr His Ser Ala Val Leu Gly Ile Ser Asp Leu Met Ser Gln Arg
   145 150 155 160
   aat tgt tcc aag gat gga ccc aca tcc tct acc aac ccc gaa gtg acc 528
   Asn Cys Ser Lys Asp Gly Pro Thr Ser Ser Thr Asn Pro Glu Val Thr
   165 170 175
   cat gat cct tgc aac tat cac agc cac gct gga gcc agg gaa cac agg 576
   His Asp Pro Cys Asn Tyr His Ser His Ala Gly Ala Arg Glu His Arg
   180 185 190
   aga ggg gac cag aac cct ccc agt tac ctt ttt tgt ggc ttg ttt gga 624
   Arg Gly Asp Gln Asn Pro Pro Ser Tyr Leu Phe Cys Gly Leu Phe Gly
   195 200 205
   gat cct cac ctc aga act ttc aag gat aac ttc caa aca tgc aaa gta 672
   Asp Pro His Leu Arg Thr Phe Lys Asp Asn Phe Gln Thr Cys Lys Val
   210 215 220
   gaa ggg gcc tgg cca ctc ata gat aat aat tat ctt tca gtt caa gtg 720
   Glu Gly Ala Trp Pro Leu Ile Asp Asn Asn Tyr Leu Ser Val Gln Val
   225 230 235 240
   aca aac gta cct gtg gtc cct gga tcc agt gct act gct aca aat aag 768
   Thr Asn Val Pro Val Val Pro Gly Ser Ser Ala Thr Ala Thr Asn Lys
   245 250 255
   atc act att atc ttc aaa gcc cac cat gag tgt aca gat cag aaa gtc 816
   Ile Thr Ile Ile Phe Lys Ala His His Glu Cys Thr Asp Gln Lys Val
   260 265 270
   tac caa gct gtg aca gat gac ctg ccg gcc gcc ttt gtg gat ggc acc 864
   Tyr Gln Ala Val Thr Asp Asp Leu Pro Ala Ala Phe Val Asp Gly Thr
   275 280 285
   acc agt ggt ggg gac agc gat gcc aag agc ctg cgt atc gtg gaa agg 912
   Thr Ser Gly Gly Asp Ser Asp Ala Lys Ser Leu Arg Ile Val Glu Arg
   290 295 300
   gag agt ggc cac tat gtg gag atg cac gcc cgc tat ata ggg acc aca      960
   Glu Ser Gly His Tyr Val Glu Met His Ala Arg Tyr Ile Gly Thr Thr
   305                 310                 315                 320
   gtg ttt gtg cgg cag gtg ggt cgc tac ctg acc ctt gcc atc cgt atg      1008
   Val Phe Val Arg Gln Val Gly Arg Tyr Leu Thr Leu Ala Ile Arg Met
                   325                 330                 335
   cct gaa gac ctg gcc atg tcc tac gag gag agc cag gac ctg cag ctg      1056
   Pro Glu Asp Leu Ala Met Ser Tyr Glu Glu Ser Gln Asp Leu Gln Leu
               340                 345                 350
   tgc gtg aac ggc tgc ccc ctg agt gaa cgc atc gat gac ggg cag ggc      1104
   Cys Val Asn Gly Cys Pro Leu Ser Glu Arg Ile Asp Asp Gly Gln Gly
           355                 360                 365
   cag gtg tct gcc atc ctg gga cac agc ctg cct cgc acc tcc ttg gtg      1152
   Gln Val Ser Ala Ile Leu Gly His Ser Leu Pro Arg Thr Ser Leu Val
       370                 375                 380
   cag gcc tgg cct ggc tac aca ctg gag act gcc aac act caa tgc cat      1200
   Gln Ala Trp Pro Gly Tyr Thr Leu Glu Thr Ala Asn Thr Gln Cys His
   385                 390                 395                 400
   gag aag atg cca gtg aag gac atc tat ttc cag tcc tgt gtc ttc gac      1248
   Glu Lys Met Pro Val Lys Asp Ile Tyr Phe Gln Ser Cys Val Phe Asp
                   405                 410                 415
   ctg ctc acc act ggt gat gcc aac ttt act gcc gca gcc cac agt gcc      1296
   Leu Leu Thr Thr Gly Asp Ala Asn Phe Thr Ala Ala Ala His Ser Ala
               420                 425                 430
   ttg gag gat gtg gag gcc ctg cac cca agg aag gaa cgc tgg cac att      1344
   Leu Glu Asp Val Glu Ala Leu His Pro Arg Lys Glu Arg Trp His Ile
           435                 440                 445
   ttc ccc agc agt ggc aat ggg act ccc cgt gga ggc agt gat ttg tct      1392
   Phe Pro Ser Ser Gly Asn Gly Thr Pro Arg Gly Gly Ser Asp Leu Ser
       450                 455                 460
   gtc agt cta gga ctc acc tgc ttg atc ctt atc gtg ttt ttg tag          1437
   Val Ser Leu Gly Leu Thr Cys Leu Ile Leu Ile Val Phe Leu
   465                 470                 475
<210> 4
   <211> 478
   <212> PRT
   <213> Homo sapiens
<400> 4
   Met Ile Arg Lys Lys Arg Lys Arg Ser Ala Pro Pro Gly Pro Cys Arg
   1               5                   10                  15
   Ser His Gly Pro Arg Pro Ala Thr Ala Pro Ala Pro Pro Pro Ser Pro
               20                  25                  30
   Glu Pro Thr Arg Pro Ala Trp Thr Gly Met Gly Leu Arg Ala Ala Pro
           35                  40                  45
   Ser Ser Ala Ala Ala Ala Ala Ala Glu Val Glu Gln Arg Arg Arg Pro
       50                  55                  60
   Gly Leu Cys Pro Pro Pro Leu Glu Leu Leu Leu Leu Leu Leu Phe Ser
   65                  70                  75                  80
   Leu Gly Leu Leu His Ala Gly Asp Cys Gln Gln Pro Ala Gln Cys Arg
                   85                  90                  95
   Ile Gln Lys Cys Thr Thr Asp Phe Val Ser Leu Thr Ser His Leu Asn
               100                 105                 110
   Ser Ala Val Asp Gly Phe Asp Ser Glu Phe Cys Lys Ala Leu Arg Ala
           115                 120                 125
   Tyr Ala Gly Cys Thr Gln Arg Thr Ser Lys Ala Cys Arg Gly Asn Leu
       130                 135                 140
   Val Tyr His Ser Ala Val Leu Gly Ile Ser Asp Leu Met Ser Gln Arg
   145                 150                 155                 160
   Asn Cys Ser Lys Asp Gly Pro Thr Ser Ser Thr Asn Pro Glu Val Thr
                   165                 170                 175
   His Asp Pro Cys Asn Tyr His Ser His Ala Gly Ala Arg Glu His Arg
               180                 185                 190
   Arg Gly Asp Gln Asn Pro Pro Ser Tyr Leu Phe Cys Gly Leu Phe Gly
           195                 200                 205
   Asp Pro His Leu Arg Thr Phe Lys Asp Asn Phe Gln Thr Cys Lys Val
       210                 215                 220
   Glu Gly Ala Trp Pro Leu Ile Asp Asn Asn Tyr Leu Ser Val Gln Val
   225                 230                 235                 240
   Thr Asn Val Pro Val Val Pro Gly Ser Ser Ala Thr Ala Thr Asn Lys
                   245                 250                 255
   Ile Thr Ile Ile Phe Lys Ala His His Glu Cys Thr Asp Gln Lys Val
               260                 265                 270
   Tyr Gln Ala Val Thr Asp Asp Leu Pro Ala Ala Phe Val Asp Gly Thr
           275                 280                 285
   Thr Ser Gly Gly Asp Ser Asp Ala Lys Ser Leu Arg Ile Val Glu Arg
       290                 295                 300
   Glu Ser Gly His Tyr Val Glu Met His Ala Arg Tyr Ile Gly Thr Thr
   305                 310                 315                 320
   Val Phe Val Arg Gln Val Gly Arg Tyr Leu Thr Leu Ala Ile Arg Met
                   325                 330                 335
   Pro Glu Asp Leu Ala Met Ser Tyr Glu Glu Ser Gln Asp Leu Gln Leu
               340                 345                 350
   Cys Val Asn Gly Cys Pro Leu Ser Glu Arg Ile Asp Asp Gly Gln Gly
           355                 360                 365
   Gln Val Ser Ala Ile Leu Gly His Ser Leu Pro Arg Thr Ser Leu Val
       370                 375                 380
   Gln Ala Trp Pro Gly Tyr Thr Leu Glu Thr Ala Asn Thr Gln Cys His
   385                 390                 395                 400
   Glu Lys Met Pro Val Lys Asp Ile Tyr Phe Gln Ser Cys Val Phe Asp
                   405                 410                 415
   Leu Leu Thr Thr Gly Asp Ala Asn Phe Thr Ala Ala Ala His Ser Ala
               420                 425                 430
   Leu Glu Asp Val Glu Ala Leu His Pro Arg Lys Glu Arg Trp His Ile
           435                 440                 445
   Phe Pro Ser Ser Gly Asn Gly Thr Pro Arg Gly Gly Ser Asp Leu Ser
       450                 455                 460
   Val Ser Leu Gly Leu Thr Cys Leu Ile Leu Ile Val Phe Leu
   465                 470                 475
<210> 5
   <211> 1281
   <212> DNA
   <213> Homo sapiens
<220>
   <221> CDS
   <222> (1)..(1281)
<400> 5
atg ggg gag cca ggc cag tcc cct agt ccc agg tcc tcc cat ggc agt      48
   ccc cca act cta agc act ctc act ctc ctg ctg ctc ctc tgt gga cat      96
   gct cat tct caa tgc aag atc ctc cgc tgc aat gct gag tac gta tcg     144
   tcc act ctg agc ctt aga ggt ggg ggt tca tca gga gca ctt cga gga     192
   gga gga gga gga ggc cgg ggt gga ggg gtg ggc tct ggc ggc ctc tgt     240
   cga gcc ctc cgc tcc tat gcg ctc tgc act cgg cgc acc gcc cgc acc        288
   tgc cgc ggg gac ctc gcc ttc cat tcg gcg gta cat ggc atc gaa gac        336
   ctg atg atc cag cac aac tgc tcc cgc cag ggc cct aca gcc cct ccc        384
   ccg ccc cgg ggc ccc gcc ctt cca ggc gcg ggc tcc ggc ctc cct gcc        432
   ccg gac cct tgt gac tat gaa ggc cgg ttt tcc cgg ctg cat ggt cgt        480
   ccc ccg ggg ttc ttg cat tgc gct tcc ttc ggg gac ccc cat gtg cgc        528
   agc ttc cac cat cac ttt cac aca tgc cgt gtc caa gga gct tgg cct        576
   cta ctg gat aat gac ttc ctc ttt gtc caa gcc acc agc tcc ccc atg        624
   gcg ttg ggg gcc aac gct acc gcc acc cgg aag ctc acc atc ata ttt        672
   aag aac atg cag gaa tgc att gat cag aag gtg tat cag gct gag gtg        720
   gat aat ctt cct gta gcc ttt gaa gat ggt tct atc aat gga ggt gac        768
   cga cct ggg gga tcc agt ttg tcg att caa act gct aac cct ggg aac        816
   cat gtg gag atc caa gct gcc tac att ggc aca act ata atc att cgg      864
   cag aca gct ggg cag ctc tcc ttc tcc atc aag gta gca gag gat gtg      912
   gcc atg gcc ttc tca gct gaa cag gac ctg cag ctc tgt gtt ggg ggg      960
   tgc cct cca agt cag cga ctc tct cga tca gag cgc aat cgt cgg gga      1008
   gct ata acc att gat act gcc aga cgg ctg tgc aag gaa ggg ctt cca      1056
   gtg gaa gat gct tac ttc cat tcc tgt gtc ttt gat gtt tta att tct      1104
   ggt gat ccc aac ttt acc gtg gca gct cag gca gca ctg gag gat gcc      1152
   cga gcc ttc ctg cca gac tta gag aag ctg cat ctc ttc ccc tca gat      1200
   gct ggg gtt cct ctt tcc tca gca acc ctc tta gct cca ctc ctt tct      1248
   ggg ctc ttt gtt ctg tgg ctt tgc att cag taa                          1281
<210> 6
   <211> 426
   <212> PRT
   <213> Homo sapiens
<400> 6
<210> 7
   <211> 19
   <212> PRT
   <213> Artificial
<220>
   <223> Consensus
<220>
   <221> MISC_FEATURE
   <222> (2)..(3)
   <223> Xaa is any amino acid
<220>
   <221> MISC_FEATURE
   <222> (6)..(7)
   <223> Xaa is any amino acid
<220>
   <221> misc_feature
   <222> (9)..(9)
   <223> Xaa can be any naturally occurring amino acid
<220>
   <221> MISC_FEATURE
   <222> (15)..(17)
   <223> Xaa is any amino acid
<400> 7
   Gly Xaa Xaa Val Glu Xaa Xaa Ala Xaa Tyr Ile Gly Thr Thr Xaa Xaa
   1 5 10 15
   Xaa Arg Gln
<210> 8
   <211> 19
   <212> PRT
   <213> Artificial
<220>
   <223> peptide of binding domain
<400> 8
   Gly Gln His Val Glu Ile Gln Ala Lys Tyr Ile Gly Thr Thr Ile Val
   1 5 10 15
   Val Arg Gln
<210> 9
   <211> 19
   <212> PRT
   <213> Artificial
<220>
   <223> peptide of binding domain
<400> 9
   Gly His Tyr Val Glu Met His Ala Arg Tyr Ile Gly Thr Thr Val Phe
   1 5 10 15
   Val Arg Gln
<210> 10
   <211> 19
   <212> PRT
   <213> Artificial
<220>
   <223> peptide of binding domain
<400> 10
   Gly Asn His Val Glu Ile Gln Ala Ala Tyr Ile Gly Thr Thr Ile Ile
   1 5 10 15
   Ile Arg Gln
<210> 11
   <211> 79
   <212> DNA
   <213> Artificial
<220>
   <223> PCR Primer
<400> 11
   ggggacaagt ttgtacaaaa aagcaggctt tctggaagtt ctgttccagg ggcccccaca 60
   cctcaggact ttcaccgac 79
<210> 12
   <211> 55
   <212> DNA
   <213> Artificial
<220>
   <223> PCR Primer
<400> 12
   ggggaccact ttgtacaaga aagctgggtg ctcgtccatc tcagcctggt acacc 55
<210> 13
   <211> 83
   <212> DNA
   <213> Artificial
<220>
   <223> PCR Primer
<400> 13
   ggggacaagt ttgtacaaaa aagcaggctt tctggaagtt ctgttccagg ggcccatcat 60
   cttcaagaac ttccaggagt gtg 83
<210> 14
   <211> 49
   <212> DNA
   <213> Artificial
<220>
   <223> PCR Primer
<400> 14
   ggggaccact ttgtacaaga aagctgggtg gcgcaccacg atggtggtg 49
<210> 15
   <211> 77
   <212> DNA
   <213> Artificial
<220>
   <223> PCR Primer
<400> 15
   ggggacaagt ttgtacaaaa aagcaggctt tctggaagtt ctgttccagg ggccctcagg 60
   ccagcacgtg gagatcc 77
<210> 16
   <211> 52
   <212> DNA
   <213> Artificial
<220>
   <223> PCR Primer
<400> 16
   ggggaccact ttgtacaaga aagctgggtc ctcagcattg gtgtggaagg cc 52
<210> 17
   <211> 73
   <212> DNA
   <213> Artificial
<220>
   <223> PCR Primer
<400> 17
   ggggacaagt ttgtacaaaa aagcaggctt tctggaagtt ctgttccagg ggcccctgcg 60
   gggctgcccc ctc 73
<210> 18
   <211> 49
   <212> DNA
   <213> Artificial
<220>
   <223> PCR Primer
<400> 18
   ggggaccact ttgtacaaga aagctgggtc gcccgtggtg aggaggtcg 49
<210> 19
   <211> 76
   <212> DNA
   <213> Artificial
<220>
   <223> PCR Primer
<400> 19
   ggggacaagt ttgtacaaaa aagcaggctt tctggaagtt ctgttccagg ggcccctgcc 60
   ggtggaggac ctgtac 76
<210> 20
   <211> 49
   <212> DNA
   <213> Artificial
<220>
   <223> PCR Primer
<400> 20
   ggggaccact ttgtacaaga aagctgggtt gcctggcagg tcccgagtc 49
<210> 21
   <211> 79
   <212> DNA
   <213> Artificial
<220>
   <223> PCR Primer
<400> 21
   ggggacaagt ttgtacaaaa aagcaggctt tctggaagtt ctgttccagg ggcccccaca 60
   cctcaggact ttcaccgac 79
<210> 22
   <211> 49
   <212> DNA
   <213> Artificial
<220>
   <223> PCR Primer
<400> 22
   ggggaccact ttgtacaaga aagctgggtt gcctggcagg tcccgagtc 49
<210> 23
   <211> 32
   <212> PRT
   <213> Artificial
<220>
   <223> peptide of binding domain
<400> 23
   Lys Ile Thr Glu Lys Val Ser Gly Gln His Val Glu Ile Gln Ala Lys
   1 5 10 15
   Tyr Ile Gly Thr Thr Ile Val Val Arg Gln Val Gly Arg Tyr Leu Thr
   20 25 30
<210> 24
   <211> 32
   <212> PRT
   <213> Artificial
<220>
   <223> peptide of binding domain
<400> 24
   Arg Ile Val Glu Arg Glu Ser Gly His Tyr Val Glu Met His Ala Arg
   1 5 10 15
   Tyr Ile Gly Thr Thr Val Phe Val Arg Gln Val Gly Arg Tyr Leu Thr
   20 25 30
<210> 25
   <211> 32
   <212> PRT
   <213> Artificial
<220>
   <223> peptide of binding domain
<400> 25
   Ser Ile Gln Thr Ala Asn Pro Gly Asn His Val Glu Ile Gln Ala Ala
   1               5                   10                  15
   Tyr Ile Gly Thr Thr Ile Ile Ile Arg Gln Thr Ala Gly Gln Leu Ser
               20                  25                  30
<210> 26
   <211> 16
   <212> PRT
   <213> Artificial
<220>
   <223> peptide of binding domain
<400> 26
   Lys Ile Thr Glu Lys Val Ser Gly Gln His Val Glu Ile Gln Ala Lys
   1               5                   10                  15
<210> 27
   <211> 16
   <212> PRT
   <213> Artificial
<220>
   <223> peptide of binding domain
<400> 27
   Tyr Ile Gly Thr Thr Ile Val Val Arg Gln Val Gly Arg Tyr Leu Thr
   1               5                   10                  15
<210> 28
   <211> 16
   <212> PRT
   <213> Artificial
<220>
   <223> peptide of binding domain
<400> 28
   Val Val Asn Ala Val Glu Asp Trp Asp Ser Gln Gly Leu Tyr Leu Cys
   1 5 10 15
<210> 29
   <211> 18
   <212> PRT
   <213> Artificial
<220>
   <223> peptide of binding domain
<400> 29
   Thr Ile Ile Phe Lys Asn Phe Gln Glu Cys Val Asp Gln Lys Val Tyr
   1 5 10 15
   Gln Ala
<210> 30
   <211> 18
   <212> PRT
   <213> Artificial
<220>
   <223> peptide of binding domain
<400> 30
   Ala Phe Val Asp Gly Ser Lys Asn Gly Gly Asp Lys His Gly Ala Asn
   1 5 10 15
   Ser Leu
<210> 31
   <211> 16
   <212> PRT
   <213> Artificial
<220>
   <223> peptide of binding domain
<400> 31
   Arg Ile Val Glu Arg Glu Ser Gly His Tyr Val Glu Met His Ala Arg
   1 5 10 15
<210> 32
   <211> 16
   <212> PRT
   <213> Artificial
<220>
   <223> peptide of binding domain
<400> 32
   Tyr Ile Gly Thr Thr Val Phe Val Arg Gln Val Gly Arg Tyr Leu Thr
   1               5                   10                  15
<210> 33
   <211> 16
   <212> PRT
   <213> Artificial
<220>
   <223> peptide of binding domain
<400> 33
   Ser Ile Gln Thr Ala Asn Pro Gly Asn His Val Glu Ile Gln Ala Ala
   1               5                   10                  15
<210> 34
   <211> 16
   <212> PRT
   <213> Artificial
<220>
   <223> peptide of binding domain
<400> 34
   Tyr Ile Gly Thr Thr Ile Ile Ile Arg Gln Thr Ala Gly Gln Leu Ser
   1               5                   10                  15

## Beschreibung

Die vorliegende Erfindung betrifft die Identifizierung und Verwendung der Rezeptor-bindenden Domäne von Mitgliedern der Repulsive Guidance Molecule (RGM) Proteinfamilie sowie davon abgeleitete Polypeptidfragmente. Die erfindungsgemäßen Domänen beziehungsweise Peptidfragmente eignen sich als Mittel zur aktiven oder passiven Immunisierung von Individuen sowie als diagnostische und therapeutische Mittel zur Verwendung bei Krankheiten oder Krankheitszuständen, an deren Entstehung oder Verlauf ein Mitglied der RGM-Familie und ein diesem Molekül zugeordneter zellulärer Rezeptor beteiligt ist. Die Erfindung betrifft außerdem monoklonale und polyklonale Antikörper gegen die erfindungsgemäßen Bindungsdomänen und gegen die davon abgeleiteten Polypeptide sowie Verfahren zur Herstellung der erfindungsgemäßen Domänen, Polypeptide und Antikörper.

### Stand der Technik

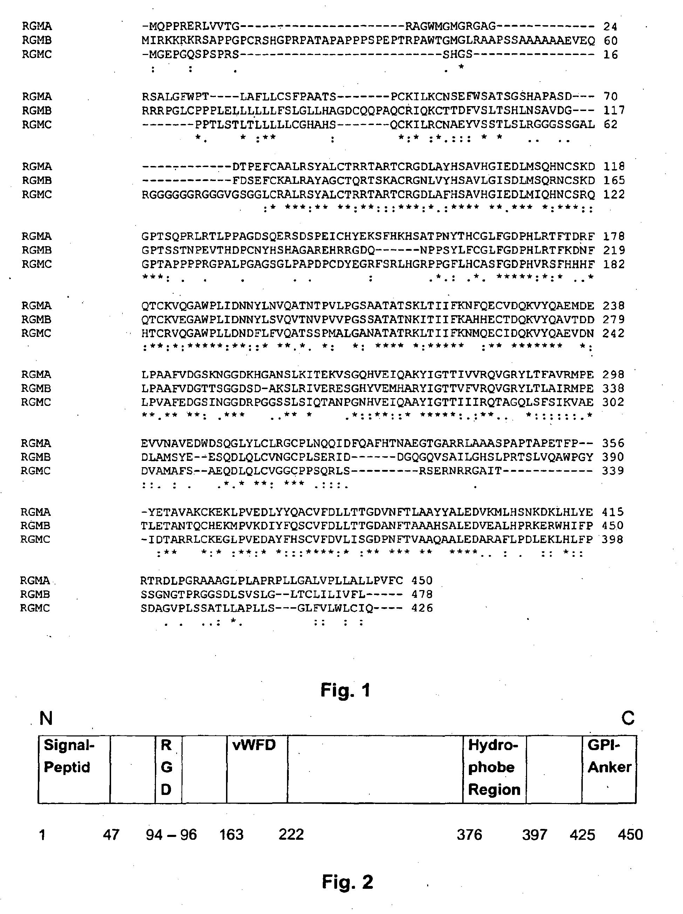

Die Funktion der Mitglieder der RGM-Proteinfamilie wurde erstmals von Monnier, P.P. et al, Nature, 419, p392-395, 2002 beschrieben. Diese Familie umfasst drei bisher bekannte Mitglieder, die als RGM A, RGM B (auch bezeichnet als DRAGON) und RGM C (auch bezeichnet als Hemojuvelin) bezeichnet werden (Niederkofler V. et al. J. Neurosci. 24, 808-18, 2004). Es handelt sich dabei um Glykoproteine die über einen Lipid-Anker (Glycosylphosphatidylinositol-Anker = GPI-Anker) an die Plasmamembran gebunden sind. Die Mitglieder dieser Proteinfamilie weisen keine ausgedehnte Sequenzhomologie zu anderen Proteinen auf und als Strukturmerkmale sind im Wesentlichen folgende Bereiche identifiziert worden: ein N-terminales Signalpeptid; eine RGD-Sequenz; eine proteolytische Spaltstelle um die Aminosäuresequenz GDPH; eine Struktur-homologe von-Willebrand-Faktor-Domäne (vWF D); eine hydrophobe Sequenz in Nähe des C-Terminus und eine C-terminale GPI-Anker Consensus Sequenz (vgl. auch Figur 2).

Beim Menschen sind die kodierende Sequenzen für RGM A auf Chromosom 15, für RGM B auf Chromosom 5 und für RGM C auf Chromosom 1 lokalisiert. Es ist ein charakteristisches Expressionsmuster zu beobachten: RGM A und B werden insbesondere in adultem Gehirn und Rückenmark, RGM C wird insbesondere in Skelettmuskel, Leber und Herzmuskel, und RGM B außerdem im Knorpelgewebe exprimiert.

RGM Proteine wurden ursprünglich identifiziert als Kandidatenproteine, die bei der Bildung topographischer neuronaler Projektionen eine wichtige Rolle spielen (Stahl B. et al. Neuron 5:p 735-43, 1990; Mueller B.K. et al. Curr. Biol. 6, p. 1497-1502, 1996; Mueller BK in Molecular Basis of Axon Growth and Nerve Pattern Formation, Edited by H. Fujisawa, Japan Scientific Societies Press, 215-229, 1997). Ihre Fähigkeit abstoßend (repulsiv) oder hemmend (inhibitorisch) auf wachsende Nervenfasern zu wirken, war ein entscheidendes funktionelles Merkmal, welches bei ihrer Isolierung, Klonierung und Charakterisierung eine wichtige Rolle spielte. In einfachen zellulären Testsystemen war die Aktivität gut nachweisbar. In zwei unterschiedlichen zellulären Assays wirken RGM Proteine hemmend oder repulsiv. Im Kollapstest werden zu auswachsenden Nervenfasern RGM-Proteine gegeben. Die Bindung von RGM and den RGM-Rezeptor löst eine heftige Reaktion aus, bei der sämtliche membranösen Elemente des neuronalen Wachstumskegels eingezogen werden. Der ursprüngliche ausgedehnte hand-ähnliche Wachstumskegel wird dabei zu einem dünnen Faden umgewandelt. In Gegenwart von RGM bleiben die Nervenfasern gehemmt, ziehen sich stark zurück und können ihr Wachstum nicht mehr weiter fortsetzen.

RGM-Proteine üben einen Teil ihrer Wirkung durch Bindung an den RGM-Rezeptor Neogenin aus (Rajagopalan S. et al., Nat Cell Biol. 6 p.756-62, 2004). Neogenin ist nahe verwandt mit dem DCC Rezeptor (Deleted in Colorecal Cancer). Beide Rezeptoren sind Mitglieder der Immunoglobulin-Superfamilie und besitzen eine extrazelluläre, eine Transmembran- und eine intrazelluläre Domäne. Beide wurden als Rezeptoren für einen weiteren Liganden, das Netrin-1, beschrieben, aber lediglich Neogenin, nicht jedoch DCC, bindet RGM Proteine. Die extrazellulären Domänen dieser Rezeptoren bestehen aus 4 Immunoglobulin-ähnlichen Domänen, auf die 6 Fibronektin-Repeat Domänen folgen.

Am besten verstanden ist die Funktion von RGM A im Nervensystem und besonders auffällig ist dessen Wirkung, in niedrigsten Konzentrationen das Wachstum von Nervenfasern zu hemmen. Eine Verletzung des zentralen Nervensystems beim erwachsenen Menschen und bei der adulten Ratte führt zu einer Akkumulation von RGM-Proteinen an der Läsionsstelle (Schwab JM. et al. Arch. Neurol. in press, 2005; Schwab J.M. et al. Eur. J. Neurosci. 21 :p. 387-98, 2005). Dadurch wird das erneute Auswachsen der verletzten Nervenfasern verhindert und es kommt zu permanenten, in Abhängigkeit vom Ort der Läsionsstelle, mehr oder weniger schweren Funktionsausfällen. Diese für das Nervenfaserwachstum inhibitorische Aktivität von RGM wird durch Bindung an den Rezeptor Neogenin vermittelt (Rajagopalan S. et al., a.a.O.). Der gleiche Rezeptor vermittelt über die Bindung von Netrin-1 aber auch eine gegenteilige, das Nervenfaserwachstum stimulierende Wirkung.

Neueste Ergebnisse weisen darauf hin, dass die RGM-Proteine auch wichtige Aufgaben erfüllen im zentralen und peripheren Nervensystem (Matsunaga Eiji et al., Nature Cell Biology, 6, p. 749-55, 2004; Matsunaga Eiji et al., Development, Growth & Differentiation, 46, p. 481-6, 2004), bei der Regulation des Eisenstoffwechsels (WO 2004/092405), bei Tumorerkrankungen, bei Entzündungsvorgängen und bei der Bildung von Knochen und Knorpelgewebe. Die WO 03/089608 beschreibt Methoden und Proteinzusammensetzungen zur Behandlung und Diagnose von Erkrankungen des zentralen Nervensystems, der Retina, der Haut oder der Muskulatur mit einem Protein der auch mit DRAGON bezeichneten Familie (RGM-Proteine). Die WO 00/73801 beschreibt eine Methode zur Diagnose einer Erkrankung bei der ein nukleinsäurekodiertes humanes krebsassoziiiertes Antigen exprimiert wird. Die WO 02/051438 beschreibt die Verwendung eines Modulators eines RGM Polypeptids oder eines funktionellen Fragments oder Derivats davon für die Herstellung einer pharmazeutischen Zusammensetzung zur Prophylaxe, Linderung oder Behandlung von Krankheiten oder Zuständen, die mit der Degeneration oder der Verletzung des Nervengewebes von Vertebraten verbunden sind.

Auf Grund der Beobachtung, dass RGM A, B und C mit hoher Affinität an den Neogenin-Rezeptor binden, bestand die Aufgabe der vorliegenden Erfindung darin, die funktionale Bindungsdomäne dieser Moleküle, insbesondere von RGM A, näher zu charakterisieren. Dies würde es ermöglichen, Antikörper gegen die Domäne beziehungsweise gegen davon abgeleitete aktive RGM-Peptide zu erzeugen, die mit hoher Wahrscheinlichkeit die inhibitorische Aktivität von RGM neutralisieren und die dadurch die Regeneration oder ein erneutes Auswachsen von Nervenfasern stimulieren könnten. Darüber hinaus könnten Bindungsdomänen oder davon abgeleitete aktive RGM-Peptide als therapeutisch wirksame Agenzien per se bereitgestellt werden.

### Kurze Beschreibung der Erfindung

Obige Aufgabe wurde überraschenderweise durch Isolierung und Charakterisierung der Bindungsdomäne von humanen RGM Proteinen, insbesondere RGM A, und aktiver Polypeptidfragmente davon gelöst.

### Figurenbeschreibung

Figur 1 zeigt das Sequenz-Alignment der humanen Formen von RGM A (GenBank # NP_064596.1) RGM B (GenBank # NP_001012779) und RGM C (GenBank # NP_998818.1).

Figur 2 zeigt in schematischer Darstellung den Aufbau von RGM-Molekülen. Zwischen dem N-terminalen Signalpeptid und dem C-terminalen GPI-Anker sind die RGD-Sequenz, die von Willebrand-Faktor-Domäne (vWF D) sowie eine hydrophobe Sequenz im Bereich des C-Terminus vor der Ankerregion dargestellt. Zwischen vWFD und hydrophober Region wird der erfindungsgemäß interessierende Bindungsdomänenbereich vermutet. Unterhalb des Diagramms sind die entsprechenden Aminosäurepositionen für humanes RGM A, die proteolytische Spaltstelle zwischen den Aminosäuren 168 und 169.

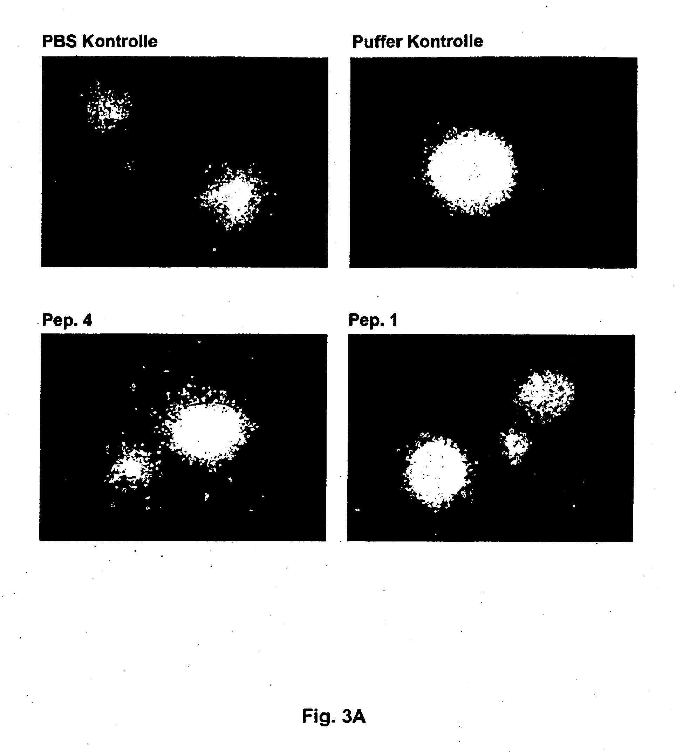

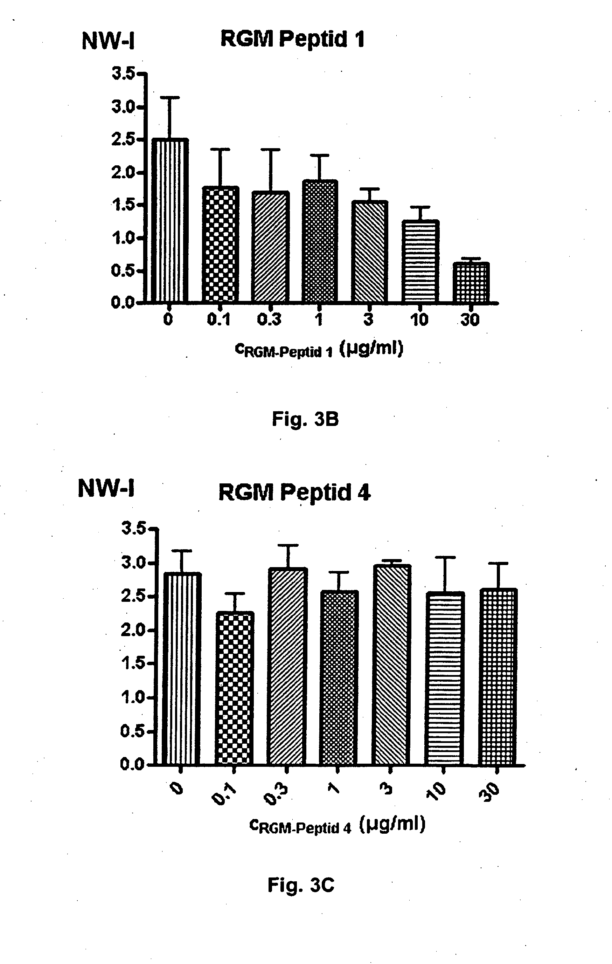

Figur 3A zeigt anhand fluoreszenzmikroskopischer Aufnahmen den Einfluss verschiedener RGM A-Peptidfragmente auf das Nervenfaserwachstum neuronaler Zellen der Ratte. Während Peptid 1 bei einer Konzentration von 10 µg/ml inhibitorische Aktivität zeigt, ist Peptid 4 bei gleicher Konzentration unwirksam. Entsprechende Kontrollansätze mit Puffer bzw. PBS sind zum Vergleich dargestellt. Figur 3B zeigt ein Balkendiagramm ermittelter Neuriten-Wachstums-Indices (ein Maß für die von Neuriten bedeckte Fläche in Relation zur Zellaggregatgröße; gezeigt ist Mittelwert und Standardabweichung) zur Veranschaulichung der Konzentrationsabhängigkeit der Neuritenwachstuminhibierenden Aktivität des erfindungsgemäßen RGM-A-Peptids 1. Das entsprechende Balkendiagramm für das RGM-A-Peptid 4 ist in Figur 3C gezeigt.

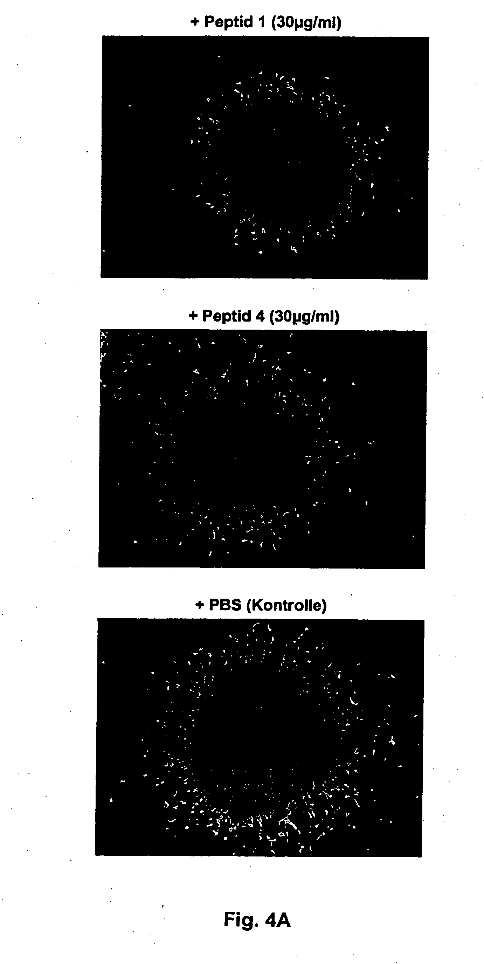

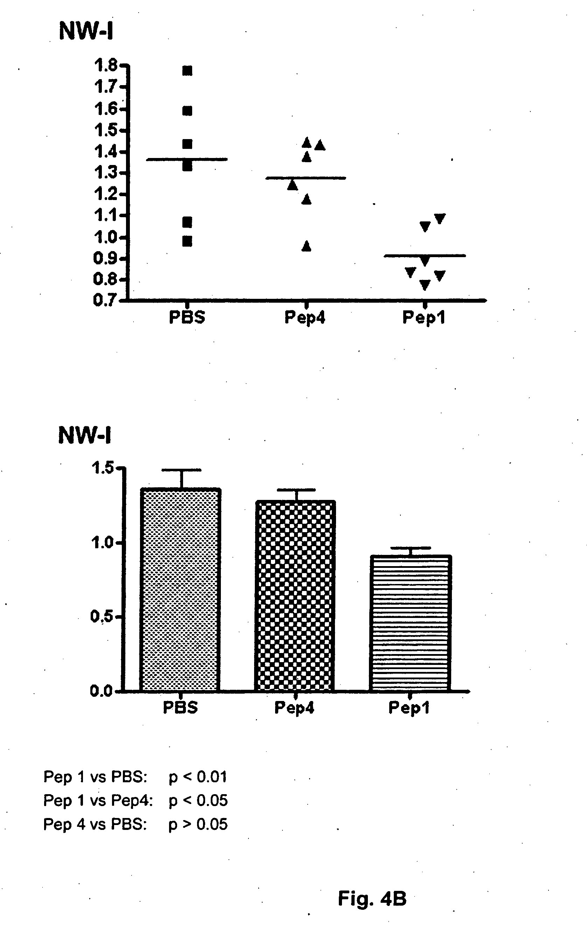

In Figur 4A sind fluoreszenzmikroskopische Aufnahmen von humanen neuronalen NTera-Zellen dargestellt, welche den Einfluss von RGM-A-Peptid 1 und -Peptid 4 (jeweils in einer Konzentration von 30 µg/ml) auf das Nervenfaserwachstum dieser Zellen veranschaulichen. Wie der Vergleich mit der ebenfalls dargestellten Kontrollaufnahme (Inkubation mit PBS an Stelle von Peptid) zeigt, bewirkt ausschließlich Peptid 1 eine Inhibition des Nervenfaserwachstums. In Figur 4B sind die entsprechend ermittelten Neuritenwachstums-Indizes für Versuchsreihen mit Peptid 1, Peptid 4 und Kontrolle (PBS) dargestellt (oberes Diagramm: Einzelmessungen und Mittelwert, unteres Diagramm: Balkendiagramm mit Mittelwert und Standardabweichung).

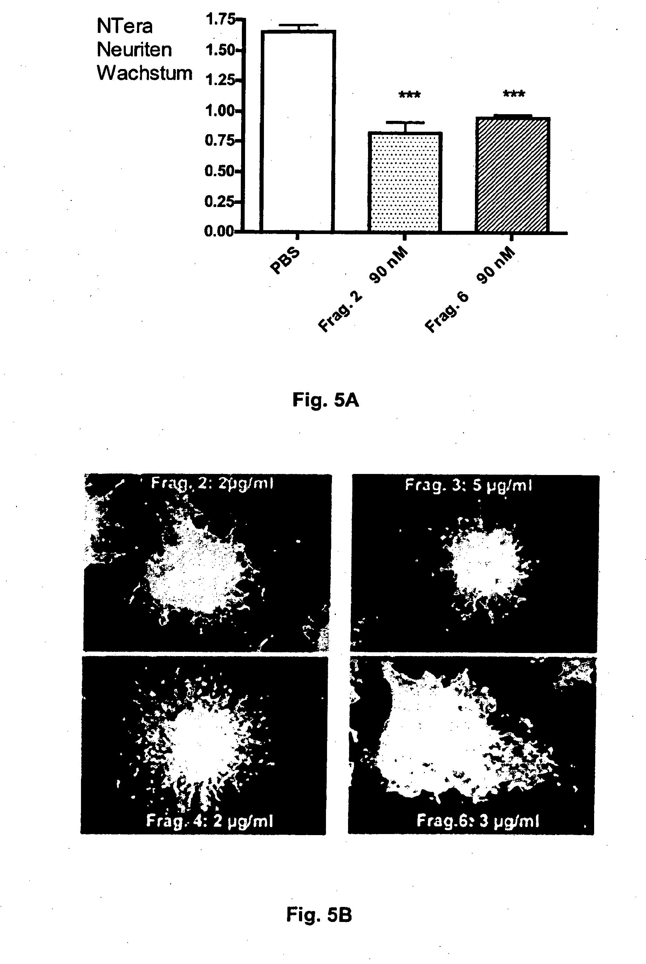

Figur 5A zeigt die Ergebnisse einer Analyse von RGM A Fragmenten im NTera Nervenfaserwachstumstest. Die beiden RGM A Fragmente 2 (218 - 284) und 6 (168 - 422) wurden in einer Konzentration von 90 nM zu NTera Neuronen gegeben. Beide Fragmente hemmen stark das Nervenfaser-Wachstum der NTera Neuronen. ***= p < 0.001 Signifikanz gegenüber PBS Kontrolle.

Figur 5B zeigt fluoreszenzmikroskopische Aufnahmen der Testresultate von RGM A Fragmenten im NTera Nervenfaserwachstumstest. Die RGM A Fragmente 2 (218 - 284), 3 (266 -335), 4 (316-386) und 6 (168 - 422) wurden in den angegebenen Konzentrationen zu NTera Neuronen pipettiert. Aktiv, das heisst inhibitorisch für das Neuritenwachstum waren, Fragmente 2, 3 und 6, inaktiv war Fragment 4.

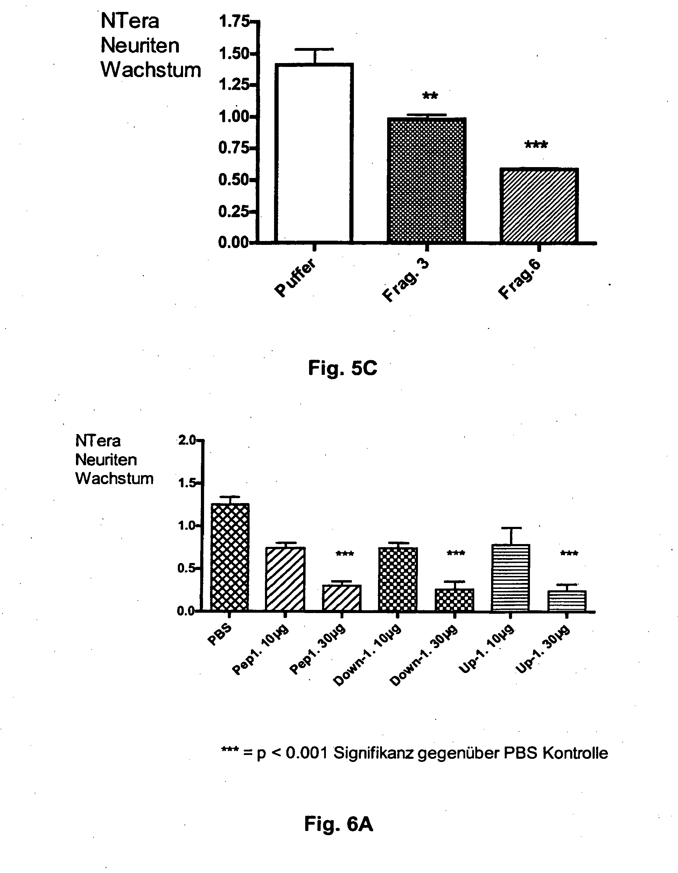

Figur 5C zeigt weitere Ergebnisse der Analyse von RGM A Fragmenten im NTera Nervenfaserwachstumstest. Die beiden RGM A Fragmente 3 (286 - 335) und 6 (168 - 422) wurden in einer Konzentration von 6 µg/ml zu NTera Neuronen gegeben. Beide Fragmente hemmen stark das Nervenfaser-Wachstum der NTera Neuronen. **= p < 0.01 Signifikanz gegenüber Puffer Kontrolle, ***= p < 0.001 Signifikanz gegenüber Puffer Kontrolle

Figur 6A zeigt das Ergebnis der Analyse von RGM A Peptid 1 und zwei damit teilweise überlappenden Peptiden (Down-1 und Up-1) im NTera Nervenfaserwachstumstest. Die Peptide wurden in einer Konzentration von 10 µg/ml bzw. 30 µg/ml zu NTera Aggregaten gegeben. 24 - 36 Stunden später wurden die Kulturen fixiert, gefärbt und ausgewertet. In einer Konzentration von 30 µg/ml, waren alle 3 RGM A Peptide aktiv und hemmten das Nervenfaserwachstum hochsignifikant.

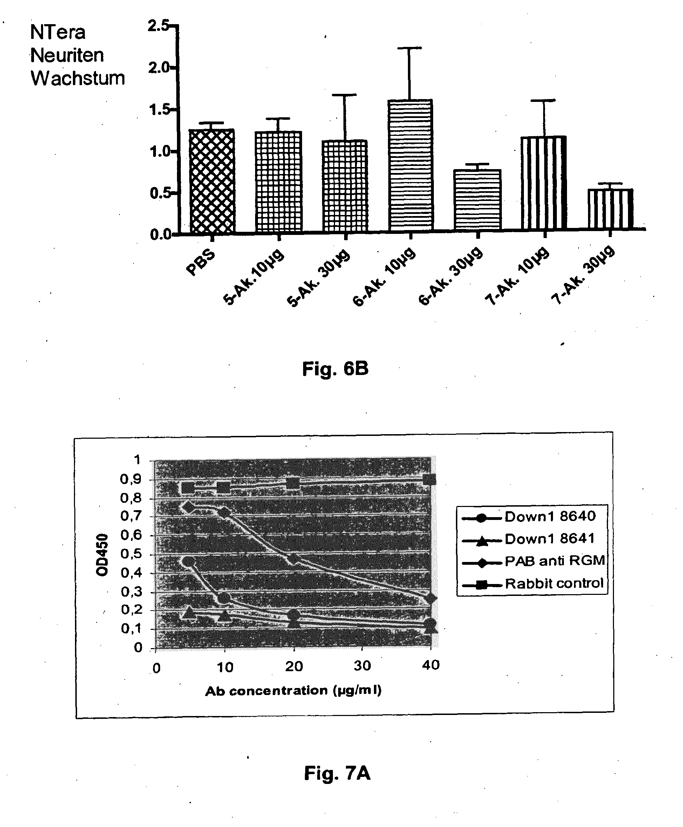

Figur 6B zeigt das Ergebnis der Analyse von drei weiteren RGM A Peptiden im NTera Nervenfaserwachstumstest. Die RGM A Peptide wurden in einer Konzentration von 10 µg/ml bzw. 30 µg/ml zu NTera Aggregaten gegeben. 24 - 36 Stunden später wurden die Kulturen fixiert, gefärbt und ausgewertet. In einer Konzentration von 10 µg/ml, waren alle 3 RGM A Peptide (5-Ak, 6-Ak, 7-Ak) inaktiv; lediglich die Peptide 7-Ak und 6-Ak zeigten bei der höheren Konzentration einen deutlichen Trend in Richtung Hemmung des Nervenfaserwachstums.

Figur 7A zeigt das Ergebnis des RGM A - Neogenin Bindungstests. In diesem Test waren die polyklonalen Antikörper, die gegen das Peptid Down-1 gerichtet sind, sehr potent und blockierten selbst bei niedrigsten Konzentrationen (Down 1 8641 & Down 1 8640) die Interaktion des RGM A Liganden mit seinem Rezeptor, dem Neogenin Protein. Ein Kontrollantikörper (Rabbit control) hatte keinen Effekt, während ein kommerziell erhältlicher polyklonaler Antikörper der Firma R & D Systems (PAB anti RGM) die RGM A-Neogenin Interaktion ebenfalls blockierte, allerdings wesentlich weniger effizient.

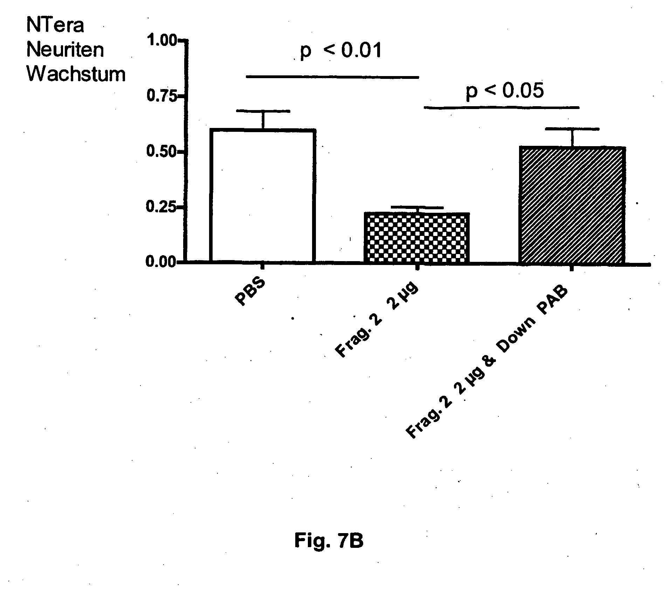

Figur 7B zeigt, wie der Down-1 spezifische poyklonale Antikörper die inhibitorische Aktivität des potenten RGM A Fragmentes 2 im NTera Nervenfaserwachstumstest neutralisiert. NTera Kulturen wurden im Auswachstest mit PBS, Fragment 2 (2 µg/ml) oder mit Fragment 2 (2 µg/ml) und gleichzeitig mit polyclonalem Antikörper Down-1 (0,6 µg/ml) behandelt.

### Detaillierte Beschreibung der Erfindung

### I. Erläuterungen allgemeine Begriffe

Als "Rezeptoren" bezeichnet man im Rahmen der vorliegenden Erfindung insbesondere an einer Zellmembran gebundene Oberflächenmoleküle, welche mit einem, beispielsweise löslichen, Liganden in Wechselwirkung treten können und als Folge dieser Wechselwirkung ein beispielsweise in das Zellinnere gerichtetes Signal oder eine Signalkaskade (auch als Signalling bezeichnet) auslösen können.

Als "Ligand" bezeichnet man einen natürlichen, d.h. in vivo gebildeten oder künstlich erzeugten, nieder- oder hochmolekularen Bindungspartner für eine "Rezeptor". Der Ligand ist vorzugsweise in der extrazellulären Umgebung frei beweglich.

Als "Immunogen" bezeichnet man ein erfindungsgemäßes Peptidfragment in glycosylierter oder nicht-glycosylierter Form, welches zur Induktion der Bildung von Antikörpern gegen das Immunogen geeignet ist. Gegebenenfalls kann eine Bindung des Immunogens (als Hapten) an einen makromolekularen Träger von Vorteil sein.

Als "Epitop" oder antigene Determinante bezeichnet man den die Spezifität eines Antikörpers bestimmenden Bereich eines Antigens, wie z.B. eines Proteins. Wird dieses Epitop, beispielsweise durch äußere Einflüsse, wie z.B. eine Wechselwirkung eines Proteins mit einem Liganden, in einem Abschnitt des Proteins neu gebildet oder auf der zugänglichen Moleküloberfläche exprimiert, so spricht man von einem "Neoepitop".

Als "Domäne" eines Proteins oder Antikörpers bezeichnet man eine durch alpha-Helix- und/oder beta-Faltblattelemente gebildete komplexe, innerhalb des Proteins abgegrenzte Struktur.

Werden keine anderen Angaben gemacht, so umfasst der Begriff "erfindungsgemäßes RGM-Protein" sowohl die Neogenin-Bindungsdomäne als auch davon abgeleitete Polypeptide eines Mitglieds der Familie von RGM-Molekülen, insbesondere RGM A, B und C, im Umfang der Ansprüche. Insbesondere werden auch funktionale, "inhibitorisch aktive" Polypeptide mitumfasst.

"Inhibierende" oder "inhibitorisch aktive" Polypeptide sind solche, die das Nervenzellenwachstum in einem hierin beschriebenen Nervenzellenwachstumstest reduzieren oder vollständig inhibieren.

Werden keine anderen Angaben gemacht so steht "RGM" für RGM A, B und C insbesondere RGM A.

"Neogenin" oder "Neogenin-Rezeptor" sind synonyme Begriffe.

### II. Spezielle Gegenstände der Erfindung

Ein erster Gegenstand der Erfindung betrifft Neogenin-Rezeptor-bindende Domänen des Repulsive Guidance Molecule (RGM) in glykosylierter oder insbesondere nichtglykosylierter Form, abgeleitet von RGM von Menschen gemäß Anspruch 1.

Eine Ausführungsform betrifft die in Anspruch 2 spezifizierten Neogenin-Rezeptor-bindende Domänen, abgeleitet von einem humanen RGM A gemäß SEQ ID NO: 2, humanen RGM B gemäß SEQ ID NO:4 oder humanen RGM C gemäß SEQ ID NO: 6. Dabei umfassen die bindenden Domänen insbesondere eine Aminosäuresequenz mit einer Länge von bis zu 150, wie z.B. bis zu 125, bis zu 100, bis zu 80, bis zu 30, oder bis zu 20, wie z.B. 10 bis 20, wie z.B. 11, 12, 13, 14, 15, 16, 17, 18 oder 19 zusammenhängenden Aminosäureresten aus einen Aminosäuresequenzbereich von RGM und zwar C-terminal relativ zur RGD-Schnittstelle und N-terminal relativ zur GPI-Ankerregion des RGM, wie insbesondere RGM A.

Als konkrete Beispiele sind zu nennen:
GQHVEIQAKYIGTTIVVRQ (SEQ ID NO:8)
GHYVEMHARYIGTTVFVRQ (SEQ ID NO:9)
GNHVEIQAAYIGTTIIIRQ (SEQ ID NO:10)

Beispiele für Neogenin-Rezeptor-bindende Domänen umfassen eine Aminosäuresequenz von Aminosäureposition 200-350 gemäß SEQ ID NO:2oder funktionale Neogenin-Rezeptor-bindende Fragmente davon. Insbesondere umfassen solche Domänen oder Fragmente:
die Aminosäurepositionen 200-325, 200-300, 200-285, 250-285 oder 260-285 oder 260-280 oder die Fragmente 215-340, 260-340, 250-300, 260-291, 210-260, oder 290-350 gemäß SEQ ID NO:2.

Weitere konkrete Beispiele für erfindungsgemäß geeignete Bindungsdomänen sind:
KITEKVSGQHVEIQAKYIGTTIWRQVGRYLT (SEQ ID NO: 23) abgeleitet von RGM A;
RIVERESGHYVEMHARYIGTTVFVRQVGRYLT (SEQ ID NO: 24) abgeleitet von RGM B;
SIQTANPGNHVEIQAAYIGTTIIIRQTAGQLS (SEQ ID NO: 25) abgeleitet von RGM C.
KITEKVSGQHVEIQAK (SEQ ID NO: 26) abgeleitet von RGM A;
YIGTTIVVRQVGRYLT (SEQ ID NO: 27) abgeleitet von RGM A;
WNAVEDWDSQGLYLC (SEQ ID NO: 28) abgeleitet von RGM A;
TIIFKNFQECVDQKVYQA (SEQ ID NO: 29) abgeleitet von RGM A;
RIVERESGHY VEMHAR (SEQ ID NO: 31) abgeleitet von RGM B;
YIGTTVFVRQ VGRYLT (SEQ ID NO: 32) abgeleitet von RGM B;
SIQTANPGNH VEIQAA (SEQ ID NO: 33) abgeleitet von RGM C; und
YIGTTIIIRQ TAGQLS (SEQ ID NO: 34) abgeleitet von RGM C.

Weitere Beispiele für Neogenin-Rezeptor-bindende Domänen oder bindende Fragmente davon umfassen wenigstens 10, wie z.B. 10-30, 10-25, 10-20 oder 10-15, wie insbesondere 10, 11, 12, 13, 14, oder 15, zusammenhängende Aminosäurereste aus einem der oben genannten Peptide oder aus dem Sequenzbereich von Position 260 bis 291 oder 267 bis 285 gemäß SEQ ID NO:2, aus dem Sequenzbereich von Position 260 bis 325 gemäß SEQ ID NO:4 oder aus dem Sequenzbereich von Position 250 bis 300 gemäß SEQ ID NO:6.

Ein weiterer Gegenstand der Erfindung betrifft antigene Polypeptid-Fragmente der Neogenin-Rezeptor-bindenden Domänen nach obiger Definition. Insbesondere sind solche antigenen Polypeptid-Fragmente Gegenstand der Erfindung, welche zur Herstellung von Immunglobulin-Molekülen brauchbar sind und die Bindung von RGM an den Neogenin-Rezeptor modulieren, insbesondere teilweise oder vollständig antagonosieren. Beispielsweise seien solche antigenen Polypeptid-Fragmente genannt, welche wenigstens 10, wie z.B. 10-30, 10-25, 10-20 oder 10-15, wie insbesondere 10, 11, 12, 13, 14, oder 15, zusammenhängende Aminosäurereste eines Peptids gemäß SEQ ID NO:7, 8, 9, 10, 23 bis 29 oder 31 bis 34 umfassen.

Ein weiterer Gegenstand der Erfindung betrifft die Verwendung einer Neogenin-Rezeptor-bindenden Domäne nach obiger Definition oder eines Polypeptid-Fragments nach obiger Definition zur Herstellung eines polyklonalen Antiserums oder monoklonaler Antikörper gegen RGM, wobei das Antiserum oder der Antikörper insbesondere die Bindung von RGM an den Neogenin-Rezeptor moduliert, vorzugsweise teilweise oder vollständig antagonisiert.

Gegenstand der Erfindung sind auch solche polyklonalen Antiseren oder monoklonale Antikörper gegen RGM gemäß obiger Definition zur Verwendung in der Diagnose oder Therapie.

Gegenstand der Erfindung ist auch die Verwendung eines erfindungsgemäßen polyklonalen Antiserums oder monoklonalen Antikörpers zur Herstellung eines pharmazeutischen Mittels zur Diagnose oder Therapie von Krankheiten oder Krankheitsstadien, welche durch eine Wechselwirkung des Neogenin-Rezeptors mit RGM oder einem RGM-Fragment mediiert werden. Insbesondere sind diese Krankheiten oder Krankheitsstadien ausgewählt unter:
a) mechanischen Verletzungen von Schädel, Gehirn und Rückenmark,
b) chronischen Erkrankungen, wie neurodegenerativen, inflammatorischen und Autoimmun-Erkrankungen,
c) Störungen der neuronalen Regeneration, des axonalen Sproutings, der Neuritenextension und der neuronalen Plastizität,
d) Tumorerkrankungen und Tumormetastasierung

Gegenstand der Erfindung ist weiterhin die Verwendung einer Neogenin-Rezeptorbindenden Domäne nach obiger Definition oder eines Polypeptid-Fragments nach obiger Definition zur Herstellung eines Mittels zur Diagnose oder Therapie von Krankheiten oder Krankheitsstadien welche durch eine gestörte Wechselwirkung von RGM oder einem RGM-Fragment mit dem zugeordneten Rezeptor (wie z.B. dem Neogenin- Rezeptor) mediiert werden. Insbesondere sind diese Krankheiten oder Krankheitsstadien ausgewählt unter:
a) veränderten Neuritogeneseprozessen bei psychotischen Erkrankungen und chronischen Schmerzzuständen die durch exzessive Neuritensprossung und/oder pathologische Synaptogenese hervorgerufen werden.
b) Erkrankungen, assoziiert mit einem gestörten Eisenstoffwechsel, insbesondere juvenile Hemochromatose
c) Erkrankungen, assoziiert mit einem gestörten Knochenwachstum
d) Erkrankungen, assoziiert mit degenerativen Knorpelveränderungen
e) Erkrankungen, assoziiert mit Bandscheiben- und Wirbelkörperschäden
f) Erkrankungen, assoziiert mit deregulierten, unkontrollierten Zellmigrationsvorgängen.

Ein weiterer Gegenstand der Erfindung betrifft die Neogenin-Rezeptor-bindende Domäne nach obiger Definition oder eines Polypeptid-Fragments nach obiger Definition zur Verwendung als Target zum Nachweis oder zur Identifizierung von RGMbindenden Liganden.

Ein anderer Gegenstand der Erfindung betrifft die Neogenin-Rezeptor-bindende Domäne nach obiger Definition oder eines Polypeptid-Fragments nach obiger Definition zur Verwendung als Immunogen zur aktiven oder passiven Immunisierung.

Gegenstand der Erfindung sind auch polyklonale Antiseren, erhältlich durch Immunisierung eines Säugers mit einer antigenen Menge einer Neogenin-Rezeptor-bindenden Domäne nach obiger Definition oder eines Polypeptid-Fragments nach obiger Definition; sowie monoklonale Antikörper gegen eine Neogenin-Rezeptor-bindende Domäne nach obiger Definition oder gegen ein Polypeptid-Fragment nach obiger Definition, sowie Antigen-bindende Fragmente davon, gegebenenfalls in humanisierter Form.

Gegenstand der Erfindung sind auch pharmazeutische Mittel, umfassend in einem pharmazeutisch verträglichen Träger wenigstens einen aktiven Bestandteil ausgewählt unter:
a) einer Neogenin-Rezeptor-bindenden Domäne nach obiger Definition oder einem Polypeptid-Fragment nach obiger Definition oder
b) monoklonalen oder polyklonalen Antikörpern nach obiger Definition.

Solche pharmazeutischen Mittel dienen insbesondere zur intrathecalen, intravenösen, subkutanen, oralen oder parenteralen, nasalen und Inhalations-Verabreichung.

Gegenstand der Erfindung ist weiterhin ein Expressionsvektor, umfassend wenigstens eine kodierende Nukleinsäuresequenz für eine Neogenin-Rezeptor-bindende Domäne nach obiger Definition oder für ein Polypeptid-Fragment nach obiger Definition, operativ verknüpft mit wenigstens einer regulativen Nukleinsäuresequenz.

Weiterhin betrifft die Erfindung:
- Rekombinante Mikroorganismen, welche wenigstes einen Vektor nach obiger Definition tragen.
- Hybridomzelllinien, welche einen monoklonalen Antikörper nach obiger Definition produzieren.
- Verfahren zur Herstellung einer Neogenin-Rezeptor-bindenden Domäne nach obiger Definition oder eines Polypeptid-Fragments nach obiger Definition, wobei man einen rekombinanten Mikroorganismus nach obiger Definition kultiviert und das produzierte Proteinprodukt aus der Kultur isoliert. Dabei wird eine Hybridomzelllinie nach obiger Definition kultiviert und das produzierte Proteinprodukt aus der Kultur isoliert.

### III. Weitere Angaben zur Ausführung der Erfindung

### 1. Polypeptide

Gegenstand der Erfindung sind insbesondere die in den Ansprüchen 1 und 2 spezifizierten Bindungsdomänen von Proteinen der RGM Familie und von diesen Domänen abgeleitete Peptidfragmente. Während erfindungsgemäß insbesondere RGM A und dessen Bindungsdomäne und davon abgeleitete Fragmente untersucht wurden, sind Gegenstand der Erfindung auch entsprechende Domänen und Fragmente homologer Proteine, wie insbesondere homologer Mitglieder der RGM Familie, wie insbesondere RGM B und RGM C.

"Funktionale Äquivalente" oder Analoga der konkret offenbarten RGM-Domänen oder Polypeptide sind im Rahmen der vorliegenden Erfindung davon verschiedene Polypeptide, wie z. B. solche mit einem Homologiegrad von weniger als 100% und wenigstens 75% zu den Neogenin-Bindungsdomänen von Proteinen gemäß SEQ ID NO: 2, 4 oder 6, welche aber weiterhin die gewünschte biologische Aktivität besitzen. Insbesondere sollten diese zur Bindung an den Neogenin-Rezeptor befähigt sein und/oder in einem hierin beschriebenen Nervenfaserwachstumstest einen inhibitorischen Effekt zeigen und dabei das Nervenfaserwachstum teilweise oder vollständig, statistisch signifikant (p <= 0,05), inhibieren.

Unter "funktionalen Äquivalenten" versteht man erfindungsgemäß insbesondere Mutanten, welche in wenigstens einer der Sequenzpositionen der oben genannten konkreten Sequenzen eine andere als die konkret genannte Aminosäure aufweisen aber trotzdem eine der hierin genannten biologischen Aktivitäten besitzen. "Funktionale Äquivalente" umfassen somit die durch eine oder mehrere Aminosäure-Additionen, -Substitutionen, - Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche biologische Effekte nur unterschiedlich stark ausgeprägt zu beobachten sind. Beispiele für geeignete Substitutionen von Aminosäureresten sind folgende:

| **Ursprünglicher Rest** | **Beispiele der Substitution** |
|---|---|
| Ala | Ser |
| Arg | Lys |
| Asn | Gin; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch Präkursoren der beschriebenen Polypeptide sowie funktionale Derivate und Salze der Polypeptide. Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung. "Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen, zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" sind außerdem Fusionsproteine, welche ein der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Enzyme und Immunoglobuline.

Erfindungsgemäß mit umfasste "funktionale Äquivalente" sind Homologe zu den konkret offenbarten Proteinen bzw. Peptiden. Diese besitzen wenigstens 75% oder insbesondere wenigsten 85 %, wie z.B. 90%, 95% oder 99%, Homologie zu einer der konkret offenbarten Sequenzen, z.B. berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Unter einer "abgeleiteten" Aminosäuresequenz wird erfindungsgemäß, wenn keine anderen Angaben gemacht werden, eine Sequenz verstanden, die mit der Ausgangssequenz eine Identität von mindestens 80% oder mindestens 90%, insbesondere 91%, 92%, 93%, 94%, 95%, 96%, 97%, 98% und 99% aufweist.

Unter "Identität" zwischen zwei Sequenzen wird Identität der Aminosäurereste über die jeweils gesamte Sequenzlänge verstanden, wie z.B. die Identität, die durch Vergleich mit Hilfe der Vector NTI Suite 7.1 Software der Firma Informax (USA) unter Anwendung oder Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| FAST algorithm | on |
|---|---|
| K-tuple size | 1 |
| Gap penalty | 3 |
| Window size | 5 |
| Number of best diagonals | 5 |

Zur weiteren Veranschaulichung erfindungsgemäß umfasster Homologer wird auf folgende Tabelle verwiesen, welche die prozentuale Übereinstimmung von RGM B und C mit RGM A für das Volllänge-Protein sowie für bestimmte erfindungsgemäß besonders interessierende Teilsequenzbereiche zeigt.

| | RGM A - RGM B | RGM A - RGMC |
|---|---|---|
| Volllänge-Proteine | 51%²⁾³⁾ | 47% |
| AS¹⁾ 200 - 350 | 62%²⁾ | 55% |
| AS 200 - 325 | 61% | 55% |
| AS 200 - 300 | 66% | 60% |
| AS 200 - 285 | 65% | 66% |
| AS 250 - 285 | 58% | 61% |
| AS 260 - 285 | 57% | 78% |

| | | |
|---|---|---|
| 1) Aminosäurerest, Positionsangabe bezogen auf RGM A Sequenz (SEQ ID NO:2) 2) Aminosäureidentität der Volllänge-Proteine und der Peptidfragmente in Prozent 3) Methode: BLAST 2 SEQUENCES VERSION BLASTP 2.2.5 [Nov-16-2002] Matrix: Blosum62 (gap open: 11 gap extension: 1) | | |

Im Falle einer möglichen Proteinglykosylierung umfassen die erfindungsgemäßen Äquivalente Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Peptide können durch Screening kombinatorischer Banken von Mutanten, wie z.B. Verkürzungsmutanten, identifiziert werden. Beispielsweise kann eine variegierte Bank von Peptid-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen codieren. Verfahren zur Synthese degenerierter Oligonukleotide sind dem Fachmann bekannt (Z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

### 2. Nukleinsäuren

Gegenstand der Erfindung sind weiterhin die kodierenden Nukleinsäuresequenzen für die oben beschriebenen RGM-Bindungsdomänen und -Polypetide sowie davon abgeleitete Nukleinsäuresequenzen oder -teilsequenzen.

Alle erfindungsgemäßen Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Unter einer "abgeleiteten" Nukleinsäuresequenz wird erfindungsgemäß, wenn keine anderen Angaben gemacht werden, eine Sequenz verstanden, die mit der Ausgangssequenz eine Identität von mindestens 80% oder mindestens 90%, insbesondere 91%, 92%, 93%, 94%, 95%, 96%, 97%, 98% und 99% aufweist.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (siehe oben) ermittelt wird.

Gegenstand der Erfindung sind auch Nukleinsäuresequenzen, kodierend für eines der obigen Peptide und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Peptide oder biologisch aktive Abschnitte davon kodieren, sowie Nukleinsäurefragmente, die z.B. als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequertzen vom 3'- und/oder 5'-Ende des kodierenden Genbereichs enthalten.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der erfindungsgemäßen Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die Erfindung umfasst weiterhin die zu den konkret beschriebenen Nukleotidsequenzen komplementären Nukleinsäuremoleküle oder einen Abschnitt davon.

Die erfindungsgemäß Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Weitere erfindungsgemäße Nukleinsäuresequenzen sind abgeleitet von kodierenden Sequenzen zu den erfindungsgemäßen RGM-Domänen und -Peptiden und unterscheiden sich davon durch Addition, Substitution, Insertion oder Deletion einzelner oder mehrerer Nukleotide, kodieren aber weiterhin für Peptide mit dem gewünschten Eigenschaftsprofil.

Erfindungsgemäß umfasst sind auch solche Nukleinsäuresequenzen, die sogenannte stumme Mutationen umfassen oder entsprechend der Codon-Nutzung eines speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon. Gegenstand sind ebenso durch konservative Nukleotid-Substitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung ist auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Weiterhin umfasst die Erfindung auch Nukleinsäuresequenzen, welchen mit oben genannten kodierenden Sequenzen hybridisieren oder dazu komplementär sind. Diese Polynukleotide lassen sich bei Durchmusterung von genomischen oder cDNA-Banken auffinden und gegebenenfalls daraus mit geeigneten Primem mittels PCR vermehren und anschließend beispielsweise mit geeigneten Sonden isolieren. Eine weitere Möglichkeit bietet die Transformation geeigneter Mikroorganismen mit erfindungsgemäßen Polynukleotiden oder Vektoren, die Vermehrung der Mikroorganismen und damit der Polynukleotide und deren anschließende Isolierung. Darüber hinaus können erfindungsgemäße Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, insbesondere zu 90-100%, wie z.B. 95%, 96%, 97%, 98% oder 99%, komplementär sein. Die Eigenschaft komplementärer Sequenzen, spezifisch aneinander binden zu können, macht man sich beispielsweise in der Northem- oder Southem-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1 x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben.

Ein weiterer Aspekt der Erfindung betrifft "Antisense "Nukleinsäuren. Diese umfasst eine Nukleotidsequenz, die zu einer kodierenden "Sense-"Nukleinsäure, komplementär ist. Die Antisense-Nukleinsäure kann zum gesamten kodierenden Strang oder nur zu einem Abschnitt davon komplementär sein. Bei einer weiteren Ausführungsform ist das Antisense-Nukleinsäuremolekül antisense zu einem nicht-kodierenden Bereich des kodierenden Stranges einer Nukleotidsequenz. Der Begriff "nicht-kodierender Bereich" betrifft die als 5'- und 3'-untranslatierte Bereiche bezeichneten Sequenzabschnitte.

Ein Antisense-Oligonukleotid kann z.B. etwa 5, 10, 15, 20, 25, 30, 35, 40, 45 oder 50 Nukleotide lang sein. Eine erfindungsgemäße Antisense-Nukleinsäure kann durch chemische Synthese und enzymatische Ligationsreaktionen mittels im Fachgebiet bekannter Verfahren konstruiert werden. Eine Antisense-Nukleinsäure kann chemisch synthetisiert werden, wobei natürlich vorkommende Nukleotide oder verschieden modifizierte Nukleotide verwendet werden, die so gestaltet sind, dass sie die biologische Stabilität der Moleküle erhöhen, oder die physikalische Stabilität des Duplexes erhöhen, der zwischen der Antisense- und Sense-Nukleinsäure entstanden ist. Beispielsweise können Phosphorthioat-Derivate und acridinsubstituierte Nukleotide verwendet werden. Beispiele modifizierter Nukleotide, die zur Erzeugung der AntisenseNukleinsäure verwendet werden können, sind u.a. 5-Fluoruracil, 5-Bromuracil, 5-Chloruracil, 5-loduracil, Hypoxanthin, Xanthin, 4-Acetylcytosin, 5-(Carboxyhydroxylmethyl)uracil, 5-Carboxymethylaminomethyl-2-thiouridin, 5-Carboxymethylaminomethyluracil, Dihydrouracil, Beta-D-Galactosylqueuosin, Inosin, N6-lsopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, Beta-D-Mannosylqueosin, 5'-Methoxycarboxymethyluracil, 5-Methoxyuracil, 2-Methylthio-N6-isopentenyladenin, Uracil-5-oxyessigsäure (v), Wybutoxosin, Pseudouracil, Queuosin, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure (v), 5-Methyl-2-thiouracil, 3-(3-Amino-3-N-2-carboxypropyl)uracil, (acp3)w und 2,6-Diaminopurin. Die Antisense-Nukleinsäure kann auch biologisch hergestellt werden, indem ein Expressionsvektor verwendet wird, in den eine Nukleinsäure in Antisense-Richtung subkloniert worden ist.

### 3. Expressionskonstrukte und Vektoren

Gegenstand der Erfindung sind außerdem Expressionskonstrukte, enthaltend unter der genetischen Kontrolle regulativer Nukleinsäuresequenzen eine für ein erfindungsgemäßes RGM-Protein oder funktionales Äquivalent oder Immunglobulin kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, lpp-lac-, laclq-, T7-, T5-, T3-, gal-, trc-, ara-, SP6-, lambda-PR- oder lambda-PL-Promotor, die vorteilhafterweise in gram-negativen Bakterien Anwendung finden; sowie die grampositiven Promotoren amy und SPO2, die Hefepromotoren ADC1, MFalpha , AC, P-60, CYC1, GAPDH oder die Pflanzenpromotoren CaMV/35S, SSU, OCS, lib4, usp, STLS1, B33, not oder der Ubiquitin- oder Phaseolin-Promotor. Besonders bevorzugt ist die Verwendung induzierbarer Promotoren, wie z.B. licht- und insbesondere temperaturinduzierbarer Promotoren, wie der PᵣPₗ-Promotor. Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekanntten Vektoren, wie beispielsweise Phagen, Viren, wie SV40, CMV, Baculovirus und A-denovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Beispiele für geeignete Expressionsvektoren können genannt werden:
Übliche Fusionsexpressionsvektoren, wie pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird.

Nicht-Fusionsprotein-Expressionsvektoren wie pTrc (Amann et al., (1988) Gene 69:301-315) und pET 11 d (Studier et al. Gene Expression Technology: Methods in Enzymology 185, Academic Press, San Diego, Kalifornien (1990) 60-89).

Hefe-Expressionsvektor zur Expression in der Hefe S. *cerevisiae,* wie pYepSec1 (Baldari et al., (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge. Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3:2156-2165) und die pVL-Reihe (Lucklow und Summers (1989) Virology 170:31-39).

Pflanzen-Expressionsvektoren, wie solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721.

Säugetier-Expressionsvektoren, wie pCDM8 (Seed, B. (1987) Nature 329:840) und pMT2PC (Kaufman et al. (1987) EMBO J. 6:187-195).

Weitere geeignete Expressionssysteme für prokaryontische und eukaryotische Zellen sind in Kapitel 16 und 17 von Sambrook, J., Fritsch, E.F. und Maniatis, T., Molecular cloning: A Laboratory Manual. 2. Auflage, Cold Spring Harbor Laboratory, Cold Spring Harbor Laboratory Press, Cold Spring Harbor, NY, 1989 beschrieben.

### 4. Rekombinante Wirtsorganismen

Mit Hilfe der erfindungsgemäßen Vektoren sind rekombinante Organismen herstellbar, welche beispielsweise mit wenigstens einem erfindungsgemäßen Vektor transformiert sind und zur Produktion der erfindungsgemäßen Domänen oder Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z. B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9-, CHO- oder HEK293-Zellen.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Gewünschtenfalls kann das Genprodukt auch in transgenen Organismen, wie transgenen Tieren, insbesondere Mäusen, Schafen oder transgenen Pflanzen zur Expression, gebracht werden.

Gegenstand der Erfindung sind weiterhin Verfahren zur rekombinanten Herstellung erfindungsgemäßer RGM-Domänen oder -Polypeptide oder funktioneller, biologisch aktiver Fragmente davon, wobei man einen Peptide-produzierenden rekombinanten Wirtsorganismus kultiviert, gegebenenfalls die Expression der Polypeptide induziert und diese aus der Kultur isoliert. Die Peptide können so auch in großtechnischem Maßstab produziert werden, falls dies erwünscht ist.

Der rekombinante Wirt kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Peptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, T. G., Biochemische Arbeitsmethoden, Verlag Walter de Gruyter, Berlin, New York oder in Scopes, R., Protein Purification, Springer Verlag, New York, Heidelberg, Berlin beschrieben.

Besonders geeignet ist es, zur Isolierung des rekombinanten Peptids Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Peptide an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Peptide verwendet werden. Zur Erkennung der Peptidekönnen außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Peptide verwendet werden.

### 5. Immunglobuline

### 5.1 Definition

Gegenstand der vorliegenden Erfindung sind monoklonale oder polyklonale Antikörper, die an ein erfindungsgemäßes RGM-Protein oder Derivat/Äquivalent davon spezifisch binden, d.h. Antikörper mit Spezifität für ein erfindungsgemäßes RGM-Protein oder Derivat/Äquivalent davon. Gegenstand der vorliegenden Erfindung sind auch Teile dieser Antikörper, insbesondere antigenbindende Teile davon, d.h. Antikörperfragmente, die ein erfindungsgemäßes RGM-Protein oder ein Derivat/Äquivalent davon binden. Vorzugsweise wählt man den erfindungsgemäßen Antikörper so, dass er eine bestimmte Bindungskinetik aufweist (z. B. hohe Affinität, geringe Dissoziation, niedrige off-Geschwindigkeit (k_{off}), starke neutralisierende Aktivität) für die spezifische Bindung an erfindungsgemäßes RGM-Protein oder Derivat/Äquivalent davon.

So können Antikörper mit einer Affinität für das erfindungsgemäße RGM-Protein oder Derivat/Äquivalent davon im Bereich von K_{D}=10⁻⁶-10⁻¹² M bereitgestellt werden.

Einem weiteren Aspekt zufolge kann man die erfindungsgemäßen Antikörper so wählen, dass sie das RGM-Protein oder Derivat/Äquivalent davon mit einer k_{off}-Geschwindigkeitskonstanten von 0,1 1 s⁻¹ oder weniger binden.

Bei den Antikörpern handelt es sich vorzugsweise um isolierte Antikörper. Einem weiteren Aspekt zufolge sind die Antikörper neutralisierende Antikörper. Zu den erfindungsgemäßen Antikörpern gehören insbesondere monoklonale und rekombinante Antikörper. Der erfindungsgemäße Antikörper kann eine Aminosäuresequenz umfassen, die vollständig von einer einzigen Spezies abstammt, kann also z.B. ein humaner Antikörper oder ein Mausantikörper sein. Weiteren Ausführungsformen zufolge kann der Antikörper ein chimärer Antikörper oder ein CDR-Graft-Antikörper oder eine andere Form eines humanisierten Antikörpers sein.

Der Begriff "Antikörper" soll sich auf Immunglobulinmoleküle beziehen, die aus 4 Polypeptidketten, zwei schweren (H) Ketten und zwei leichten (L) Ketten, gebildet sind. Die Ketten sind in der Regel durch Disulfid-Bindungen miteinander verknüpft. Jede schwere Kette setzt sich aus einer variablen Region der schweren Kette (hier als HCVR oder VH abgekürzt) und einer konstanten Region der schweren Kette zusammen. Die konstante Region der schweren Kette wird aus drei Domänen CH1, CH2 und CH3 gebildet. Jede leichte Kette setzt sich aus einer variablen Region der leichten Kette (hier als LCVR oder VL abgekürzt) und einer konstanten Region der leichten Kette zusammen. Die konstante Region der leichten Kette wird aus einer Domäne CL gebildet. Die VH- und VL-Regionen können weiter unterteilt werden in hypervariable Regionen, die als komplementaritätsbestimmende Regionen (CDR für Complementarity Determining Region) bezeichnet werden und mit konservierteren Regionen, die als Gerüstregionen (FR für Frame Work Region) bezeichnet werden, durchsetzt sind. Jede VH- und VL-Region wird aus drei CDRs und vier FRs gebildet, die vom N-Terminus zum C-Terminus in folgender Reihenfolge angeordnet sind: FR1, CDR1, FR2, CDR2, FR3, CDR3, FR4.

Der Begriff "antigenbindender Teil" eines Antikörpers (oder einfach "Antikörperteil") bezieht sich auf ein oder mehrere Fragmente eines Antikörpers mit Spezifität für ein erfindungsgemäßes RGM-Protein oder Derivat/Äquivalent davon, wobei das oder die Fragmente nach wie vor die Fähigkeit besitzen, das RGM-Protein oder Derivat/Äquivalent davon spezifisch zu binden. Es ist gezeigt worden, dass die antigenbindende Funktion eines Antikörpers von Fragmenten eines vollständigen Antikörpers wahrgenommen werden können. Zu Beispielen bindender Fragmente gehören im Sinne des Begriffs "antigenbindender Teil" eines Antikörpers (i) ein Fab-Fragment, d.h. ein aus den VL-, VH-, CL- und CH1-Domänen zusammengesetztes monovalentes Fragment; (ii) ein F(ab')₂-Fragment, d.h. ein bivalentes Fragment, welches zwei in der Hinge-Region über eine Disulfid-Brücke miteinander verknüpfte Fab-Fragmente beinhaltet; (iii) ein Fd-Fragment, das sich aus den VH- und CH1-Domänen zusammengesetzt; (iv) ein Fv-Fragment, das sich aus den VL- und VH-Domänen eines einzelnen Arms eines Antikörpers zusammengesetzt; (v) ein dAb-Fragment (Ward et al., (1989) Nature 341:544-546 ), das aus einer VH-Domäne oder VH, CH1, CH2, DH3, oder VH, CH2, CH3 besteht; und (vi) eine isolierte komplementaritätsbestimmente Region (CDR). Wenngleich die beiden Domänen des Fv-Fragments, nämlich VL und VH, von getrennten Genen kodiert sind, können sie des weiteren mit einem synthetischen Linker unter Verwendung rekombinanter Verfahren miteinander verbunden werden, wodurch sie als eine einzige Proteinkette hergestellt werden können, worin die VL- und VH-Regionen sich zusammenfinden, um monovalente Moleküle zu bilden (als Einzelkette-Fv (ScFv) gekannt; siehe z.B. Bird et al. (1988) Science 242:423-426; und Huston et al. (1988) Proc. Natl. Acad. Sci. USA 85:5879-5883). Derartige Einzelketten-Antikörper sollen auch von dem Begriff "antigenbindender Teil" eines Antikörpers erfasst werden. Andere Formen von Einzelketten-Antikörpern wie "Diabodies" gehören ebenfalls dazu. Diabodies sind bivalente, bispezifische Antikörper, in denen VH- und VL-Domänen auf einer einzelnen Polypeptidkette exprimiert sind, wobei allerdings ein Linker verwendet wird, der zu kurz ist, als dass sich die beiden Domänen auf derselben Kette zusammenfinden können, wodurch man die Domänen zwingt, mit komplementären Domänen einer anderen Kette zu paaren und zwei antigenbindende Stellen zubilden (siehe z.B. Holliger, P., et al. (1993) Proc. Natl. Acad. Sci. USA 90:6444-6448; Poljak, R.J., et al. (1994) Structure 2:1121-1123).

Des weiteren kann ein Antikörper oder antigenbindender Teil davon Teil eines größeren Immunadhäsionsmoleküls sein, das durch kovalente oder nicht kovalente Assoziierung des Antikörpers oder Antikörperteils mit einem oder mehreren weiteren Proteinen oder Peptiden gebildet wird. Zu solchen Immunadhäsionsmolekülen gehört die Verwendung der Streptavidin-Kernregion, um ein tetrameres scFv-Molekül herzustellen (Kipriyanov, S.M., et al. (1995) Human Antibodies und Hybridomas 6:93-101) und die Verwendung eines Cysteinrestes, eines Markerpeptids und eines C-terminalen Polyhistidin-Tags, um bivalente und biotinylierte scFv-Moleküle zu machen (Kipriyanov, S.M., et al. (1994) Mol. Immunol. 31:1047-1058).

Antikörperteile, wie Fab und F(ab')₂-Fragmente, können aus ganzen Antikörpern hergestellt werden, indem man konventionelle Techniken verwendet, wie die Verdauung mit Papain bzw. Pepsin. Darüber hinaus können Antikörper, Antikörperteile und Immunadhäsionsmoleküle erhalten werden, indem man standardisierte rekombinante DNA-Techniken verwendet. Ein "isolierter Antikörper mit Spezifität für ein erfindungsgemäßes RGM-Protein oder Derivat/Äquivalent davon" umschreibt einen Antikörper mit Spezifität für ein erfindungsgemäßes RGM-Protein oder Derivat/Äquivalent davon, der im wesentlichen frei von anderen Antikörpern mit unterschiedlichen Antigenspezifitäten ist.

Der Begriff "neutralisierender Antikörper" beschreibt einen Antikörper, dessen Bindung an ein bestimmtes Antigen zur Inhibierung der biologischen Aktivität des Antigens führt. Diese Inhibierung der biologischen Aktivität des Antigens kann beurteilt werden, indem man einen oder mehrere Indikatoren für die biologische Aktivität des Antigens misst, wobei man einen geeigneten in vitro- oder in vivo-Assay verwendet.

Der Begriff "monoklonaler Antikörper" beschreibt einen Antikörper, der von einem Hybridom abstammt (z. B. ein Antikörper, der von einem mittels Hybridomtechnologie, wie der standardisierten Hybridommethodik nach Köhler und Milstein, hergestellten Hybridom sekretiert wird). Ein von einem Hybridom abstammender Antikörper mit Spezifität für ein erfindungsgemäßes RGM-Protein oder Derivat/Äquivalent davon wird daher als monoklonaler Antikörper bezeichnet.

Der Begriff "rekombinanter Antikörper" beschreibt Antikörper, die mit rekombinanten Mitteln hergestellt, exprimiert, erzeugt oder isoliert werden, wie Antikörper, die unter Verwendung eines in eine Wirtszelle transfizierten, rekombinanten Expressionsvektors exprimiert werden; Antikörper, die aus einer rekombinanten kombinatorischen Antikörperbank isoliert sind; Antikörper, die aus einem Tier (z. B. einer Maus), das durch humane Immunglobulingene transgen ist, isoliert sind (siehe z. B. Taylor, L.D., et al. (1992) Nucl. Acids Res. 20:6287-6295); oder Antikörper, die auf irgend eine andere Weise, bei der bestimmte Immunoglobulin-Gensequenzen (wie humane Immunglobulin-Gensequenzen) mit anderen DNA-Sequenzen zusammengesetzt werden, hergestellt, exprimiert, erzeugt oder isoliert werden. Zu rekombinanten Antikörpern gehören beispielsweise chimäre, CDR-Graft- und humanisierte Antikörper.

Der Begriff "humaner Antikörper" beschreibt Antikörper, deren variable und konstante Regionen Immunglobulinsequenzen der Humankeimbahn, wie beispielsweise von Kabat *et al*. (siehe Kabat, et al. (1991) Sequences of Proteins of Immunological Interest, Fifth Edition, U.S. Department of Health und Human Services, NIH Publication Nr. 91-3242) beschrieben, entsprechen oder davon abstammen. Die erfindungsgemäßen humanen Antikörper können allerdings Aminosäurereste beinhalten, die nicht von humanen Keimbahn-Immunglobulinsequenzen kodiert sind (z. B. Mutationen, die durch zufällige oder ortsspezifische Mutagenese in vitro oder durch somatische Mutation in vivo eingeführt sind), beispielsweise in den CDRs, und insbesondere in CDR3. Erfindungsgemäße rekombinante humane Antikörper haben variable Regionen und können auch konstante Regionen beinhalten, die von Immunglobulinsequenzen der Humankeimbahn abstammen (siehe Kabat, E.A., et al. (1991) Sequences of Proteins of Immunological Interest, Fifth Edition, U.S. Department of Health und Human Services, NIH Publication Nr. 91-3242). Bestimmten Ausführungsformen zufolge werden derartige rekombinante humane Antikörper allerdings einer in vitro-Mutagenese (oder falls ein durch humane Ig-Sequenzen transgenes Tier verwendet wird, einer somatischen in vivo-Mutagenese) unterzogen, so dass die Aminosäuresequenzen der VH- und VL-Regionen der rekombinanten Antikörper Sequenzen sind, die, wenngleich sie mit VH- und VL-Sequenzen der Humankeimbahn verwandt sind oder davon abstammen, innerhalb des humanen Antikörper-Keimbahnrepertoirs in vivo natürlicherweise nicht existieren. Bestimmten Ausführungsformen zufolge sind derartige rekombinante Antikörper das Resultat einer selektiven Mutagenese oder Rückmutation, oder beides.

Der Begriff "Rückmutation" bezieht sich auf ein Verfahren, bei dem einige oder sämtliche der somatisch mutierten Aminosäuren eines humanen Antikörpers mit den entsprechenden Keimbahnresten einer homologen Keimbahnantikörpersequenz ersetzt werden. Die Sequenzen für die schwere und leichte Kette eines erfindungsgemäßen humanen Antikörpers werden getrennt mit den Keimbahnsequenzen in der VBASE-Datenbank verglichen, um die Sequenzen mit der größten Homologie zu identifizieren. Abweichungen im erfindungsgemäßen humanen Antikörper werden auf die Keimbahnsequenz zurückgeführt, indem man an definierten Nukleotidpositionen, die solche abweichenden Aminosäuren kodieren, mutiert. Die direkte oder indirekte Bedeutung jeder auf diese Weise als Kandidat für eine Rückmutation identifizierten Aminosäure für die Antigenbindung sollte untersucht werden, und eine Aminosäure, die nach Mutation eine wünschenswerte Eigenschaft des humanen Antikörpers beeinträchtigt, sollte in den endgültigen humanen Antikörper nicht miteinbezogen werden. Um die Anzahl der Aminosäuren für eine Rückmutation so gering wie möglich zu halten, können diejenigen Aminosäurepositionen unverändert bleiben, die zwar von der am nächsten kommenden Keimbahnsequenz abweichen aber mit der entsprechenden Aminosäuresequenz einer zweiten Keimbahnsequenz identisch sind, vorausgesetzt, daß die zweite Keimbahnsequenz mit der Sequenz des erfindungsgemäßen humanen Antikörpers zumindest in 10 und vorzugsweise in 12 Aminosäuren auf beiden Seiten der fraglichen Aminosäure identisch und co-linear ist. Rückmutationen können auf beliebiger Stufe der Antikörperoptimierung vorgenommen werden.

Der Begriff "chimärer Antikörper" umfasst Antikörper, in denen einzelne Teile des Moleküls aus verschiedenen Spezies abgeleitet sind. So sind chimäre Antikörper, ohne darauf beschränkt zu sein, z.B. Antikörper, die Sequenzen für die variable Region der schweren und leichten Kette aus einer Spezies beinhalten, in denen aber die Sequenzen einer oder mehrerer der CDR-Regionen aus VH und/oder VL mit CDR-Sequenzen einer anderen Spezies ersetzt sind. In solchen Antikörpern können die variablen Regionen der schweren und leichten Kette aus Maus aufweisen, in denen eine oder mehrere der Maus-CDRs (z. B. CDR3) durch humane CDR-Sequenzen ersetzt sind.

Der Begriff "humanisierter Antikörper" beschreibt Antikörper, die Sequenzen der variablen Region schwerer und leichter Kette aus einer nichthumanen Spezies (z. B. Maus, Ratte, Kaninchen, Huhn, Kamelid, Ziege) beinhalten, in denen jedoch wenigstens ein Teil der VH- und/oder VL-Sequenz verändert worden ist, um "humanähnlicher" zu sein, d. h. variablen Sequenzen der humanen Keimbahn ähnlicher zu sein. Eine Art eines humanisierten Antikörpers ist ein CDR-Graft-Antikörper, bei dem humane CDR-Sequenzen in nichthumane VH- und VL-Sequenzen eingesetzt sind, um die entsprechenden nichthumanen CDR-Sequenzen zu ersetzen.

Eine Methode, die Bindungskinetik eines Antikörpers zu messen, basiert auf der sogenannten Oberflächenplasmonräsonanz. Der Begriff "Oberflächenplasmonresonanz" bezieht sich auf ein optisches Phänomen, mit dem sich biospezifische Wechselwirkungen analysieren lassen, indem man Veränderungen von Proteinkonzentrationen mit einer Biosensormatrix nachweist, wobei man beispielsweise das BIAcore-System verwendet (Pharmacia Biosensor AB, Uppsala, Sweden und Piscataway, NJ). Für weitere Beschreibungen siehe Jönsson, U., et al. (1993) Ann. Biol. Clin. 51:19-26; Jönsson, U., et al. (1991) Biotechniques 11:620-627; Johnsson, B., et al. (1995) J. Mol. Recognit. 8:125-131; und Johnnson, B., et al. (1991) Anal. Biochem. 198:268-277.

Der Begriff "K_{off}" beschreibt die off-Geschwindigkeitskonstante für die Dissoziation eines Antikörpers aus dem Antikörper/Antigen-Komplex.

Der Begriff "K_{d}" beschreibt die Dissoziationskonstante einer bestimmten Antikörper-Antigen-Wechselwirkung.

Die Bindungsaffinität der erfindungsgemäßen Antikörper kann beurteilt werden, indem man standardisierte in vitro-Immunoassays, wie ELISA- oder BIAcore-Analysen, verwendet.

### 5.2 Herstellung von Immunglobulinen

### 5.2.1 Herstellung von polyklonalen Antikörpern

Die vorliegende Erfindung betrifft polyklonale Antikörper gegen erfindungsgemäße RGM-Domänen und -Polypeptide und deren Herstellung.

Dazu wird ein Wirt mit wenigstens einem erfindungsgemäßen RGM-Protein oder Derivat/Äquivalent davon immunisiert; und ein als Antwort auf die Immunisierung gebildetes Antikörper-haltiges Serum des Wirts gewonnen.

Sind die zu verwendenden RGM-Polypetide nicht oder nur schwach immunogen, so kann man ihre Immunogenität dadurch erhöhen, daß man sie an Träger, vorzugsweise ein Trägerprotein, wie Keyhole Limpet Hemocyanin (KLH), Limulus Polyphemus Hemocyanin (LPH), Rinderserumalbumin (BSA) oder Ovalbumin (OVA), koppelt. Dazu stehen dem Fachmann eine Reihe von Kopplungsmöglichkeiten zur Verfügung, die allgemein bekannt sind. Zweckmäßig kann beispielsweise die Umsetzung mit Glutardialdehyd sein, beispielsweise durch Inkubation von RGM-Protein mit einem geeigneten Peptid oder Peptid-Gemisch in Wasser oder einem wässrigen Lösungsmittel. Diese Umsetzung kann bequemerweise bei Umgebungstemperatur, also in der Regel Raumtemperatur, durchgeführt werden. Es kann allerdings auch zweckmäßig sein, zu kühlen oder leicht zu erwärmten. In der Regel führt die Umsetzung innerhalb weniger Stunden zum erwünschten Ergebnis, eine Reaktionsdauer von beispielsweise 2 h liegt im üblichen Bereich. Die Glutardialdehyd-Konzentration liegt in der Regel im ppm- bis %-Bereich, zweckmäßigerweise von 10 ppm bis zu 1 %, vorzugsweise von 100 ppm bis 0,5 %. Die Optimierung der Reaktionsparameter liegt im Bereich des fachmännischen Könnens.

Zusätzlich zum Antigen enthalten die Zusammensetzungen in der Regel weitere Hilfsstoffe, insbesondere üblicherweise zur Immunisierung eingesetzte Adjuvantien, z.B. Freund-Adjuvanz. Insbesondere verwendet man komplettes Freund-Adjuvanz für die erste Immunisierung wohingegen alle weiteren Immunisierungen mit inkomplettem Freund-Adjuvanz durchgeführt werden. Zur Herstellung der Immunisierungscocktails wird das Antigen (Immunogen), vorzugsweise als oben beschriebenes Komponenten-Gemisch, zu dem oder den Hilfsstoffen gegeben. In der Regel wird dabei das Antigen emulgiert.

Als Wirt eignen sich insbesondere Nager oder auch Kaninchen. Diesen oder anderen geeigneten Wirten werden die Immunisierungscocktails, vorzugsweise subkutan, injiziert. Die Antikörpertiter können mit einem Immunoassay, beispielsweise kompetitiv mit einem gegen Wirt-IgG gerichteten Schaf-Antiserum und markiertem RGM-Protein, bestimmt werden. So kann gegen Ende der Immunisierung entschieden werden, ob ein bestimmter Wirt zur Antikörpergewinnung geeignet ist. Werden beispielsweise vier Immunisierungen durchgeführt, so kann man nach der dritten Immunisierung den Antikörpertiter bestimmen und dann aus Tieren, die einen ausreichenden Antikörpertiter aufweisen, Antikörper gewinnen.

Zur Gewinnung gebildeter Antikörper ist es bevorzugt, den Wirten über mehrere Wochen oder Monate Blut abzunehmen. Abschließend kann man den Wirt ausbluten lassen. Aus dem so gewonnenen Blut kann in an sich bekannter Weise Serum gewonnen werden, das die erwünschten Antikörper enthält. Das so erhaltene Vollserum kann erforderlichenfalls in fachmännischer Weise weiter aufgereinigt werden, um die darin enthaltene Antikörperfraktion und insbesondere die RGM-Protein-erkennenden Antikörper anzureichern.

Gemäß einer besonderen Ausführungsform dieses Verfahrens selektiert man wenigstens einen Antikörper des Serums, der das als Immunogen verwendete RGM-Protein oder ein Derivat/Äquivalent davon spezifisch erkennt. Spezifität meint in diesem Zusammenhang eine höhere Bindungsaffinität des Antikörpers für das Immunogen als für andere, insbesondere immunogen verwandte Proteine.

### 5.2.2 Herstellung von monoklonalen Antikörpern

Erfindungsgemäß brauchbare Immunglobulin sind unter Anwendung an sich bekannter Methoden erhältlich. So gestattet die Hybridomtechnologie die Herstellung von monospezifischen Antikörpern für ein interessierendes Antigen. Weiterhin sind rekombinante Antikörpertechniken, wie das in-vitro-Screenen von Antikörperbanken entwickelt worden, mit deren Hilfe sich ebenfalls derartige spezifische Antikörper herstellen lassen.

So kann man beispielsweise ein Tier mit dem interessierenden Antigen immunisieren. Dieser in-vivo-Ansatz kann des weiteren beinhalten, dass man aus den Lymphozyten oder Milzzellen eines Tieres eine Reihe von Hybridomen etabliert und ein Hybridom selektiert, das einen das Antigen spezifisch bindenden Antikörper sekretiert. Bei dem zu immunisierenden Tier kann es sich beispielsweise um eine Maus, Ratte, Kaninchen, Huhn, Kamelid oder Schaf handeln, oder um eine transgene Version eines der zuvor genannten Tiere, beispielsweise eine transgene Maus mit humanen Immunglobulingenen, die nach einem antigenen Stimulus humane Antikörper macht. Zu weiteren Typen von Tieren, die immunisiert werden können, zählen Mäuse mit schwerer kombinierter Immundefizienz (SCID), die mit humanen peripheren mononukleären Blutzellen (chimäre hu-PBMC-SCID-Mäuse) oder mit lymphoiden Zellen oder Vorläufern davon rekonstituiert worden sind, genauso wie Mäuse, die mit einer letalen Gesamtkörperbestrahlung behandelt, anschließend mit Knochenmarkszellen aus einer Maus mit schwerer kombinierter Immundefizienz (SCID) gegen Strahlung geschützt und anschließend mit funktionalen humanen Lymphozyten transplantiert worden sind (das sogenannte Trimera-System). Eine weiterer Typus eines zu immunisierenden Tieres ist ein Tier (z.B. eine Maus), in dessen Genom ein endogenes Gen, welches das interessierende Antigen kodiert, ausgeschaltet wurde ("knocked out"), z.B. durch homologe Rekombination, so dass dieses Tier nach Immunisierung mit dem Antigen das Antigen als fremd erkennt. Für den Fachmann ist klar, dass die mit diesen Verfahren hergestellten polyklonalen oder monoklonalen Antikörper charakterisiert und selektiert werden, indem man bekannte Screening-Verfahren verwendet, zu denen ELISA-Techniken gehören, ohne jedoch darauf beschränkt zu sein.

Einer weiteren Ausführungsform zufolge, screent man eine rekombinante Antikörperbank mit dem Antigen. Die rekombinante Antikörperbank kann beispielsweise auf der Oberfläche von Bakteriophagen oder auf der Oberfläche von Hefezellen oder auf der Oberfläche von bakteriellen Zellen exprimiert sein. Die rekombinante Antikörperbank kann beispielsweise eine scFv-Bank oder eine Fab-Bank sein. Gemäß einer weiteren Ausführungsform sind Antikörperbänke als RNA-Protein-Fusionen exprimierbar.

Ein weiterer Ansatz zur Herstellung erfindungsgemäßer Antikörper beinhaltet eine Kombination aus *in vivo-* und *in vitro*-Ansätzen. Beispielsweise kann man das Antigen auf das Antikörper-Repertoire einwirken lassen, indem man ein Tier mit dem Antigen *in vivo* immunisiert und anschließend eine aus lymphoiden Zellen des Tieres hergestellte rekombinante Antikörperbank oder Einzeldomänen-Antikörperbank (z.B. mit schwerer und/oder leichter Kette) mit dem Antigen *in vitro* screent. Einem weiteren Ansatz zufolge lässt man das Antigen auf das Antikörper-Repertoire einwirken, indem man ein Tier mit dem Antigen *in vivo* immunisiert und anschließend eine aus lymphoiden Zellen des Tieres hergestellte rekombinante Antikörperbank oder Einzeldomänen-Bank einer Affinitätsreifung unterzieht. Einem weiteren Ansatz zufolge lässt man das Antigen auf das Antikörper-Repertoire einwirken, indem man ein Tier mit dem Antigen *in vivo* immunisiert, anschließend einzelne Antikörper-produzierende Zellen, die einen interessierenden Antikörper sekretieren, selektiert und aus diesen selektierten Zellen cDNAs für die variable Region der schweren und leichten Kette gewinnt (z.B. mittels PCR) und die variablen Regionen der schweren und leichten Kette *in vitro* in Säugerwirtszellen exprimiert (was als das Lymphozyten-Antikörper-Selektionsverfahren, oder SLAM für "Selected Lymphocyte Antibody Method", bezeichnet wird), wodurch sich die selektierten Antikörper-Gensequenzen weiter selektieren und manipulieren lassen. Außerdem können monoklonale Antikörper durch Expressionsklonierung selektiert werden, indem man die Antikörpergene für die schwere und leichte Kette in Säugerzellen exprimiert und diejenigen Säugerzellen selektiert, die einen Antikörper mit der gewünschten Bindungsaffinität sekretieren.

Mit vorliegender Erfindung werden definierte Antigene in Form von RGM-Bindungsdomänen oder -Polypetiden zum Screenen und Gegenscreenen zur Verfügung gestellt. So können erfindungsgemäß diejenigen polyklonalen und monoklonalen Antikörper selektiert werden, die ein erfindungsgemäß gewünschtes Eigenschaftsprofil gemäß obiger Definition aufweisen.

Mit den erfindungsgemäßen Verfahren zur Herstellung von Antikörpern lassen sich verschiedene Antikörpertypen herstellen. Hierzu gehören im Wesentlichen humane Antikörper, chimäre Antikörper, humanisierte Antikörper und CDR-Graft-Antikörper sowie Antigen-bindende Teile davon.

Im Folgenden werden Verfahren zur Herstellung erfindungsgemäßer Antikörper beschrieben. Dabei wird zwischen In-vivo-Ansätzen, In-vitro-Ansätzen, oder einer Kombination aus beiden, unterschieden.

### In-vivo-Ansätze:

Ausgehend von den in-vivo erzeugten Antikörper produzierenden Zellen können monoklonale Antikörper mittels standardisierter Techniken, wie der ursprünglich von Köhler und Milstein (1975, Nature 256:495-497) (siehe auch Brown et al. (1981) J. Immunol 127:539-46; Brown et al. (1980) J Biol Chem 255:4980-83; Yeh et al. (1976) PNAS 76:2927-31; und Yeh et al. (1982) Int. J. Cancer 29:269-75) beschriebenen Hybridomtechnik, hergestellt werden. Die Technologie zur Produktion monoklonaler Antikörperhybridome ist hinlänglich bekannt (siehe allgemein R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet., 3:231-36). Eine immortalisierte Zelllinie (typischerweise ein Myelom) wird dazu mit Lymphozyten (typischerweise Splenozyten oder Lymphknotenzellen oder periphere Blutlymphozyten) eines mit dem erfindungsgemäßen RGM-Protein oder Derivat/Äquivalent davon immunisierten Säugers fusioniert, und die Kulturüberstände der resultierenden Hybridomzellen werden gescreent, um ein Hybridom zu identifizieren, das einen monoklonalen Antikörper mit Spezifität für erfindungsgemäßes RGM-Protein oder für ein Derivat/Äquivalent davon produziert. Dazu kann man ein beliebiges, von vielen hinlänglich bekannten Protokollen für die Fusion von Lymphozyten und immortalisierten Zelllinien anwenden (siehe auch G. Galfre et al. (1977) Nature 266:550-52; Gefter et al. Somatic Cell Genet.*,* cited *supra;* Lerner, Yale J. Biol. Med.*,* cited *supra*; Kenneth, Monoclonal Antibodies*,* cited *supra*). Darüber hinaus sind dem Fachmann mannigfaltige Variationen solcher Verfahren, die ebenfalls brauchbar sind, bekannt. Typischerweise stammten die immortalisierten Zelllinien (z.B. eine Myelomzelllinie) von der gleichen Säugerspezies ab wie die Lymphozyten. Beispielsweise kann man murine Hybridome etablieren, indem man Lymphozyten aus einer mit einer erfindungsgemäßen immunogenen Zubereitung immunisierten Maus mit einer immortalisierten Mauszelllinie fusioniert. Bevorzugte immortalisierte Zelllinien sind Maus-Myelomzelllinien, welche für Hypoxanthin, Aminopterin und Thymidin enthaltendes Kulturmedium (HAT-Medium) sensitiv sind. Man kann eine beliebige von vielen Myelomzelllinien als Fusionspartner standardmäßig verwenden, z.B. die P3-NS1/1-Ag4-1-, P3-x63-Ag8.653- or Sp2/O-Ag14-Myelomlinie. Diese Myelomzelllinien sind von der American Type Culture Collection (ATCC), Rockville, MD, erhältlich. Typischerweise werden HAT-sensitive Maus-Myelomzellen mit Maus-Splenozyten unter Verwendung von Polyethylenglykol (PEG) fusioniert. Die aus der Fusion resultierenden Hybridomzellen werden dann unter Verwendung von HAT-Medium selektiert, wodurch nicht fusionierte und nicht produktiv fusionierte Myelomzellen abgetötet werden (nicht fusionierte Splenozyten sterben nach mehreren Tagen ab, da sie nicht transformiert sind). Monoklonale Antikörper-produzierende Hybridomzellen, die ein erfindungsgemäßes RGM-Protein oder ein Derivat/Äquivalent davon spezifisch erkennen, werden identifiziert, indem man die Hybridomkulturüberstände auf solche Antikörper screent, z.B. indem man einen Standard-ELISA-Assay verwendet, um diejenigen Antikörper zu selektieren, die erfindungsgemäßes RGM-Protein oder ein Derivat/Äquivalent davon spezifisch binden können.

Je nach Art des gewünschten Antikörpers, können verschiedene Wirtstiere für die invivo-Immunisierung verwendet werden. Ein Wirt, der eine endogene Version des interessierenden Antigens selbst exprimiert, kann verwendet werden. Alternativ kann ein Wirt verwendet werden, der für eine endogene Version des interessierenden Antigens defizient gemacht wurde. Es wurde beispielsweise gezeigt, dass Mäuse, die über homologe Rekombination am entsprechenden endogenen Gen für ein bestimmtes endogenes Protein defizient gemacht wurden (d.h. Knockout-Mäuse) eine humorale Antwort gegen das Protein, mit dem sie immunisiert wurden, erzeugen und daher zur Herstellung hochaffiner monoklonaler Antikörper gegen das Protein verwendet werden können (siehe z.B. Roes, J. et al. (1995) J. Immunol. Methods 183:231-237; Lunn, M.P. et al. (2000) J. Neurochem. 75:404-412).

Für die Herstellung nicht humaner Antikörper gegen erfindungsgemäßes RGM-Protein oder ein Derivat/Äquivalent davon sind viele nicht-menschliche Säuger als Wirte für die Antikörperherstellung geeignet. Hierzu gehören Mäuse, Ratten, Hühner, Kamelide, Kaninchen und Ziegen (und Knockout-Versionen davon), wenngleich Mäuse zur Hybridomherstellung bevorzugt sind. Desweiteren kann man zur Herstellung im Wesentlichen humaner Antikörper gegen ein humanes Antigen mit dualer Spezifität ein nicht-humanes Wirtstier verwenden, das ein humanes Antikörper-Repertoire exprimiert. Zu solchen nicht-humanen Tieren gehören transgene Tiere (z.B. Mäuse), die humane Immunglobulin-Transgene tragen (chimäre hu-PBMC-SCID-Mäuse) und Mensch/Maus-Bestrahiungschimären, die nachstehend eingehender beschrieben sind.

Einer Ausführungsform zufolge ist das Tier, das mit einem erfindungsgemäßen RGM-Protein oder Derivat/Äquivalent davon immunisiert wird, ein nicht-humaner Säuger, vorzugsweise eine Maus, der durch humane Immunglobulingene transgen ist, so dass der nicht-humane Säuger nach einem antigenen Stimulus humane Antigkörper macht. Typischerweise werden in solche Tiere Immunglobulin-Transgene für schwere und leichte Kette mit humaner Keimbahnkonfiguration eingeführt, wobei die Tiere so verändert wurden, dass ihre endogenen Loci für schwere und leichte Kette inaktiv sind. Stimuliert man solche Tiere mit Antigen (z.B. mit einem humanen Antigen), werden Antikörper, die von den humanen Immunglobulinsequenzen abstammen (d.h. humane Antikörper), produziert. Aus den Lymphozyten solcher Tiere können mittels standardisierter Hybridomtechnologie humane monoklonale Antikörper gemacht werden. Zur weiteren Beschreibung transgener Mäuse mit humanen Immunglobulinen und ihre Verwendung bei der Herstellung humaner Antikörper siehe beispielsweise US-Patent Nr. 5,939,598, WO 96/33735, WO 96/34096, WO 98/24893 und WO 99/53049 (Abgenix Inc.), und US-Patent Nr. 5,545,806, Nr. 5,569,825, Nr. 5,625, 126, Nr. 5,633, 425, Nr. 5,661,016, Nr. 5,770,429, Nr. 5,814,318, Nr. 5,877,397 und WO 99/45962 (Genpharm Inc.); siehe ebenfalls MacQuitty, J.J. und Kay, R.M. (1992) Science 257:1188; Taylor, L.D. et al. (1992) Nucleic Acids Res. 20:6287-6295; Lonberg, N. et al. (1994) Nature 368:856-859; Lonberg, N. und Huszar, D. (1995) Int. Rev. Immunol. 13:65-93; Harding, F.A. und Lonberg, N. (1995) Ann. N.Y. Acad. Sci. 764:536-546; Fishwild, D. M. et al. (1996) Nature Biotechnology 14:845-851; Mendez, M. J. et al. (1997) Nature Genetics 15:146-156; Green, L.L. und Jakobovits, A. (1998) J. Exp. Med. 188:483-495; Green, L.L. (1999) J. Immunol. Methods 231:11-23; Yang, X.D. et al. (1999) J. Leukoc. Bio/. 66:401-410; Gallo, M.L. et al. (2000) Eur. J. Immunol. 30:534-540.

Gemäß einer weiteren Ausführungsform kann das Tier, das mit erfindungsgemäßem RGM-Protein oder einem Derivat/Äquivalent davon immunisiert wird, eine Maus mit schwerer kombinierter Immundefizienz (SCID) sein, die mit humanen peripheren mononukleären Blutzellen oder lymphoiden Zellen oder Vorläufern davon rekonstituiert wurde. Solche Mäuse, die als chimäre hu-PBMC-SCID-Mäuse bezeichnet werden, produzieren nachgewiesenermaßen humane Immunglobulinantworten nach einem antigenen Stimulus. Zur weiteren Beschreibung dieser Mäuse und ihrer Verwendung für die Erzeugung von Antikörpern siehe beispielsweise Leader, K.A. et al. (1992) Immunology 76:229-234; Bombil, F. et al. (1996) Immunobiol. 195:360-375; Murphy, W.J. et al. (1996) Semin. Immunol. 8:233-241; Herz, U. et al. (1997) Int. Arch. Allergy Immunol. 113:150-152; Albert, S.E. et al. (1997) J. Immunol. 159:1393-1403; Nguyen, H. et al. (1997) Microbiol. Immunol. 41:901-907; Arai, K. et al. (1998) J. Immunol. Methods 217:79-85; Yoshinari, K. und Arai, K. (1998) Hybridoma 17:41-45; Hutchins, W.A. et al. (1999) Hybridoma 18:121-129; Murphy, W.J. et al. (1999) Clin. Immunol. 90:22-27; Smithson, S.L. et al. (1999) Mol. Immunol. 36:113-124; Chamat, S. et al. (1999) J. Infect. Diseases 180:268-277; und Heard, C. et al. (1999) Molec. Med. 5:35-45.

Gemäß einer weiteren Ausführungsform ist das Tier, das mit erfindungsgemäßem RGM-Protein oder einem Derivat/Äquivalent davon immunisiert wird, eine Maus, die mit einer letalen Ganzkörperbestrahlung behandelt, anschließend mit Knochenmarkszellen aus Mäusen mit schwerer kombinierter Immundefizienz (SCID) gegen Strahlung geschützt, und anschließend mit funktionalen humanen Lymphozyten transplantiert worden ist. Dieser als das Trimera-System bezeichnete Chimärentyp wird verwendet, um humane monoklonale Antikörper herzustellen, indem man die Mäuse mit dem interessierenden Antigen immunisiert und anschließend monoklonale Antikörper unter Verwendung von standardisierter Hybridomtechnologie herstellt. Zur weiteren Beschreibung dieser Mäuse und ihrer Verwendung für die Erzeugung von Antikörpern siehe beispielsweise Eren, R. et al. (1998) Immunology 93:154-161; Reisner, Y und Dagan, S. (1998) Trends Biotechnol 16:242-246; Ilan, E. et al. (1999) Hepatology 29:553-562; und Bocher, W.O. et al. (1999) Immunology 96:634-641.

### In-vitro-Ansätze:

Alternativ zur Herstellung erfindungsgemäßer Antikörper durch Immunisierung und Selektion können erfindungsgemäße Antikörper identifiziert und isoliert werden, indem man eine rekombinante kombinatorische Immunglobulin-Bank mit einem erfindunggemäßen RGM-Protein oder Derivat/Äquivalent davon screent, um so Mitglieder der Immunoglobulin-Bank, die spezifisch an das RGM-Protein oder Derivat/Äquivalent davon binden, zu isolieren. Kits zur Erzeugung und zum Screenen von Display-Banken sind kommerziell erhältlich (z.B. das Recombinant Phage Antibody System von Pharmacia, Katalog-Nr. 27-9400-01; und der SurfZAP® Phage Display Kit von Stratagene, Katalog-Nr. 240612). In vielen Ausführungsformen ist die Display-Bank eine scFv-Bank oder eine Fab-Bank. Die Phagendisplay-Technik zum Screenen rekombinanter Antikörperbänke ist hinlänglich beschrieben worden. Beispiele für Verfahren und Verbindungen, die bei der Erzeugung und dem Screenen von Antikörper-Display-Banken besonders vorteilhaft verwendet werden können, sind beispielsweise in McCafferty et al. WO 92/01047, US-Patent Nr. 5,969,108 und EP 589 877 (beschreibt insbesondere den Display von scFv), Ladner et al. US-Patent Nr. 5,223,409, Nr. 5,403,484, Nr. 5,571,698, Nr. 5,837,500 und EP 436 597 (beschreibt beispielsweise die pIII-Fusion); Dower et al. WO 91/17271, US-Patent Nr. 5,427,908, US-Patent Nr. 5,580,717 und EP 527 839 (beschreibt insbesondere den Display von Fab); Winter et al. International Publication WO 92/20791 und EP 368,684 (beschreibt insbesondere die Klonierung von Sequenzen für variable Immunoglobulindomänen); Griffiths et al. US-Patent Nr. 5,885,793 und EP 589 877 (beschreibt insbesondere die Isolierung von humanen Antikörpern gegen humane Antigene unter Verwendung rekombinanter Bänke); Garrard et al. WO 92/09690 (beschreibt insbesondere Phagenexpressionstechniken); Knappik et al. WO 97/08320 (beschreibt die humane rekombinante Antikörperbank HuCal); Salfeld et al. WO 97/29131, (beschreibt die Herstellung eines rekombinanten humanen Antikörpers gegen ein humanes Antigen (humanen Tumor-Nekrose-Faktor alpha), sowie in-vitro-Affinitätsreifung des rekombinanten Antikörpers) und Salfeld et al. U.S. Provisional Application Nr. 60/126,603 und die hierauf basierenden Patentanmeldungen (beschreibt ebenfalls die Herstellung rekombinanter humaner Antikörper gegen humanes Antigen (humanes Interleukin-12), sowie die *in-vitro*-Affinitätsreifung des rekombinanten Antikörpers) zu finden.

Weitere Beschreibungen von Screenings rekombinanter Antikörperbänke sind in wissenschaftlichen Publikationen zu finden, wie Fuchs et al. (1991) Bio/Technology 9:1370-1372; Hay et al. (1992) Hum Antibod Hybridomas 3:81-85; Huse et al. (1989) Science 246:1275-1281; Griffiths et al. (1993) EMBO J 12:725-734; Hawkins et al. (1992) J Mol Biol 226:889-896; Clarkson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; Barbas et al. (1991) PNAS 88:7978-7982; McCafferty et al. Nature (1990) 348:552-554; und Knappik et al. (2000) J. Mol. Biol. 296:57-86.

Alternativ zur Verwendung von Bakteriophagen-Display-Systemen können rekombinante Antikörper-Banken auf der Oberfläche von Hefezellen oder bakteriellen Zellen exprimiert werden. Verfahren zur Herstellung und zum Screenen von Banken, die auf der Oberfläche von Hefezellen exprimiert sind, sind in WO 99/36569 beschrieben. Verfahren zur Herstellung und zum Screenen von Banken, die auf der Oberfläche von bakteriellen Zellen exprimiert sind, sind in WO 98/49286 genauer beschrieben.

Sobald ein interessierender Antikörper aus einer kombinatorischen Bank identifiziert ist, werden die DNAs, welche die leichten und schweren Ketten des Antikörpers kodieren, mittels standardisierter molekularbiologischer Techniken isoliert, beispielsweise mittels PCR-Amplifikation von DNA aus der Display-Packung (z.B. dem Phagen), die man während des Screenens der Bank isoliert hat. Nukleotidsequenzen von Genen für leichte und schwere Antikörperketten, mit denen PCR-Primer hergestellt werden können, sind dem Fachmann bekannt. Viele solcher Sequenzen sind beispielsweise in Kabat, E.A., et al. (1991) Sequences of Proteins of Immunological Interest, Fifth Edition, U.S. Department of Health und Human Services, NIH Publication Nr. 91-3242 und der Datenbank für Sequenzen der Humankeimbahn VBASE beschrieben.

Ein erfindungsgemäßer Antikörper oder Antikörperteil kann hergestellt werden, indem man die Gene für leichte und schwere Immunglobulinketten in einer Wirtszelle rekombinant exprimiert. Um einen Antikörper rekombinant zu exprimieren, wird eine Wirtszelle mit einem oder mehreren rekombinanten Expressionsvektoren, die DNA-Fragmente tragen, welche die leichten und schweren Immunglobulinketten des Antikörpers kodieren, transfiziert, so dass die leichten und schweren Ketten in der Wirtszelle exprimiert und vorzugsweise in das Medium, in dem die Wirtszellen kultiviert werden, sekretiert werden. Aus diesem Medium können die Antikörper gewonnen werden. Man verwendet standardisierte rekombinante DNA-Methodik, um Gene für schwere und leichte Antikörperketten zu erhalten, diese Gene in rekombinante Expressionsvektoren einzusetzen und die Vektoren in Wirtszellen einzuführen. Eine derartige Methodik ist beispielsweise in Sambrook, Fritsch und Maniatis (Hrsg.), Molecular Cloning; A Laboratory Manual, Second Edition, Cold Spring Harbor, N.Y., (1989), Ausubel, F.M. et al. (Hrsg.) Current Protocols in Molecular Biology, Greene Publishing Associates, (1989) und im US-Patent Nr. 4,816,397 von Boss et al. beschrieben.

Sobald DNA-Fragmente, die VH- und VL-Segmente des interessierenden Antikörpers kodieren, erhalten werden, können diese DNA-Fragmente mit standardisierten rekombinanten DNA-Techniken weiter manipuliert werden, beispielsweise um die Gene für variable Regionen in Gene für Antikörperketten voller Länge, in Gene für Fab-Fragmente oder in ein scFv-Gen zu überführen. Bei diesen Manipulationen wird ein VL- oder VH-kodierendes DNA-Fragment operativ mit einem weiteren ein weiteres Protein, z.B. eine konstante Antikörperregion oder einen flexiblen Linker, kodierenden DNA-Fragment verknüpft. Der Begriff "operativ verknüpft" soll hier meinen, dass die beiden DNA-Fragmente so miteinander verbunden sind, dass die von den beiden DNA-Fragmenten kodierten Aminosäuresequenzen im Leseraster (in-frame) bleiben.

Die isolierte, die VH-Region kodierende DNA kann in ein Gen für eine schwere Kette voller Länge überführt werden, indem man die VH-Region kodierende DNA mit einem weiteren, konstante Regionen der schweren Kette (CH1, CH2 und CH3) kodierenden DNA-Molekül operativ verknüpft. Die Sequenzen von Genen für konstante Regionen humaner schwerer Ketten sind hinlänglich bekannt (siehe z.B. Kabat, E.A., et al. (1991) Sequences of Proteins of Immunological Interest, Fifth Edition, U.S. Department of Health und Human Services, NIH Publication Nr. 91-3242), und DNA-Fragmente, die diese Regionen überspannen, können mittels standardisierter PCR-Amplifikation erhalten werden. Die konstante Region der schweren Kette kann eine konstante Region aus IgG1, IgG2, IgG3, IgG4, IgA, IgE, IgM or IgD sein, wobei eine konstante Region aus IgG1 oder IgG4 bevorzugt ist. Um ein Gen für ein Fab-Fragment der schweren Kette zu erhalten, kann die VH-kodierende DNA mit einem weiteren, lediglich die konstante Region CH1 der schweren Kette kodierenden DNA-Molekül operativ verknüpft werden.

Die isolierte, die VL-Region kodierende DNA kann in ein Gen für eine leichte Kette voller Länge (sowie ein Gen für eine Fab-leichte Kette) überführt werden, indem man die VL-kodierende DNA mit einem weiteren, die konstante Region CL der leichten Kette kodierenden DNA-Molekül operativ verknüpft. Die Sequenzen von Genen der konstanten Region humaner leichter Kette sind hinlänglich bekannt (siehe Kabat, E.A., et al. (1991) Sequences of Proteins of Immunological Interest, Fifth Edition, U.S. Department of Health und Human Services, NIH Publication Nr. 91-3242), und DNA-Fragmente, die diese Regionen überspannen, können mittels standardisierter PCR-Amplifikation erhalten werden. Die konstante Region der leichten Kette kann eine konstante kappa- oder lambda-Region sein, wobei eine konstante kappa-Region bevorzugt ist.

Um ein scFv-Gen zu erzeugen, können die VH- und VL-kodierenden DNA-Fragmente mit einem weiteren, einen flexiblen Linker, z.B. die Aminosäuresequenz (Gly₄-Ser)₃ kodierenden Fragment operativ verknüpft werden, so dass die VH- und VL-Sequenzen als fortlaufendes einzelkettiges Protein exprimiert werden, wobei die VL- und VH-Regionen über den flexiblen Linker miteinander verbunden sind (siehe Bird et al. (1988) Science 242:423-426; Huston et al. (1988) Proc. Natl. Acad. Sci. USA 85:5879-5883; McCafferty et al., Nature (1990) 348:552-554).

VH- und VL-Einzeldomänen mit Spezifität für erfindungsgemäßes RGM-Protein oder ein Derivat/Äquivalent davon können mit den oben beschriebenen Verfahren aus Einzeldomänen-Banken isoliert werden. Zwei VH-Einzeldomänen-Ketten (mit oder ohne CH1) oder zwei VL-Ketten oder ein Paar aus einer VH- und einer VL-Kette mit der gewünschten Spezifität können verwendet werden, um erfindungsgemäße RGM-Proteine oder Derivate/Äquivalente davon zu binden.

Um die erfindungsgemäßen rekombinanten Antikörper oder Antikörperteile zu exprimieren, können die DNAs, welche die leichten und schweren Ketten von partieller oder voller Länge kodieren, in Expressionsvektoren insertiert werden, so das die Gene mit transkriptionalen und translationalen Kontrolisequenzen operativ verknüpft sind. In diesem Zusammenhang soll der Begriff "operativ verknüpft" meinen, dass ein Antikörpergen in einem Vektor so ligiert ist, dass transkriptionale und translationale Kontrollsequenzen innerhalb des Vektors ihre beabsichtigte Funktion zur Regulation der Transkription und Translation des Antikörpergens erfüllen.

Der Expressionsvektor und die Expressionskontrollsequenzen werden so gewählt, dass sie mit der zur Expression verwendeten Wirtszelle kompatibel sind. Das Gen für die leichte Antikörperkette und das Gen für die schwere Antikörperkette können in getrennte Vektoren insertiert werden, oder beide Gene werden in denselben Expressionsvektor insertiert, was der Regelfall ist. Die Antikörpergene werden in den Expressionsvektor mittels standardisierter Verfahren insertiert (z.B. Ligation komplementärer Restriktionsschnittstellen am Antikörpergenfragment und Vektor, oder Ligation stumpfer Enden, falls keine Restriktionsschnittstellen vorhanden sind). Vor der Insertion der Sequenzen für die leichte und schwere Kette kann der Expressionsvektor bereits Sequenzen für konstante Antikörperregionen tragen. Beispielsweise ist es ein Ansatz, die VH- und VL-Sequenzen in Antikörpergene voller Länge zu überführen, indem man sie in Expressionsvektoren insertiert, die bereits die konstanten Regionen für schwere bzw. leichte Kette kodieren, so dass das VH-Segment mit dem oder den CH-Segment(en) innerhalb des Vektors operativ verknüpft ist, und auch das VL-Segment mit dem CL-Segment innerhalb des Vektors operativ verknüpft ist. Zusätzlich oder alternativ kann der rekombinante Expressionsvektor ein Signalpeptid kodieren, welches die Sekretion der Antikörperkette aus der Wirtszelle erleichtert. Das Gen für die Antikörperkette kann in den Vektor kloniert werden, so dass das Signalpeptid in Leseraster mit dem N-Terminus des Gens für die Antikörperkette verknüpft ist. Das Signalpeptid kann ein Immunglobulin-Signalpeptid oder ein heterologes Signalpeptid sein (d.h. ein Signalpeptid aus einem Nicht-Immunoglobulin-Protein). Zusätzlich zu den Genen für die Antikörperkette können die erfindungsgemäßen Expressionsvektoren regulatorische Sequenzen aufweisen, welche die Expression der Gene für die Antikörperkette in einer Wirtszelle kontrollieren. Der Begriff "regulatorische Sequenz" soll Promotoren, Enhancer und weitere Expressionskontrollelemente (z.B. Polyadenylierungssignale) einschließen, welche die Transkription oder Translation der Gene für die Antikörperkette kontrollieren. Solche regulatorischen Sequenzen sind beispielsweise in Goeddel; Gene Expression Technology: Methods in Enzymology 185, Academic Press, San Diego, CA (1990) beschrieben. Dem Fachmann ist bekannt, dass das Design des Expressionsvektors, wozu die Selektion regulatorischer Sequenzen gehört, von Faktoren abhängen kann, wie der Wahl der zu transformierenden Wirtszelle, der gewünschten Expressionsstärke des Proteins, etc. Zu bevorzugten regulatorischen Sequenzen für eine Expression in Säugerwirtszellen gehören virale Elemente, die zu einer starken Proteinexpression in Säugerzellen führen, wie Promotoren und/oder Enhancer, die vom Cytomegalovirus (CMV) (wie der CMV-Promoter/Enhancer), Simian-Virus 40 (SV40) (wie der SV40-Promotor/Enhancer), Adenovirus (z.B. der Späte Adenovirus-Hauptpromotor (AdMLP für Adenovirus Major Late Promoter) und Polyoma abstammen. Zur weiteren Beschreibung viraler regulatorischer Elemente und Sequenzen davon, siehe z.B. US-Patent Nr. 5,168,062 von Stinski, US-Patent Nr. 4,510,245 von Bell *et al.* und US-Patent Nr. 4,968,615 von Schaffner et al.

Zusätzlich zu den Genen für die Antikörperkette und die regulatorischen Sequenzen können die erfindungsgemäßen rekombinanten Expressionsvektoren zusätzliche Sequenzen aufweisen, wie Sequenzen, welche die Replikation des Vektors in Wirtszellen regulieren (z.B. Replikationsstartpunkte) und selektierbare Markergene. Die selektierbaren Markergene erleichtern die Selektion von Wirtszellen, in die der Vektor eingeführt wurde (siehe z.B. US-Patents Nr. 4,399,216, 4,634,665 und 5,179,017, alle von Axel *et al.*). Zum Beispiel ist es üblich, dass das selektierbare Markergen eine Wirtszelle, in die der Vektor eingesetzt wurde, gegen Wirkstoffe wie G418, Hygromycin oder Methotrexat resistent macht. Zu bevorzugten selektierbaren Markergenen gehören das Gen für die Dihydrofolatreduktase (DHFR) (zur Verwendung in dhfr-Wirtzellen mit Methotrexat-Selektion/Amplifikation) und das neo-Gen (zur G418-Selektion).

Für die Expression der leichten und schweren Ketten wird der oder werden die schwere und leichte Ketten kodierende(n) Expressionsvektor(en) mittels standardisierter Techniken in eine Wirtszelle transfiziert. Die verschiedenen Formen des Begriffs "Transfektion" sollen eine Vielzahl von Techniken erfassen, die gewöhnlicherweise zur Einführung exogener DNA in eine prokaryotische oder eukaryotische Wirtszelle verwendet werden, z.B. Elektroporation, Calcium-Phosphat-Fällung, DEAE-Dextran-Transfektion und ähnliches. Wenngleich es theoretisch möglich ist, die erfindungsgemäßen Antikörper entweder in prokaryotischen oder eukaryotischen Wirtszellen zu exprimieren, ist die Expression der Antikörper in eukaryotischen Zellen und insbesondere in Säugerwirtszellen bevorzugt, da die Wahrscheinlichkeit, dass ein korrekt gefalteter und immunologisch aktiver Antikörper zusammengesetzt und sekretiert wird, in solchen eukaryotischen Zellen und insbesondere Säugerzellen höher ist als in prokaryotischen Zellen. Über die prokaryotische Expression von Antikörpergenen ist berichtet worden, dass sie für die Produktion großer Ausbeuten aktiven Antikörpers ineffektiv sei (Boss, M.A. und Wood, C. R. (1985) Immunology Today 6:12-13).

Zu Säugerwirtszellen, die für die Expression erfindungsgemäßer rekombinanter Antikörper bevorzugt sind, gehören CHO-Zellen (einschließlich dhfr⁻-CHO-Zellen, die in Urlaub und Chasin, (1980) Proc. Natl. Acad. Sci. USA 77:4216-4220 beschrieben sind und mit einem DHFR-selektierbaren Marker verwendet werden, wie z.B. in R.J. Kaufman und P.A. Sharp (1982) Mol. Biol. 159:601-621 beschrieben ist), NS0-Myelomzellen, COS-Zellen und SP2-Zellen. Werden rekombinante Expressionsvektoren, welche die Antikörpegene kodieren, in Säugerwirtszellen eingeführt, so werden die Antikörper hergestellt, indem man die Wirtszellen so lange kultiviert, bis der Antikörper in den Wirtszellen exprimiert oder vorzugsweise der Antikörper in das Kulturmedium, in dem die Wirtszellen wachsen, sekretiert wird. Die Antikörper können aus dem Kulturmedium gewonnen werden, indem man standardisierte Verfahren zur Aufreinigung von Proteinen verwendet.

Es können ebenfalls Wirtszellen verwendet werden, um Teile intakter Antikörper, wie Fab-Fragmente oder scFv-Moleküle, herzustellen. Variationen der zuvor beschriebenen Vorgehensweise gehören selbstverständlich zur Erfindung. Beispielsweise kann es wünschenswert sein, eine Wirtszelle mit DNA zu transfizieren, die entweder die leichte Kette oder die schwere Kette (aber nicht beide) eines erfindungsgemäßen Antikörpers kodiert. Sind leichte oder schwere Ketten vorhanden, die für die Bindung des interessierenden Antigens nicht erforderlich sind, so kann die DNA, die entweder eine solche leichte oder eine solche schwere Kette oder beide kodiert, mittels rekombinanter DNA-Technologie zum Teil oder vollständig entfernt werden. Moleküle, die von solchen verkürzten DNA-Molekülen exprimiert werden, gehören ebenfalls zu den erfindungsgemäßen Antikörpern. Darüber hinaus können bifunktionale Antikörper hergestellt werden, in denen eine schwere und eine leichte Kette ein erfindungsgemäßer Antikörper sind und die andere schwere und leichte Kette Spezifität für ein anderes als das interessierende Antigen besitzen, indem man einen erfindungsgemäßen Antikörper mit einem zweiten Antikörper mittels standardisierter chemischer Verfahren quervernetzt.

In einem bevorzugten System zur rekombinanten Expression eines erfindungsgemäßen Antikörpers oder Antigen-bindenden Teils davon wird ein rekombinanter Expressionsvektor, der sowohl die schwere Antikörperkette als auch die leichte Antikörperkette kodiert, mittels Calcium-Phosphat-vermittelter Transfektion in dhfr⁻-CHO-Zellen eingeführt. Innerhalb des rekombinanten Expressionsvektors sind die Gene für die schwere und leichte Antikörperkette jeweils mit regulatorischen CMV-Enhancer/AdMLP-Promotor-Elementen operativ verknüpft, um eine starke Transkription der Gene zu bewirken. Der rekombinante Expressionsvektor trägt auch ein DHFR-Gen, mit dem sich CHO-Zellen, die mit dem Vektor transfiziert sind, selektieren lassen, indem man die Methotrexat-Selektion/Amplification verwendet. Die selektierten transformierten Wirtszellen werden kultiviert, so dass die schweren und leichten Antikörperketten exprimiert werden, und intakter Antikörper wird aus dem Kulturmedium gewonnen. Man verwendet standardisierte molekularbiologische Techniken, um den rekombinanten Expressionsvektor herzustellen, die Wirtszellen zu transfizieren, die Transformanten zu selektieren, die Wirtszellen zu kultivieren und den Antikörper aus dem Kulturmedium zu gewinnen. So betrifft die Erfindung ein Verfahren zur Synthese eines erfindungsgemäßen rekombinanten Antikörpers, indem man eine erfindungsgemäße Wirtszelle in einem geeigneten Kulturmedium kultiviert, bis ein erfindungsgemäßer rekombinanter Antikörper synthetisiert ist. Das Verfahren kann des weiteren beinhalten, dass man den rekombinanten Antikörper aus dem Kulturmedium isoliert.

Alternativ zum Screenen rekombinanter Antikörperbänke durch Phage-Display können weitere, dem Fachmann bekannte Methoden zum Screenen großer kombinatorischer Bänke eingesetzt werden, um die erfindungsgemäßen Antikörper zu identifizieren. Bei einer Art eines alternativen Expressionssytems ist die rekombinante Antikörperbank in Form von RNA-Protein-Fusionen exprimiert, wie in WO 98/31700 von Szostak und Roberts, und in Roberts, R.W. und Szostak, J.W. (1997) Proc. Natl. Acad. Sci. USA 94:12297-12302 beschrieben. In diesem System erzeugt man durch in-vitro-Translation synthetischer mRNAs, die an ihrem 3'-Ende Puromycin, ein Peptidylakzeptor-Antibiotikum, tragen, zwischen einer mRNA und dem Peptid oder Protein, das sie kodiert, eine kovalente Fusion. So kann eine spezifische mRNA aus einem komplexen Gemisch von mRNAs (z.B. einer kombinatorischen Bank) anhand der Eigenschaften des kodierten Peptids oder Proteins (z.B. des Antikörpers oder eines Teils davon) wie dem Binden des Antikörpers oder Teils davon an erfindungsgemäßes RGM-Protein oder ein Derivat/Äquivalent davon angereichert werden. Antikörper oder Teile davon kodierende Nukleinsäuresequenzen, die aus dem Screenen solcher Bänke gewonnen werden, können mit rekombinanten Mitteln in der oben beschriebenen Weise exprimiert werden (z.B. in Säugerwirtszellen) und darüber hinaus einer weiteren Affinitätsreifung unterzogen werden, indem man entweder in weiteren Durchgängen mRNA-Peptid-Fusionen screent, wobei man in die ursprünglich selektierte(n) Sequenz(en) Mutationen einführt, oder indem man andere Verfahren zur in-vitro-Affinitätsreifung rekombinanter Antikörper in der oben beschriebenen Weise verwendet.

### Kombinationen aus in vivo- und in vitro-Ansätzen:

Die erfindungsgemäßen Antikörper können ebenfalls hergestellt werden, indem man eine Kombination aus in-vivo- und in-vitro-Ansätzen anwendet, wie Verfahren, in denen man zunächst erfindungsgemäßes RGM-Protein oder ein Derivat/Äquivalent davon auf ein Antikörper-Repertoire in vivo in einem Wirtstier einwirken lässt, um die Produktion von RGM-Protein- oder Derivat/Äquivalent-bindenden Antikörpern zu stimulieren, und anschließend die weitere Antikörperselektion und/oder Antikörperreifung (d.h. Optimierung) mit Hilfe einer oder mehrerer in-vitro-Techniken bewerkstelligt wird. Einer Ausführungsform zufolge kann ein solches kombiniertes Verfahren beinhalten, dass man zunächst ein nicht-humanes Tier (z.B. eine Maus, Ratte, Kaninchen, Huhn, Kamelide, Ziege oder eine transgene Version davon oder eine chimäre Maus) mit dem erfindungsgemäßen RGM-Protein oder Derivat/Äquivalent davon immunisiert, um eine Antikörper-Antwort gegen das Antigen zu stimulieren, und anschließend unter Verwendung von Immunglobulinsequenzen aus Lymphozyten, die durch die Einwirkung des RGM-Proteins oder Derivats/Äquivalents in vivo stimuliert worden sind, eine Phage-Display-Antikörperbank herstellt und screent. Der erste Schritt dieser kombinierten Vorgehensweise kann in der oben im Zusammenhang mit den in-vivo-Ansätzen beschriebenen Weise durchgeführt werden, während der zweite Schritt dieser Vorgehensweise in der oben in Zusammenhang mit den in-Vitro-Ansätzen beschriebenen Weise durchgeführt werden kann. Zu bevorzugten Methoden für die Hyperimmunisierung von nicht-humanen Tieren mit anschließendem in-vitro-Screening von Phage-Display-Bänken, die aus den stimulierten Lymphozyten hergestellt wurden, gehören diejenigen, die von BioSite Inc., siehe z.B. WO 98/47343, WO 91/17271, US-Patent Nr. 5,427,908 und US-Patent Nr. 5,580,717 beschrieben sind.

Einer weiteren Ausführungsform zufolge beinhaltet ein kombiniertes Verfahren, dass man zunächst ein nicht-humanes Tier (z.B. eine Maus, Ratte, Kaninchen, Huhn, Kamelide, Ziege oder eine Knockout- und/oder transgene Version davon, oder eine chimäre Maus) mit einem erfindungsgemäßen RGM-Protein oder Derivat/Äquivalent davon immunisiert, um eine Antikörperantwort gegen das RGM-Protein oder Derivat/Äquivalent davon zu stimulieren, und die Lymphozyten, welche die Antikörper mit der gewünschten Spezifität produzieren, selektiert, indem man (z.B. aus den immunisierten Tieren hergestellte) Hybridome screent. Die Gene für die Antikörper oder Einzeldomän-Antikörper werden aus den selektierten Klonen isoliert (mittels standardisierter Klonierungsverfahren, wie der Reversen Transkriptase-Polymerasekettenreaktion) und einer in-vitro-Affinitätsreifung unterzogen, um dadurch die Bindungseigenschaften des selektierten Antikörpers oder der selektierten Antikörper zu verbessern. Der erste Schritt dieser Vorgehensweise kann in der oben in Zusammenhang mit den in-vivo-Ansätzen beschriebenen Weise vollzogen werden, Während der zweite Schritt dieser Vorgehensweise in der oben in Zusammenhang mit den in-vitro-Ansätzen beschriebenen Weise vollzogen werden kann, insbesondere indem man Verfahren zur in-vitro-Affinitätsreifung verwendet, wie diejenigen, die in WO 97/29131 und WO 00/56772 beschrieben sind.

In einem weiteren kombinierten Verfahren werden die rekombinanten Antikörper aus einzelnen isolierten Lymphozyten erzeugt, indem man eine Vorgehensweise verwendet, die dem Fachmann als Lymphozyten-Antikörper-Selektionsverfahren (SLAM) bekannt und in US-Patent Nr. 5,627,052, WO 92/02551 und Babcock, J.S. et al. (1996) Proc. Natl. Acad. Sci. USA 93:7843-7848 beschrieben ist. In diesem Verfahren wird ein nicht-humanes Tier (z.B. eine Maus, Ratte, Kaninchen, Huhn, Kamelide, Ziege, oder eine transgene Version davon, oder eine chimäre Maus) zunächst in vivo mit erfindungsgemäßem RGM-Protein oder einem Derivat/Äquivalent davon immunisiert, um eine Immunantwort gegen das RGM-Protein oder Derivat/Äquivalent zu stimulieren, und dann werden einzelne, interessierende Antikörper sekretierende Zellen selektiert, indem man einen antigenspezifischen hämolytischen Plaque-Assay verwendet. Hierzu können das RGM-Protein oder Derivat/Äquivalent davon, oder interessierende strukturell verwandte Moleküle an Schaf-Erythrozyten gekoppelt werden, wobei man einen Linker wie Biotin verwendet, wodurch sich einzelne Zellen, die Antikörper mit geeigneter Spezifität sekretieren, unter Verwendung des hämolytischen Plaque-Assays identifizieren lassen. In Anschluss an die Identifizierung von Zellen, die interessierende Antikörper sekretieren, werden cDNAs für die variablen Regionen der leichten und schweren Ketten aus den Zellen durch Reverse Transkriptase-PCR gewonnen und diese variablen Regionen können dann in Zusammenhang mit geeigneten konstanten Immunglobulinregionen (z.B. humanen konstanten Regionen) in Säugerwirtszellen wie COS- oder CHO-Zellen exprimiert werden. Die mit den aus in vivo selektierten Lymphozyten abstammenden, amplifizierten Immungrobulinsequenzen transfizierten Wirtszellen können dann einer weiteren in-vitro-Analyse und -Selektion unterzogen werden, indem man beispielsweise die transfizierten Zellen ausbreitet, um Zellen zu isolieren, die Antikörper mit der gewünschten Spezifität exprimieren. Die amplifizierten Immunglobulinsequenzen können des weiteren in vitro manipuliert werden.

### 6. Pharmazeutische Mittel

### 6.1 Allgemein

Gegenstand der vorliegenden Erfindung sind auch pharmazeutische Mittel (Zusammensetzungen), die als Wirkstoff ein erfindungsgemäßes Protein (RGM-Protein; RGM-Prötein-bindende Liganden, wie anti-RGM-Protein-Antikörper,) oder eine kodierende RGM-Protein-Nukleinsäuresequenz und gegebenenfalls einen pharmazeutisch verträglichen Träger beinhalten. Erfindungsgemäße pharmazeutische Zusammensetzungen können des weiteren wenigstens ein zusätzliches therapeutisches Agens, z. B. ein oder mehrere zusätzliche therapeutische Agenzien zur Behandlung einer der hierin beschriebenen Erkrankungen beinhalten.

Zu pharmazeutisch verträglichen Trägern gehören sämtliche Lösungsmittel, Dispersionsmedien, Überzüge, antimikrobielle Agenzien, isotonisierende und die Absorption verzögernde Agenzien, und dergleichen, sofern sie physiologisch kompatibel sind.

Zu pharmazeutisch akzeptablen Trägern gehören beispielsweise Wasser, Kochsalzlösung, Phosphat-gepufferte Kochsalzlösung, Lactose, Dextrose, Sucrose, Sorbitol, Mannitol, Stärken, Akaziengummi, Calciumphosphat, Alginate, Traganth, Gelatine, Calciumsilikat, mikrokristalline Cellulose, Polyvinylpyrrolidon, Cellulose, Sirup und Methylcellulose. Ferner können die Formulierungen pharmazeutisch akzeptable Träger oder übliche Hilfsstoffe, wie Gleitmittel, beispielsweise Talk, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder ÖlGrundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Cantor-Verlag, 1996, dargestellt ist. Vgl. auch Hager's Handbuch der Pharmazeutischen Praxis, Springer Verlag, Heidelberg.

Die pharmazeutischen Zusammensetzungen können beispielsweise zur parenteralen Verabreichung geeignet sein. Hierzu wird der Wirkstoff, wie z.B. der Antikörper, vorzugsweise als injizierbare Lösungen mit einem Wirkstoffgehalt von 0,1 - 250 mg/ml zubereitet. Die injizierbaren Lösungen können in flüssiger oder lyophilisierter Form in einem Flintglas oder Vial, einer Ampulle oder einer gefüllten Spritze als Dosierungsform zubereitet sein.

Der Puffer kann L-Histidin (1 - 50 mM, vorzugsweise 5 - 10 mM) enthalten und einen pH-Wert von 5,0 - 7,0, vorzugsweise von 6,0, aufweisen. Zu weiteren geeigneten Puffern gehören, ohne darauf beschränkt zu sein, Natriumsuccinat-, Natriumcitrat-, Natriumphosphat- oder Kaliumphosphat-Puffer.

Man kann Natriumchlorid verwenden, um die Tonizität der Lösung auf eine Konzentration von 0 - 300 mM (vorzugsweise 150 mM für eine flüssige Dosierungsform) einzustellen. Cryoschutzmittel können für eine lyophilisierte Dosierungsform mit einbezogen werden, wie z.B. Sucrose (z.B. 0 - 10 %, vorzugsweise 0,5 -1,0 % (w/w)). Zu weiteren geeigneten Cryoschutzmitteln gehören Trehalose und Laktose. Füllstoffe können für eine lyophilisierte Dosierungsform mit einbezogen werden, z.B. Mannitol (z.B. 1 - 10 %, vorzugsweise 2 - 4 %(w/w)). Stabilisatoren können sowohl in flüssigen als auch lyophilisierten Dosierungsformen verwendet werden, z.B. L-Methionin (z.B. 1 - 50 mM, vorzugsweise 5 - 10 mM). Zu weiteren geeigneten Füllstoffen gehören Glycin und Arginin. Ebenso können Tenside verwendet werden, beispielsweise Polysorbat-80 (z.B. 0 - 0,05 %, vorzugsweise 0,005 - 0,01 %(w/w)). Zu weiteren Tensiden gehören Polysorbat-20 und BRIJ-Tenside.

Die erfindungsgemäßen Zusammensetzungen können eine Vielzahl von Formen annehmen. Zu diesen gehören flüssige, halbfeste und feste Dosierungsformen, wie flüssige Lösungen (z. B. injizierbare und infundierbare Lösungen, Lotionen, Augen- und Ohrentropfen), Liposome, Dispersionen oder Suspensionen und feste Formen, wie Pulver, Puder, Granulate, Tabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster. Auch implantierte Abgabevorrichtungen können zur Verabreichung erfindungsgemäßer Wirkstoffe verwendet werden. Die bevorzugte Form hängt von der beabsichtigten Verabreichungsart und der therapeutischen Anwendung ab. Typischerweise werden Zusammensetzungen in Form von injizierbaren oder infundierbaren Lösungen bevorzugt. Ein geeigneter Verabreichungsweg ist z.B. parenteral (z. B. intravenös, subkutan, intraperitoneal, intramuskulär). Gemäß einer bevorzugten Ausführungsform wird der Wirkstoff durch intravenöse Infusion oder Injektion verabreicht. Einer weiteren bevorzugten Ausführungsform zufolge wird der Wirkstoff durch intramuskuläre oder subkutane Injektion verabreicht.

Therapeutische Zusammensetzungen müssen typischerweise steril und unter den Herstellungs- und Lagerungsbedingungen stabil sein. Die Zusammensetzungen können als Lösung, Mikroemulsion, Dispersion, liposomal oder einer weiteren für hohe Wirkstoffkonzentrationen geeigneten, geordneten Struktur formuliert werden. Sterile injizierbare Lösungen können hergestellt werden, indem man die aktive Verbindung (wie z.B. den Antikörper) in der benötigten Menge in ein geeignetes Lösungsmittel gegebenenfalls mit einem oder einer Kombination der vorstehend genannten inhaltsstoffe, je nach Bedarf, einbringt und anschließend steril filtriert. Dispersionen werden in der Regel zubereitet, indem man die aktive Verbindung in ein steriles Vehikel einbringt, welches ein Grunddispersionsmedium und gegebenenfalls weitere benötigte Inhaltsstoffe enthält. Im Falle eines sterilen lyophilisierten Pulvers zur Herstellung steriler injizierbarer Lösungen stellen die Vakuumtrocknung und Sprühtrocknung bevorzugte Herstellungsverfahren dar, mit denen ein Pulver des aktiven Inhaltsstoffes und gegebenenfalls weiterer erwünschter Inhaltsstoffe aus einer zuvor steril filtrierten Lösung erhalten wird. Die richtige Fliessfähigkeit einer Lösung kann beibehalten werden, indem man beispielsweise einen Überzug wie Lecithin verwendet, im Fall von Dispersionen die benötigte Partikelgröße beibehält oder Tenside verwendet. Eine verlängerte Absorption injizierbarer Zusammensetzungen kann erreicht werden, indem man ein Agens, das die Absorption verzögert, beispielsweise Monostearatsalze und Gelatine, in die Zusammensetzung mit einbringt.

Die erfindungsgemäßen Wirkstoffe können mit einer Vielzahl von Verfahren, die dem Fachmann bekannt sind, verabreicht werden, wenngleich für viele therapeutische Anwendungen die subkutane Injektion, intravenöse Injektion oder Infusion die bevorzugte Verabreichungsart darstellt. Der Fachmann weiß, dass Weg und/oder Art der Verabreichung vom gewünschten Resultat abhängen. Bestimmten Ausführungsformen zufolge kann die aktive Verbindung mit einem Träger zubereitet werden, der die Verbindung gegen rasche Freisetzung schützt, so zum Beispiel eine Formulierung mit kontrollierter Freisetzung, wozu Implantate, transdermale Pflaster und mikroverkapselte Abgabesysteme gehören. Es können biologisch abbaubare biokompatible Polymere verwendet werden, wie Ethylenvinylacetat, Polyanhydride, Polyglykolsäure, Collagen, Polyorthoester und Polymilchsäure. Die Verfahren zur Zubereitung solcher Formulierungen sind dem Fachmann allgemein bekannt, siehe zum Beispiel Sustained und Controlled Release Drug Delivery Systems, J.R. Robinson, ed., Marcel Dekker, Inc., New York, 1978.

Bestimmten Ausführungsformen zufolge kann ein erfindungsgemäßer Wirkstoff oral verabreicht werden, beispielsweise in einem inerten Verdünnungsmittel oder einem assimilierbaren essbaren Träger. Der Wirkstoff (und gewünschtenfalls weitere Inhaltsstoffe) können auch in einer Hart- oder Weichgelatinekapsel eingeschlossen, zu Tabletten verpresst oder direkt der Nahrung zugesetzt werden. Für die orale therapeutische Verabreichung können die Wirkstoffe mit Exzipienten vermischt und in Form von schluckbaren Tabletten, Buccaltabletten, Kapseln, Elixieren, Suspensionen, Sirups und dergleichen verwendet werden. Soll ein erfindungsgemäßer Wirkstoff über einen anderen als den parenteralen Weg verabreicht werden, kann es erforderlich sein, eine Beschichtung aus einem Material zu wählen, das seine Inaktivierung verhindert.

Die erfindungsgemäßen Wirkstoffe können zusammen mit einem oder mehreren zusätzlichen therapeutischen Agenzien verabreicht werden, die bei der Behandlung der zuvor beschriebenen Erkrankungen brauchbar sind.

Die pharmazeutischen Zusammensetzungen der vorliegenden Erfindung enthalten in der Regel eine therapeutisch wirksame Menge oder eine prophylaktisch wirksame Menge wenigstens eines erfindungsgemäßen Wirkstoffs. In Abhängigkeit von der gewünschten Behandlung, ob beispielsweise eine therapeutische oder prophylaktische Behandlung gewünscht ist, können Dosierungspläne gewählt und angepasst werden. Beispielsweise kann man eine Einzeldosis, mehrere getrennte Dosen über die Zeit verteilt oder eine ansteigende bzw. sinkende Dosierung je nach den Anforderungen der therapeutischen Situation verabreichen. Es ist insbesondere von Vorteil, parentale Zusammensetzungen in Einheitsdosierungsform zu formulieren, um die Verabreichung zu erleichtern und eine Gleichförmigkeit der Dosierung zu gewährleisten.

Der behandelnde Arzt kann die für die jeweilige Therapie und den jeweiligen Wirkstoff am meisten geeignete Darreichungsform, Art der Verabreichung und Dosierung ohne weiteres bestimmen.

Eine therapeutisch oder prophylaktisch wirksame Menge eines erfindungsgemäßen Wirkstoffs kann beispielsweise im Bereich von 0,1 - 20 mg/kg und vorzugsweise 1 - 10 mg/kg liegen, ohne darauf beschränkt zu sein. Natürlich können diese Mengen je nach Art und Schwere des zu lindernden Zustandes variieren.

### 6.2 Vakzine

Die erfindungsgemäßen RGM-Proteine und Derivate/Äquivalente davon sind als Immunogen zur Vakzinierung eines zu behandelnden Patienten brauchbar.

Zu diesem Zweck brauchbare Vakzine stellen in der Regel eine pharmazeutische Zusammensetzung dar, die wenigstens ein erfindungsgemäßes RGM-Protein und/oder wenigstens ein erfindungsgemäßes Derivat/Äquivalent davon enthält. Ferner kann die Zusammensetzung einen physiologisch verträglichen Träger und gegebenenfalls weitere Hilfsstoffe, beispielsweise Immunstimulantien, enthalten.

Während geeignete Träger im Prinzip beliebig gewählt werden können, richtet sich die Art des Trägers in der Regel nach dem Verabreichungsweg. So können die erfindungsgemäßen Vakzine insbesondere in einer zur parenteralen, beispielsweise intravenösen, intramuskulären und subkutanen Verabreichung geeigneten Form formuliert werden. In diesen Fällen beinhaltet der Träger vorzugsweise Wasser, Kochsalzlösung, Alkohol, ein Fett, ein Wachs und/oder einen Puffer.

Es können beliebige einer Vielzahl von Immunstimulantien in den erfindungsgemäßen Vakzinen verwendet werden. Beispielsweise kann ein Adjuvans mit einbezogen werden. Die meisten Adjuvantien enthalten eine Substanz, die das Antigen vor einem raschen Abbau schützen sollen, wie Aluminiumhydroxid oder ein Mineralöl, sowie ein von Lipid A, aus Bordetella pertussis oder Mycobacterium tuberculosis abgeleitetes Protein. Geeignete Adjuvantien sind in der Regel kommerziell erhältlich, beispielsweise komplettes oder inkomplettes Freund-Adjuvans; AS-2; Aluminiumsalze, wie Aluminiumhydroxid (gegebenenfalls alas Gel) oder Aluminiumphosphat; Calcium-, Eisen- oder Zinksalze; eine unlösliche Suspension acylierten Tyrosins; acylierte Zucker; kationisch oder anionisch derivatisierte Polysaccharide; Polyphosphazene; biologisch abbaubare Mikrosphären; Monophosphoryl-Lipid A. Cytokine, wie GM-CSF oder Interleukin-2, -7 oder -12 können ebenfalls als Adjuvantien verwendet werden.

### 7. Therapie

### 7.1. Behandlung von neuronalen Erkrankungen

Aus dem Stand der Technik ist bereits bekannt, dass bei Verletzungen des Zentralen Nervensystems eine Akkumulation von RGM-Protein an der Läsionsstelle zu beobachten ist (vgl. Schwab et al. a.a.O.). Gleichzeitig wird dadurch das erneute Auswachsen der verletzten Nervenfasern verhindert. Diese schädigende Wirkung auf das Nervenfaserwachstum wird durch die Bindung von RGM an das Rezeptormolekül Neogenin vermittelt. Eine Modulation, insbesondere Inhibition, der Wechselwirkung zwischen RGM und dem Rezeptormolekül Neogenin wäre deshalb geeignet, um die inhibitorische Aktivität von RGM auf das Nervenfaserwachstum zu unterbinden.

### 7.2. Behandlung von Tumorerkrankungen

Es gibt seit längerem Hinweise, die vermuten lassen, dass Neogenin mit der Entstehung und/oder der Progression von Tumorerkrankungen in ursächlichem Zusammenhang stehen könnte. So berichteten beispielsweise Meyerhardt et al., in Oncogene (1997) 14, 1129-1136, dass Neogenin in mehr als 50 untersuchten Krebszelllinien, darunter Glioblastom-, Medulloblastom-, Neuroblastom-Zelllinien sowie Zelllinien von Colorektal-, Brust-, Pankreas- und Zervixkarzinomen nachweisbar war. Eine Überexpression von Neogenin wurde außerdem in Oesophaguskrebszelllinien beobachtet (Hue et al., Clinical Cancer Research (2001) 7, 2213-2221). Eine systematische Untersuchung der Expressionsprofile von 3588 Genen in 211 Lungenadenocarzinom-Patienten lieferte kürzlich einen weiteren Hinweis auf die Beteiligung von Neogenin an der Entstehung und dem Verlauf der Tumorerkrankung (Berrar et al., J. Comput. Biol. (2005) 12 (5),534-544).

Da außerdem bekannt ist, dass RGM eine potentielle tumorfördernde Wirkung aufweist, in dem es Zelltod durch Bindung an den tumorzell-assoziierten Neogeninrezeptor verhindern kann (Matsunagä et al. Nature Cell Biol. 6, 749 - 755, 2004), könnte durch Modulation der RGM-Neogenin-Wechselwirkung, insbesondere durch Unterbrechung dieser Wechselwirkung mit Hilfe spezifischer Anti-RGM-Antikörper ein neuer Therapieansatz zur Behandlung von Tumorerkrankungen geschaffen werden.

### 7.3. Behandlung von Eisenstoffwechselerkrankungen

RGM C, auch Hemojuvelin genannt, ist von essentieller Bedeutung für den Eisenstoffwechsel des menschlichen und tierischen Körpers. Juvenile Hemochromatose ist eine vererbte, relativ seltene Eisenstoffwechselerkrankung, welche sich in einer Eisenüberladung des Organismus äußert. Für die Entstehung dieser Erkrankung sind Mutationen im Hemojuvelin-Molekül ursächlich (vgl. Huang et al., The Journal of Clinical Investigation (2005), 115, 2087-2091). Die Verabreichung von funktionalem erfindungsgemäßen RGM-Proteinen oder deren aktiven Domänen stellt daher einen brauchbaren Therapieansatz zur Linderung derartiger Eisenstoffwechselerkrankungen dar.

### 7.4. Förderung der Bildung von Knochengewebe

Im Stand der Technik gibt es Hinweise darauf, dass ein Mitglied der RGM-Familie von Proteinen, nämlich RGM B, auch bekannt unter der Bezeichnung DRAGON, an der Knochenmorphogenese beteiligt ist. So beschreiben beispielsweise Samad et al. in JBC Papers in Press, Ausgabe vom 25. Januar 2005, die Wechselwirkung zwischen DRAGON und den Rezeptoren vom Typ I und Typ II des Bone Morphogenetic Protein (BMP). Durch Verabreichung erfindungsgemäßer RGM-Polypeptide ist daher eine das Knochenwachstum fördernde Wirkung und damit ein neuer Therapieansatz zur Behandlung von Erkrankungen mit gestörtem Knochenwachstum oder von Knochenverletzungen denkbar.

### 8. Diagnostik

Als diagnostische Agenzien sind erfindungsgemäß insbesondere RGM-Protein und Derivate/Äquivalente gemäß obiger Definition sowie dagegen gerichtete Antikörper zu nennen.

Die vorliegende Erfindung ermöglicht daher insbesondere die verbesserte qualitative oder quantitative Bestimmung oben definierter Krankheitszustände durch Nachweis krankheitstypischer Antigene oder Antikörper.

Die Bestimmung erfolgt vorzugsweise mit immunologischen Methoden. Prinzipiell kann dies mit jedem analytischen bzw. diagnostischen Testverfahren erfolgen, bei dem Antikörper eingesetzt werden. Hierzu gehören Agglutinations- und Präzipitations-Techniken, Immunoassays, immunhistochemische Verfahren und Immunoblot-Techniken, z.B. Western-Blotting oder Dot Blot-Verfahren. Auch in vivo-Verfahren gehören dazu, beispielsweise bildgebende Verfahren.

Von Vorteil ist der Einsatz in Immunoassays. Geeignet sind sowohl kompetitive Immunoassays, d.h. Antigen und markiertes Antigen (Tracer) konkurrieren um die Antikörperbindung, als auch Sandwich-Immunoassays, d.h. die Bindung spezifischer Antikörper an das Antigen wird mit einem zweiten, meist markierten Antikörper nachgewiesen. Diese Assays können sowohl homogen, d.h. ohne eine Trennung in feste und flüssige Phase, als auch heterogen sein, d.h. gebundene Markierungen werden von ungebundenen getrennt, beispielsweise über Festphasen-gebundene Antikörper. Die verschiedenen heterogenen und homogenen Immunoassay-Formate können je nach Markierung und Meßmethode bestimmten Klassen zugeordnet werden, beispielsweise RIAs (Radioimmunoassays), ELISA (Enzyme linked immunosorbent assay), FIA (Fluoreszenz-Immunoassay), LIA (Lumineszenz-Immunoassay), TRFIA (zeitlich aufgelöster FIA), IMAC (Immunaktivierung), EMIT (Enzyme multiplied immune test), TIA (Turbidimetrischer Immunoassay), I-PCR (Immuno-PCR).

Zur erfindungsgemäßen Antigen-Bestimmung sind kompetitive Immunoassays bevorzugt. Dabei konkurriert markiertes Antigen (Tracer) mit dem zu quantifizierenden Antigen der Probe um die Bindung an den verwendeten Antikörper. Aus der Menge des verdrängten Tracers lässt sich mit Hilfe einer Standardkurve die Antigenmenge, sprich die Antigenmenge, in der Probe bestimmen.

Von den für diese Zwecke zur Verfügung stehenden Markierungen haben sich Enzyme als vorteilhaft erwiesen. Beispielsweise können Systeme auf Basis von Peroxidasen, insbesondere der Meerrettich-Peroxidase, der alkalischen Phosphatase und der ß-D-Galactosidase, verwendet werden. Für diese Enzyme stehen spezifische Substrate zur Verfügung, deren Umsetzung sich z.B. photometrisch verfolgen lässt. Geeignete Substratsysteme basieren auf p-Nitrophenylphosphat (p-NPP), 5-Brom-4-chlor-3-indolylphosphat/Nitroblau-Tetrazolium (BCIP/NPT), Fast-Red/Naphthol-AS-TS-Phosphat für die alkalische Phosphatase; 2,2-Azino-bis-(3-ethylbenzthiazolin-6-sulfonsäure) (ABTS), o-Phenylendiamin (OPD), 3,3',5,5'-Tetramethylbenzidin (TMB), o-Dianisidin, 5-Aminosalicylsäure, 3-Dimethylaminobenzoesäure (DMAB) und 3-Methyl-2-benzothiazolinonhydrazon (MBTH) für Peroxidasen; o-Nitrophenyl-β-D-galactosid (o-NPG), p-Nitrophenyl-β-D-Galactosid und 4-Methylumbelliphenyl-β-D-galactosid (MUG) für die ß-D-Galactosidase. Diese Substratsysteme sind in vielen Fällen in gebrauchsfertiger Form kommerziell erhältlich, beispielsweise in Form von Tabletten, die auch weitere Reagenzien, wie zweckmäßige Puffer und ähnliches enthalten können.

Die Kopplung von Markierungen an Peptide oder Antikörper zur Herstellung von Tracern kann in an sich bekannter Weise erfolgen. Darüber hinaus stehen eine Reihe zur Konjugation an Proteine zweckmäßig modifizierte Markierungen zur Verfügung, beispielsweise Biotin-, Avidin-, Extravidin- oder Streptavidin-konjugierte Enzyme, Maleimid-aktivierte Enzyme und ähnliches. Diese Markierungen können direkt mit dem erfindungsgemäß zu verwendenden Molekül umgesetzt werden.

Wird ein heterogenes Immunoassay-Format gewählt, so kann der Antigen-Antikörper-Komplex zwecks Trennung beispielsweise über einen an den Träger gekoppelten antiidiotypischen Antikörper, z.B. einen gegen Kaninchen-IgG gerichteten Antikörper, an den Träger gebunden werden. Träger, insbesondere Mikrotiterplatten, die mit entsprechenden Antikörpern beschichtet sind, sind bekannt und kommerziell erhältlich.

Ein weiterer Gegenstand der vorliegenden Erfindung sind Immunoassay-Sets mit wenigstens einem vorstehend beschriebenen Antikörper und weiteren Komponenten. Es handelt sich hierbei um eine Zusammenstellung, in der Regel als Verpackungseinheit, von Mitteln zur Durchführung einer erfindungsgemäßen Bestimmung. Zwecks möglichst einfacher Handhabung werden diese Mittel vorzugsweise im wesentlichen gebrauchsfertig bereitgestellt. Eine vorteilhafte Anordnung bietet den Immunoassay in Kit-Form an. Ein Kit umfasst in der Regel mehrere Behältnisse zur getrennten Anordnung von Komponenten. Alle Komponenten können in gebrauchsfertiger Verdünnung, als Konzentrat zum Verdünnen oder als Trockensubstanz oder Lyophilisat zum Lösen oder Suspendieren bereitgestellt werden; einzelne oder sämtliche Komponenten können eingefroren sein oder bei Umgebungstemperatur bis zum Gebrauch aufbewahrt werden. Seren sind vorzugsweise schockgefroren, beispielsweise bei -20°C, so dass in diesen Fällen ein Immunoassay vor Gebrauch vorzugsweise bei Gefriertemperaturen zu halten ist.

Weitere, dem Immunoassay beigefügte Komponenten können sein: Standardprotein, Tracer; Kontrollserum, Mikrotiterplatten, vorzugsweise mit Antikörper beschichtet, Puffer, beispielsweise zum Testen, zum Waschen oder zur Umsetzung des Substrats, und das Enzymsubstrat selbst.

Allgemeine Prinzipien von Immunoassays und die Erzeugung und Verwendung von Antikörpern als Hilfsmittel in Labor und Klinik finden sich beispielsweise in Antibodies, A Laboratory Manual (Harlow, E., and Lane, D., Ed., Cold Spring Harbor Laboratory, Cold Spring Harbor, NY, 1988).

### 9. Screening-Verfahren

Gegenstand der Erfindung sind auch Verfahren zum Nachweis von Effektoren des RGM-Rezeptors Neogenin, wobei man eine Probe, in der man einen Effektor vermutet, mit einem RGM-Protein oder -Polypeptid inkubiert und den Ansatz auf die Bildung eines Effektor- RGM-Protein -Komplexes untersucht.

Derartige Effektoren können eine agonistische, partiell agonistische, antagonistische oder invers agonistische Wirkung besitzen. Es können dies z.B. synthetische niedermolekulare Supstanzen, synthetische Peptide, natürliche oder synthetische Antikörpermoleküle oder Naturstoffe sein.

Solche erfindungsgemäßen Verfahren werden in der Regel als *in vitro*-Screening-Verfahren durchgeführt, mit denen man aus einer Vielzahl verschiedener Substanzen diejenigen auslesen kann, die im Hinblick auf eine künftige Anwendung am aussichtsreichsten zu sein scheinen.

Beispielsweise können mittels kombinatorischer Chemie umfangreiche Stoffbanken angelegt werden, die eine Vielzahl potentieller Wirkstoffe umfassen. Das Durchmustern kombinatorischer Substanzbibliotheken nach Stoffen mit gewünschter Aktivität ist automatisierbar. Screening-Roboter dienen der effizienten Auswertung der vorzugsweise auf Mikrotiterplatten angeordneten Einzelassays. So betrifft die vorliegende Erfindung auch Screening-Verfahren, d.h. sowohl Primär- als auch Sekundärscreening-Verfahren, bei denen vorzugsweise wenigstens eines der nachfolgend beschriebenen Verfahren zur Anwendung kommt. Kommen mehrere Verfahren zur Anwendung, so kann das zeitlich versetzt oder gleichzeitig an ein und derselben Probe oder an verschiedenen Proben einer zu untersuchenden Substanz geschehen.

Eine effektive Technologie zur Durchführung derartiger Verfahren ist der im Bereich des Wirkstoffscreenings bekannte Scintillation Proximity Assay, kurz SPA genannt. Kits und Komponenten zur Durchführung dieses Assays können kommerziell bezogen werden, beispielweise bei Amersham Pharmacia Biotech. Im Prinzip werden solubilisierte oder membrangebundene Rezeptoren auf Szintillationssubstanz enthaltenden, kleinen Fluoromikrosphären immöbilisert. Bindet beispielsweise ein Radioligand an die immobilisierten Rezeptoren, so wird die Szintillationssubstanz zur Lichtemission angeregt, da die räumliche Nähe zwischen Szintillationssubstanz und Radioligand gegeben ist.

Eine weitere effektive Technologie zur Durchführung derartiger Verfahren ist die im Bereich des Wirkstoffscreenings bekannte FlashPlateR-Technologie. Kits und Komponenten zur Durchführung dieses Assays können kommerziell bezogen werden, beispielweise bei NEN Life Science Products. Dieses Prinzip basiert ebenfalls auf Mikrotiterplatten (96er oder 384er), die mit Scintillationssubstanz beschichtet sind.

Die nach diesen Verfahren identifizierbaren Substanzen oder Teile aus Substanzgemischen sind ebenfalls Gegenstand der vorliegenden Erfindung.

Die Erfindung wird nun unter Bezugnahme auf folgende nichtlimitierende Herstellungs- und Anwendungsbeispiele näher erläutert

### Experimenteller Teil

### 1. Allgemeine Angaben

### Testmethode 1: Darstellung der Wirkung von RGM Peptiden bei neuronalen Auswachsversuchen mit kortikalen Neuronen der Ratte

Um die Wirkung von RGM Peptiden *in vitro* zu untersuchen, wurden neuronale Auswachsversuche durchgeführt. Dazu wurden kortikale Neuronen der Ratte präpariert, wobei die folgenden Medien verwendet wurden:
**Stockmedium** pro Liter: 90 ml GB5, 100 ml Minimum Essential Medium - Eagle (10x; Gibco, Bestell Nr.: 21430.020), ad 1000 ml mit Millipore Wasser. pH 7,0, 280-310 mosm.
**GB5** pro Liter: 26,6 g NaHCO₃, 44,4 g Glucose, sterilfiltriert.
**Plattiermedium (PM)** pro Liter: 0,8 mM Glutamin, 100 ml hitzeinaktiviertes fötales Kälberserum (FCS), 100 ml hitzeinaktiviertes Pferdeserum, ad 1000 ml mit Stockmedium
**Erhaltungsmedium (EM)** pro Liter: 0,8 mM Glutamin, 100 ml hitzeinaktiviertes Pferdeserum, ad 1000 ml mit Stockmedium
**Trypsinlösung:** 0,1% Trypsin, 0,04% EDTA in PBS ohne Calcium/Magnesium, sterilfiltriert.

Für die Gewinnung korticaler Neuronen wurden trächtige Ratten durch Überstrecken getötet, deren Bauchraum eröffnet, der Uterus mit den Embryonen (embryonic day 18 (E18) entnommen und in PBS gewaschen. Mit einer Feinpräparationsschere wurde der Uterus längs aufgeschnitten, die Embryonen entnommen und freipräpariert. Die Embryonen wurden durch Genickschnitt getötet, das Gehirn entnommen und der Vorderhirn-Kortex präpariert. Die Kortices werden in je 1ml einer 0,1%igen Trypsinlösung für 5 Minuten bei 37°C inkubiert, die Reaktion mit Erhaltungsmedium (EM) abgestoppt und nach 5-4minütiger Inkubation in insgesamt 10 ml EM mit feuerpolierten Pasteurpipetten mit absteigendem Öffnungsdurchmesser trituiert. Die Zellsuspension wurde 10 Minuten bei 1200 U/min abzentrifugiert und der Überstand abgesaugt und verworfen. Der Zellniederschlag wurde in 8 ml EM aufgenommen und vorsichtig resuspendiert. Die Zellen wurden in einer Neubauer Zählkammer ausgezählt, die Zellzahl auf ca. 400.000 Zellen/0,2 ml in EM eingestellt und prä-aggregiert. Dazu wurden pro Well 200 µl der Zellsuspension auf Glas-Chamberslides (LabTek, Bestell Nr. 177402) gegeben und für 24 h bei 37°C inkubiert. Dabei bilden sich Neuronenaggregate. 10-80µl) dieser Aggregate wurden pro Vertiefung einer poly-D-Lysine beschichteten 96well Platte (z.B. Becton Dickinson Biocoat 96-well black plate # 354640) ausplattiert und ggf. mit EM auf 70-90µl aufgefüllt. Nach 1h wurden die RGM-Peptide in verschiedenen Konzentrationen in einem Volumen von 10µl zugegeben und ggf. mit EM auf 100µl aufgefüllt. 24-48h später wurden das Neuritenauswachsen zunächst durch lichtmikroskopische Untersuchung beurteilt und die Kulturen danach durch Zugabe von 100µl 4% Parafomaldehydlösung fixiert und mindestens 12 Stunden bei 4°C gelagert. Alle Schritte zur Darstellung des Tubulinzytoskeletts durch Immunfluoreszenzfärbung, mit Ausnahme der Inkubation mit dem Primärantikörper, erfolgten bei Zimmertemperatur. Die Vertiefungen wurden einmal mit 100-300µl PBS für 5-15 Minuten gewaschen und danach die Zellen durch Inkubation in 100µl 0,1% Triton X-100 für 10-20min permeabilisiert. Die Vertiefungen wurden zweimal mit 300µl PBS für 5-15 Minuten gewaschen und anschließend für 60 Minuten mit 100µl 1% Rinderserumalbuminlösung in PBS inkubiert. Der Primärantikörper (z.B. Sigma, monoclonal anti-ß-Tubulin isotype III clone SDL 3D10, #T8660; Abcam, TuJI #ab14545) wurde 1:1000 in 1% Rinderserumalbuminlösung in PBS verdünnt und 50µl pro Vertiefung über Nacht bei 4°C inkubiert. Die Vertiefungen wurden dreimal mit 100-300µl PBS für 5-15 Minuten gewaschen. Der fluoreszenzmarkierten Sekundärantikörpers (Jackson ImmunoResearch, Cy3 conjugated Affinity Pure Donkey Anti Mouse # 715-165-151) wurde 1:500 in 1% Rinderserumalbuminlösung in PBS, die zusätzlich 0,5µg/ml Bisbenzimid (H33258) zur Darstellung der Zellkerne enthielt, verdünnt und 50µl dieser Verdünnung wurden pro Vertiefung für 1-2h bei Zimmertemperatur oder über Nacht bei 4°C inkubiert. Die Vertiefungen wurden zweimal mit 100-300µl PBS für 5-15 Minuten gewaschen, das PBS abgesaugt und ein Tropfen (ca. 50µl) Fluoromount G (Southern Biotechnology Associates Inc#010001) pro Vertiefung zugegeben. Die Aufnahme der Bilder erfolgte an einem inversen Fluoreszenzmikroskop (Zeiss, Axiovert 200 M), wobei jeweils die Fluoreszenz des markierten Sekundär-antikörpers und die Bisbenzimid-Fluoreszenz erfasst wurde. Die von den beiden Fluoreszenzen bedeckte Fläche wurde mit Hilfe eines Bildauswerteprogramms (Media Cybernetics, Image-Pro Plus) ermittelt. Zur Ermittlung des NeuritenWachstums-Indexes (NW-I) wurde die Flächendifferenz zwischen Sekundärantikörperfärbung (Anfärbung der ausgewachsenen Neuriten und der Zellaggregate) und der Bisbenzimidfärbung (Anfärbung der Zellaggregate) gebildet und durch die Fläche der Bisbenzimidfärbung dividiert. Damit stellt dieser Index ein Maß für die von Neuriten bedeckte Fläche in Relation zur Zellaggregatgröße dar.

### Testmethode 2: Darstellung der Wirkung von RGM Peptiden bei neuronalen Auswachsversuchen mit kortikalen humanen Neuronen

Die humane, pluripotente Karzinom-Zellinie NTera (DSMZ ACC527) ist als Zellkulturmodell etabliert. Neuriten wachsen dabei aus Zell-Aggregaten aus und bilden eine Korona aus Neuriten um das jeweilige Aggregat.

Hierzu wurden 2,5 x 10⁶ NTera Zellen in einer 175 cm² Flasche ausgesät und für 3 Wochen mit 10 µM Retinsäure (Sigma) differenziert (Medium: D-MEM (Gibco/Invitrogen 31966-021) 10% Fötales Kälberserum, 100u/ml Penicillin, 100 µg/ml Streptomycin (beides Gibco/Invitrogen)). Differenzierte Zellen wurden anschließend 1:6 in neue Flaschen aufgeteilt und ohne Retinsäure für weitere 2 Tage kultiviert. Auf dem entstandene Zellrasen angeheftete neuronale Zellen wurden durch Abschlagen gelöst und in einem Schüttelkolben in Neurobasalmedium (Neurobasal Medium (Gibco/Invitrogen 21103-049), 2mM L-Glutamin (Gibco/Invitrogen 25030-024), 100u/ml Penicillin, 100 µg/ml Streptomycin (beides Gibco/Invitrogen) über Nacht (im Brutschrank) durch langsames Schütteln aggregiert.

Ntera-Aggregate wurden am folgenden Tag in Poly-D-Lysin und Laminin (Sigma) (10µg/mL) 96-well Platten ausgesät (Biocoat Poly-D-Lysin Cellware 96-Well Black/Clear Plate (Becton Dickinson # 35 6640). Die inhibitorische Wirkung von RGM Peptiden und Fragmenten wurde durch Zugabe unterschiedlicher Konzentrationen der zu testenden Substanzen analysiert.

### Testmethode 3: RGM A - Neogenin Bindungstest:

### a) Materialen:

- Immuno-Platte: Cert. Maxi Sorp F96 (NUNC, 439454)
- Rekombinantes humanes RGM A, R&D Systems; Prod.#2495-RM (260µg/ml)
- Rekombinantes humanes Neogenin-Fc, Abbott; Ludwigshafen (ALU 1514/122; 425µg/ml)
- Peroxidase-conjugierter, affinitätsgereinigter Maus-anti human IgG-Fc Fragment AK (Jackson Immuno Research, Code:209-035-098 (0,8mg/ml))
- Entwicklersubstrate: Immuno Pure TMB Substrate Kit (Pierce,#34021)
- Schwefelsäure (Merck,#4.80354.1000)

### b) Methode:

1. RGM A Bindung an Immuno-Platte:
   - 2,5µg/ml RGM A (R&D) in 50 mM Na₂CO₃ (50 µl/ well)
   - Inkubation 1h bei 37°C
2. Waschschritt:
   - 3 x mit PBS/ 0,02% Tween20 (100)µl/ well) waschen
3. Blockierung unspezifischer Bindungsstellen
   - Blockierung mit 3% BSA in PBS/0,02% Tween (200)µl/ well)
   - Inkubation 1h bei 37°C
4. Neogenin Bindung:
   - Zugabe von Neogenin in Verdünnungen (Start Konz. 1µg/ml) in 1 %BSA PBS/0,02% Tween
   - Inkubation 1h bei 37°C
5. Waschschritt:
   - 3 x mit PBS/ 0,02% Tween20 (100µl/ well) waschen
6. Antikörper Detektion des gebundenen Neogenins:
   - Zugabe von HRP gekoppeltem Maus anti human IgG-Fc Fragment AK (1:2500 in PBS/1%BSA verdünnt) (50µl/well)
   - Inkubation 1h bei 37°C
7. Waschschritt:
   - 3 x mit PBS/ 0,02% Tween20 (100µl/well) waschen
8. Entwicklung
   - Zugabe von 50µl/ well des Entwicklungssubstrates (Immuno Pure TMB substrate, Pierce)
   - Inkubation 1-30 min, Raumtemperatur,
   - Reaktion stoppen mit 50µl 2,5M H₂SO₄ / well

### 2. Herstellungsbeispiele

### Herstellungsbeispiel 1: Herstellung von RGM A-Protein-Fragmenten in Säugetierzellen

Zur Charakterisierung der aktiven RGM A Domäne in Neuritenwachstums- und Neogenin-Bindungstestverfahren wurde RGM A (AS 168-422) sowie 70-80 AS große RGM A Fragmente (Frag. 1: 169-238; Frag. 2: 218-284; Frag. 3: 266-335; Frag. 4: 316-386; Frag. 5: 369-238; und Frag. 6: (168-422)) in Säugetierzellen (HEK293) als AP-Fusionsproteine exprimiert.

Dazu wurde die für das jeweilige Fragment kodierende DNA in den Vektor pDEST AP/ccdb/Myc/His (Invitrogen, Gateway Vektor System) (AP - Alkalische Phosphatase; PPC - PreScisson Protease) kloniert .Die für den jeweiligen Fragmentbereich kodierende DNA wurde zu diesem Zweck mittels PCR aus dem RZPD Klon (Klon AL136826 (DKFZp434D0727); publizierte RZPD Sequenz: BC015886 , AL136826) amplifiziert). Dazu wurden folgende Oligonukleotide eingesetzt:
Fragment 169-238:
Fragment 218-284:
Fragment 316-386
Fragment 266-335
Fragment 368-422
Fragment 169-422

Die erhaltenen PCR Produkte wurde über spezifische Rekombination (attL x attR) in den Vektor pDEST AP/ccdb/Myc/His kloniert und die korrekte Sequenz durch Sequenzierung überprüft.

HEK293-Zellen (ATCC CRL 1573) wurden mit den Plasmiden transfiziert. Dazu wurden Zellen am Tag vor der Transfektion in 15 cm Platten mit einer Dichte von 80% ausgesät. Am folgenden Tag wurde entsprechend der Vorschrift des Herstellers DNS mit Lipofektamin 2000 (Invitrogen) vermischt und die Zellen damit 12h inkubiert. Nach weitere vierwöchiger Kultur unter selektiven Kulturbedingungen (D-MEM (Gibco/Invitrogen 31966-021) 10% Fötales Kälberserum, 100u/ml Penicillin, 100 µg/ml Streptomycin (beides Gibco/Invitrogen), 150 µg/ml Zeocin/Invitrogen) wurden stabil exprimierende Klone durch Nachweis der Alkalischen Phosphatase im Wachstumsmedium selektiert.

Zur Protein-Produktion wurden stabil exprimierende Zellen in Wachstumsmedium amplifiziert und bei Erreichen einer Konfluenz von 70% in Produktionsmedium (Pro293-Medium (BioWhittaker/Cambrex, 12-764Q), 2mM Glutamin (Invitrogen)) überführt. Nach weiteren 4 Tagen Kultur wurde das Produktionsmedium geerntet, von Zelldebris getrennt und mit Hilfe von Membrankonzentratoren (Vivaspin30) angereichert.

Die exprimierten AP-RGM-Fusionsproteine wurden anschließend mit Hilfe von Ni-Chelat-Affinitätschromatographie aus den Überständen aufgereinigt. Dazu wurde Ni-NTA-Agarose (Quiagen) in Puffer A (50mM HEPES-KOH, 100mM NaCl, 10% Glycerol, 10 mM Imidazol) äquilibriert. Der aufkonzentrierte Überstand wurde anschließend 1 h bei 4°C unter Schwenken mit der Ni-NTA-Agarose inkubiert. Nach dreimaligem Waschen der durch Zentrifugation abgetrennten Ni-NTA-Agarose wurde gebundenes Protein mit Elutionspuffer (Puffer A mit 250 mM Imidazol) eluiert.

Die Analyse der Proteine erfolgte mit Hilfe von Dot-Blot-, SDS-PAGE- und Western-Blot Analysen. Die Konzentrationsbestimmung erfolgte über Bradford-ProteinBestimmung und ELISA-Messung der AP-Konzentration.

### 3. Ausführungsbeispiele

### Ausführungsbeispiel 1: Fragmentanalyse mit Neogenin-exprimierenden HEK293-Zellen

### a) Allgemein:

Bisher war bekannt, dass die aktive, wachstumsinhibitorische Aktivität des RGM A Proteins aus Hühnchen im Bereich zwischen der Aminosäureposition 150 und 350 liegt.

Es wurden daher Fragmente des humanen RGM A Proteins hergestellt, die diesen Bereich abdecken. HEK293 Zellen, die den RGM Rezeptor auf ihrer Oberfläche endogen tragen, wurden dann mit diesen Fragementen transfiziert um klonale Zellkulturen herzustellen, die die einzelnen Protein-Fragmente produzieren und ins Kulturmedium abgeben. Sämtliche Fragemente wurden als Fusionsproteine mit angeheftetem Enzym Alkalische Phosphatase produziert. Die Anwesenheit der aktiven Domäne sollte sich in diesen Zellen bemerkbar machen durch: geringere Zellproliferation, vermehrten Zelltod und/oder veränderte Zell-Substrat Adhäsion (Anheftung).

Vermehrter Zelltod und geringere Proliferation haben natürlich direkte Auswirkung auf die produzierte Menge des aktiven Fragmentes. Die Menge der produzierten Fragmente wurde mit Hilfe eines semi-quantitativen Dot Blot-Testes beurteilt.

### b) Durchführung:

Die gemäß Herstellungsbeispiel 1 hergestellten RGM-A-Fragmente wurden in HEK293-Zellen eingeführt. Stabil transfizierte Zellen wurden bis zur Konfluenz in Kulturmedium (D-MEM (Gibco/Invitrogen 31966-021) 10% Fötales Kälberserum, 100u/ml Penicillin, 100 µg/ml Streptomycin (beides Gibco/Invitrogen), 150 µg/ml Zeocin (Invitrogen) kultiviert. Anschließend wurden je 1 ml des Kulturüberstandes in einer Slot-Blot Kammer durch eine Nitrocellulosemembran (Sartorius) filtriert. Auf der Membran fixierte AP-RGM-Fusionsproteine wurde durch Nachweis der Alkalischen Phosphatase Aktivität durch Inkubation der Nitrocellulosemembran mit NBT/BCIP Substrat (Roche) detektiert. Die produzierte Menge des aktiven Fragmentes wurde mit Hilfe eines semiquantitativen Dot Blot-Testes bestimmt.

Auf der Basis dieses Testes wurden die stabilen Zellklone in folgende Kategorien eingeteilt:
- stark produzierende Zellklone (S producer)
- mittelstark produzierende Klone
- schwach produzierende Klone
- nicht-produzierende Klone (0 producer).

Desweiteren wurde das Verhältnis von stark zu nicht-produzierenden Klonen bestimmt.

### c) Ergebnis:

Für die einzelnen getesteten RGM A Fragmente wurden folgende Werte ermittelt:

| **Fragment (Aminosäurepositionen)** | **Verhältnis "S producer" versus "0 producer"** | **Anteil stark produzierender Zellklone [%]** |
|---|---|---|
| 1 (169 - 238) | 2.3 | 40 |
| 2 (218 - 284) | 0.5 | 17 |
| 3 (266 - 335) | 0.16 | 6.2 |
| 4 (316 - 386) | 0.5 | 16 |
| 5 (369 - 422) | 0.63 | 16 |
| 6 (168 - 422) | 0.16 | 9 |

Insbesondere die Fragmente 3 und 6 zeigten in diesem Test eine ausgeprägte Aktivität.

### Ausführungsbeispiel 2: Untersuchung synthetischer RGM A-Peptide im Nervenfaserwachstumstest

Zu diesem Zweck wurden die folgenden RGM A-Peptide synthetisiert und in dem oben beschriebenen Nervenfaserwachstumstest (Testmethode 1 und Testmethode 2) in verschiedenen Konzentrationen getestet:
Peptid 1: AS 267-285
Peptid 2: AS 308-325
Peptid 3: AS 358-377
Peptid 4: AS 378-400
(Nummerierung bezogen auf SEQ ID NO:2)

Von den bisher getesteten Peptiden war Peptid 1 zuverlässig und reproduzierbar inhibitorisch aktiv. Es ist deshalb davon auszugehen, dass Peptid 1(AS 267-285) Bestandteil der aktiven Domäne des RGM-Proteins ist.

Die Versuchsergebnisse sind in den Figuren 3A,B,C und 4A,B dargestellt.

Figur 3A zeigt, dass Peptid 1 im Gegensatz zu Peptid 4 bei einer Konzentration von 10 µg/ml in Versuchen mit neuronalen Zellen der Ratte (Testmethode 1) in signifikanter Weise das Nervenfaserwachstum inhibiert. Figur 3B veranschaulicht die Wirkung unterschiedlicher Konzentrationen (0 bis 30 µg/ml) von Peptid 1 auf das Nervenfaserwachstum von Kortikalen Neuronen. Ab einer Konzentration von mehr als 3 µg/ml ist eine signifikante inhibitorische Aktivität nachweisbar. Im Gegensatz dazu zeigt Peptid 4 unter gleichen Bedingungen keine Aktivität (vgl. Figur 3C) (NW-I = Neuritenwachstums-Index, entspricht der Gesamtfläche des neuronalen Aggregats mit den zugehörigen Neuriten, minus der Aggregatfläche.

Figur 4A zeigt, dass Peptid 1, nicht aber Peptid 4 bei einer Konzentration von 30 µg/ml in signifikanter Weise das Nervenfaserwachstum von humanen NTera-Zellen inhibiert (Testmethode 2). Figur 4B veranschaulicht die statistische Signifikanz des beobachteten inhibitorischen Effekts von Peptid 1.

### Ausführungsbeispiel 3: Untersuchung synthetischer RGM A-Fragmenten im Nervenfaserwachstumstest

Die anschließend nochmals aufgelisteten, sechs verschiedenen RGM A Fragmente, hergestellt gemäß Herstellungsbeispiel 1, wurden im Neuritenwachstumstest (vgl. obige Testmethode 2) mit humanen NTera Nervenzellen auf inhibitorische Aktivität getestet.
Fragment 1: Aminosäuren 169 - 238
Fragment 2: Aminosäuren 218 - 284
Fragment 3: Aminosäuren 266 - 335
Fragment 4: Aminosäuren 316 - 386
Fragment 5: Aminosäuren 369 - 422
Fragment 6: Aminosäuren 168 - 422
(Nummerierung jeweils bezogen auf SEQ ID NO:2)

Die Ergebnisse sind in den beiliegenden Figuren 5A, B und C zusammengefasst.

Im NTera Auswachstest wurden insgesamt 6 verschieden RGM A Fragmente getestet. Aktiv waren die Fragmente 2, 3 und 6, inaktiv waren die Fragmente 1, 4 und 5.

Fragment 6 entspricht dem aktiven, vollständig prozessierten RGM A Protein, wie es auch in vivo beschrieben wurde. Aus diesen Daten folgt, dass eine wichtige Nervenfaserwachstums-inhibitorische Domäne des humanen RGM A Proteins im Bereich der Fragmente 2 und 3 lokalisiert ist, also zwischen den Aminosäuren 218 und 335. Um diese inhibitorische Domäne genauer zu charakterisieren, werden daher in einem nächsten Schritt kurze RGM A Peptide aus den Bereichen der Fragmente 2 und 3 getestet.

### Ausführungsbeispiel 4: Untersuchung synthetischer RGM A-Peptiden im Nervenfaserwachstumstest

Die in folgender Tabelle dargestellten Peptide wurden im Neuritenwachstumstest (vgI. obige Testmethode 2) getestet.

**Tabelle: Benachbarte RGM A Peptide von Peptid 1 wurden synthetisiert und im Neuritenauswachstest eingesetzt.**

| | |
|---|---|
| Peptid 1 (267 - 285) | GQHVEIQAKYIGTTIVVRQ (SEQ ID NO:8) |
| Peptid Down-1 (260 - 275) | KITEKVSGQHVEIQAK SEQ ID NO:26) |
| Peptid Up-1 (276 - 291) | YIGTTIWRQVGRYLT (SEQ ID NO:27) |
| Peptid 5-Ak (242 - 259) | AFVDGSKNGGDKHGANSL (SEQ ID NO:30) |
| Peptid 6-Ak (300 - 316) | VVNAVEDWDSQGLYLC (SEQ ID NO:28) |
| Peptid 7-Ak (217 - 234) | TIIFKNFQECVDQKVYQA (SEQ ID NO:29) |

Peptid 1 wurde bereits in Ausführungsbeispiel 2 getestet.

Die Versuchsergebnisse sind in den Figuren 6A und B dagestellt.

Drei der getesteten RGM A Peptide führten im NTera Wachstumstest zur einer starken Reduktion des Nervenfaserwachstums (Fig. 6A). Bei diesen aktiven RGM A Peptiden handelte es sich um die folgenden Peptide: Peptid 1, Peptid Down-1 und Peptid Up-1. Nicht aktiv oder nur schwach aktiv waren folgende Peptide: Peptid5-Ak, Peptid6-Ak und Peptid 7-Ak (Fig. 6B). Dies legt nahe, dass eine der inhibitorischen Domänen von RGM A in dem Bereich zu lokalisieren ist, der durch die 3 aktiven RGM A Peptide festgelegt ist, also der Bereich des humanen RGM A Proteins der sich etwa von 250 bis 300, insbesondere von etwa 260 - 291 erstreckt und somit den Kernbereich von Position 267 bis 285 (vgl SEQ ID NO: 7, 8) umfasst. Aufgrund der Aktivität der beiden Peptide 6-Ak und 7-Ak, die bei der höheren Peptidkonzentration eine Tendenz zur Wachstumshemmung zeigten (Fig. 6B), ist durchaus vorstellbar, dass RGM A noch weitere inhibitorische Domänen besitzt und daher sind die Domänen im Bereich von etwa 210 - 260 sowie etwa 290 - 350 ebenfalls Gegenstand der Erfindung.

### Ausführungsbeispiel 5: Neutralisierung der inhibitorischen Domäne von RGM A mit Antikörpern

In diesem Experiment sollte überprüft werden, ob polyklonale Antikörper, die gegen aktive Peptide der inhibitorischen Domäne von RGM A erzeugt wurden, in der Lage sind, die Interaktion von RGM A mit seinem Rezeptor Neogenin zu blockieren und in vitro die Nervenfaserwachstumshemmung des aktiven und potentesten RGM A Fragmentes 2 (vgl. Ausführungsbeispiel 2) zu neutralsieren.

Beispielhaft wurde daher das Peptid Down-1 verwendet, da es Bestandteil des aktiven RGM A Fragments 2 ist. Gekoppelt an ein Trägerprotein (LPH), wurden mit diesem Peptid zwei Kaninchen nach folgendem Schema immunisiert

| Peptid | Peptidmenge | Tier | Erstimmunisierung | Boost | Boost | Boost | Blutung | Titer |
|---|---|---|---|---|---|---|---|---|
| Down-1 | 2,4mg | 8640 | Tag 0 | Tag 7 | Tag 14 | Tag 28 | Tag 35 | 1:150000 |
| 800790 | | 8641 | | | | | | 1:30000 |

| Letzter Boost | Ausblutung | Reinigung |
|---|---|---|
| Tag 56 | 20ml | 24,9mg in 22,5ml |
| | 20ml | 24,1 mg in 22ml |

Nach mehreren Immunisierungen wurden die erzeugten Down-1 spezifischen Antikörper gereinigt und in verschiedenen Testsystemen eingesetzt.
a) RGM A -Neogenin Bindungstest:
   Die Durchführung erfolgt gemäß Testmethode 3; die Versuchsergebnisse sind in Figur 7A dargestellt.
b) Neutralisierung des aktiven RGM A Fragmentes im Neuritenwachstumstest:
   Die gegen das Peptid Down-1 erzeugten polyklonalen Antikörper blockierten sehr effizient die Interaktion des RGM A Proteins mit seinem Rezeptor Neogenin. Im nächsten Test wurde daher untersucht, ob der Down-1 spezifische Antikörper in der Lage ist, das sehr potente RGM A Fragment 2 (vgl. Ausführungsbeispiel 3) zu neutralisieren.

Die Druchführung erfolgte gemäß Testmethode 2; die Versuchsergebnisse sind in Figur 7B dargestellt.

Zusammenfassen ist festuzustellen, dass die gegen das Peptid Down-1 gerichteten polyklonalen Antikörper im RGM A - Neogenin Bindungstest die Interaktion von Neogenin und RGM A verhindern und im Neuritenwachstumstest nahezu vollständig die inhibitorische Aktivität des RGM A Fragmentes neutralisieren. Der Bereich zwischen den aminoterminalen Aminosäuren 250 und 300, insbesondere 260 und 291, des RGM A Proteins ist daher besonders wichtig für seine inhibitorische Aktivität und wird hiermit als neue funktional relevante Domäne beschrieben.

## Patentansprüche

1. Neogenin-Rezeptor-bindende Domäne des Repulsive Guidance Molecule (RGM) mit einer Aminosäuresequenz
von Aminosäureposition 200 bis 350 gemäß SEQ ID NO: 2,
von Aminosäureposition 200 bis 325 gemäß SEQ ID NO:2, oder
von Aminosäureposition 200 bis 300 gemäß SEQ ID NO: 2, oder
von Aminosäureposition 200 bis 285 gemäß SEQ ID NO: 2, oder
von Aminosäureposition 250 bis 285 gemäß SEQ ID NO: 2, oder
von Aminosäureposition 260 bis 285 gemäß SEQ ID NO: 2, oder
von Aminosäureposition 215 bis 340 gemäß SEQ ID NO: 2, oder
von Aminosäureposition 260 bis 340 gemäß SEQ ID NO: 2, oder
von Aminosäureposition 250 bis 300 gemäß SEQ ID NO: 2, oder
von Aminosäureposition 260 bis 291 gemäß SEQ ID NO: 2, oder
von Aminosäureposition 210 bis 260 gemäß SEQ ID NO: 2, oder
von Aminosäureposition 290 bis 350 gemäß SEQ ID NO: 2,oder ein funktionales, Neogenin-Rezeptor-bindendes Fragment davon mit einer Länge von 10-30 zusammenhängenden Aminosäureresten, oder funktionales Neogenin-Rezeptor-bindendes Äquivalent davon mit einer Sequenzhomologie von wenigstens 75 %.

2. Neogenin-Rezeptor-bindende Domäne mit einer Aminosäuresequenz gemäß dem Sequenzbereich von Position 260 bis 291 oder 267 bis 285 gemäß SEQ ID NO: 2, oder
dem Sequenzbereich von Position 260 bis 325 gemäß SEQ ID NO: 4 oder
dem Sequenzbereich von Position 250 bis 300 gemäß SEQ ID NO: 6;oder Neogenin-Rezeptor-bindendes Fragment davon, welches wenigsten 10 zusammenhängende Aminosäurereste daraus aufweist.

3. Antigenes Polypeptid-Fragment der Neogenin-Rezeptor-bindenden Domäne nach einem der vorhergehenden Ansprüche, wobei das antigene Fragment wenigstens 10 zusammenhängende Aminosäurereste der Domäne aufweist.

4. Antigenes Polypeptid-Fragment nach Anspruch 3, welches zur Herstellung von Immungiobulin-Molekülen brauchbar ist, die die Bindung von RGM an den Neogenin-Rezeptor modulieren.

5. Antigenes Polypeptid-Fragment nach Anspruch 3, welches wenigsten 10 zusammenhängende Aminosäurereste eines Peptids mit einer der Sequenzen gemäß SEQ ID NO: 7, 8, 9, 10 oder 23 bis 29 oder 31 bis 34 umfasst.

6. Verwendung einer Neogenin-Rezeptor-bindenden Domäne nach einem der Ansprüche 1 und 2 oder eines Polypeptid-Fragments nach einem der Ansprüche 3 bis 5 zur Herstellung eines polyklonaler Antiserums oder monoklonaler Antikörper gegen RGM.

7. Verwendung nach Anspruch 6, wobei das Antiserum oder der Antikörper die Bindung von RGM an den Neogenin-Rezeptor moduliert.

8. Polyklonales Antiserum oder monoklonaler Antikörper gegen RGM gemäß der Definition in einem der Ansprüche 6 und 7 zur Verwendung in der Diagnose oder Therapie.

9. Verwendung eins polyklonalen Antiserums oder eines monoklonale Antikörpers nach Anspruch 8 zur Herstellung eines pharmazeutischen Mittels zur Diagnose oder Therapie von Krankheiten oder Krankheitsstadien welche durch eine Wechselwirkung des Neogenin-Rezeptors mit RGM oder einem RGM-Fragment mediiert werden.

10. Verwendung nach Anspruch 9, wobei die Krankheiten oder Krankheitsstadien ausgewählt sind unter
a) mechanischen Verletzungen von Schädel, Gehirn und Rückenmark,
b) chronische Erkrankungen, ausgewählt unter neurodegenerativen, inflammatorischen oder Autoimmun-Erkrankungen,
c) Störungen der neuronalen Regeneration, des axonalen Sproutings, der Neuritenextension und der neuronalen Plastizität,
d) Tumorerkrankungen und Tumormetastasierung.

11. Verwendung einer Neogenin-Rezeptor-bindenden Domäne nach einem der Ansprüche 1 und 2 oder eines Polypeptid-Fragments nach einem der Ansprüche 3 bis 5 zur Herstellung eines Mittels zur Diagnose oder Therapie von Krankheiten oder Krankheitsstadien welche durch eine gestörte oder beeinträchtigte Wechselwirkung von RGM oder einem RGM-Fragment mit dem zugeordneten Rezeptor mediiert werden.

12. Verwendung nach Anspruch 11, wobei die Krankheiten oder Krankheitsstadien ausgewählt sind unter
a) veränderten Neuritogeneseprozessen bei psychotischen Erkrankungen und chronischen Schmerzzuständen die durch exzessive Neuritensprossung und/oder pathologische Synaptogenese hervorgerufen werden.
b) Erkrankungen, assoziiert mit einem gestörten Eisenstoffwechsel;
c) Erkrankungen, assoziiert mit einem gestörten Knochenwachstum
d) Erkrankungen, assoziiert mit degenerativen Knorpetveränderungen
e) Erkrankungen, assoziiert mit Bandscheiben - und Wirbelkörperschäden
f) Erkrankungen, assoziiert mit deregulierten, unkontrollierten Zellmigrationsvorgängen.

13. Neogenin-Rezeptor-bindende Domäne nach einem der Ansprüche 1 und 2 oder eines Polypeptid-Fragments nach einem der Ansprüche 3 bis 5 zur Verwendung als Target zum Nachweis oder zur Identifizierung von RGM-bindenden Liganden.

14. Neogenin-Rezeptor-bindende Domäne nach einem der Ansprüche 1 und 2 oder eines Polypeptid-Fragments nach einem der Ansprüche 3 bis 5 zur Verwendung als Immunogen zur aktiven oder passiven Immunisierung.

15. Polyklonales Antiserum, erhältlich durch Immunisierung eines Säugers mit einer antigenen Menge einer Neogenin-Rezeptor-bindenden Domäne nach einem der Ansprüche 1 und 2 oder eines Polypeptid-Fragments nach einem der Ansprüche 3 bis 5.

16. Monoklonaler Antikörper gegen eine Neogenin-Rezeptor-bindende Domäne nach einem der Ansprüche 1 und 2 oder gegen ein Polypeptid-Fragment nach einem der Ansprüche 3 bis 5, oder ein Antigen-bindendes Fragment davon, gegebenenfalls in humanisierter Form.

17. Pharmazeutisches Mittel, umfassend in einem pharmazeutisch verträglichen Träger wenigsten einen aktiven Bestandteil ausgewählt unter:
a) einer Neogenin-Rezeptor-bindenden Domäne nach einem der Ansprüche 1 und 2 oder einem Polypeptid-Fragment nach einem der Ansprüche 3 bis 5,
b) monoklonale oder polyklonale Antikörpern nach einem der Ansprüche 15 und 16.

18. Pharmazeutisches Mittel nach Anspruch 17, zur intrathecalen, intravenösen, subkutanen, oralen oder parenteralen, nasalen und Inhalations-Verabreichung.

19. Expressionsvektor, umfassend wenigstens eine kodierende Nukleinsäuresequenz für eine Neogenin-Rezeptor-bindende Domäne nach einem der Ansprüche 1 und 2 oder ein Polypeptid-Fragment nach einem der Ansprüche 3 bis 5, operativ verknüpft mit wenigstens einer regulativen Nukleinsäuresequenz.

20. Rekombinanter Mikroorganismus, welcher wenigstes einen Vektor nach Anspruch 19 trägt.

21. Hybridomzelllinie, welche einen monoklonalen Antikörper nach Anspruch 16 produziert.

22. Verfahren zur Herstellung einer Neogenin-Rezeptor-bindende Domäne nach einem der Ansprüche 1 und 2 oder eines Polypeptid-Fragment nach einem der Ansprüche 3 bis 5, wobei man einen rekombinanten Mikroorganismus nach Anspruch 20 kultiviert und das produzierte Proteinprodukt aus der Kultur isoliert.

23. Verfahren zur Herstellung eines monoklonalen Antikörpers nach Anspruch 16, wobei man eine Hybridomzelllinie nach Anspruch 21 kultiviert und das produzierte Proteinprodukt aus der Kultur isoliert.

## Claims

1. A neogenin receptor-binding domain of the repulsive guidance molecule (RGM) having an amino acid sequence
from amino acid position 200 to 350 as shown in SEQ ID NO: 2,
from amino acid position 200 to 325 as shown in SEQ ID NO: 2, or
from amino acid position 200 to 300 as shown in SEQ ID NO: 2, or
from amino acid position 200 to 285 as shown in SEQ ID NO: 2, or
from amino acid position 250 to 285 as shown in SEQ ID NO: 2, or
from amino acid position 260 to 285 as shown in SEQ ID NO: 2, or
from amino acid position 215 to 340 as shown in SEQ ID NO: 2, or
from amino acid position 260 to 340 as shown in SEQ ID NO: 2, or
from amino acid position 250 to 300 as shown in SEQ ID NO: 2, or
from amino acid position 260 to 291 as shown in SEQ ID NO: 2, or
from amino acid position 210 to 260 as shown in SEQ ID NO: 2, or
from amino acid position 290 to 350 as shown in SEQ ID NO: 2, or
a functional, neogenin receptor-binding fragment thereof having a length of 10-30 consecutive amino acid residues, or a functional neogenin receptor-binding equivalent thereof having a sequence homology of at least 75 %.

2. A neogenin receptor-binding domain having an amino acid sequence from the sequence region from position 260 to 291 or 267 to 285 as shown in SEQ ID NO: 2, or
from the sequence region from position 260 to 325 as shown in SEQ ID NO: 4 or
from the sequence region from position 250 to 300 as shown in SEQ ID NO: 6; or
a neogenin receptor-binding fragment thereof having at least 10 consecutive amino acid residues thereof.

3. An antigenic polypeptide fragment of the neogenin receptor-binding domain as claimed in any of the preceding claims, wherein the antigenic fragment comprises at least 10 consecutive amino acid residues of said domain.

4. The antigenic polypeptide fragment as claimed in claim 3, which can be used to produce immunoglobulin molecules which modulate the binding of RGM to the neogenin receptor.

5. The antigenic polypeptide fragment as claimed in claim 3, which comprises at least 10 consecutive amino acid residues of a peptide having one of the sequences as shown in SEQ ID NO: 7, 8, 9, 10 or 23 to 29 or 31 to 34.

6. Use of a neogenin receptor-binding domain as claimed in either of claims 1 and 2 or of a polypeptide fragment as claimed in any of claims 3 to 5 for producing a polyclonal antiserum or monoclonal antibodies against RGM.

7. The use as claimed in claim 6, where the antiserum or the antibody modulates the binding of RGM to the neogenin receptor.

8. Polyclonal antiserum or monoclonal antibody against RGM as defined in either of claims 6 and 7 for use in diagnosis or therapy.

9. The use of a polyclonal antiserum or of a monoclonal antibody as claimed in claim 8 for producing a pharmaceutical composition for the diagnosis or therapy of diseases or disease stages which are mediated by an interaction of the neogenin receptor with RGM or an RGM fragment.

10. The use as claimed in claim 9, where the diseases or disease stages are selected from
a) mechanical injuries of the skull, brain and spinal cord,
b) chronic disorders selected from neurodegenerative, inflammatory and autoimmune diseases,
c) impairments of neuronal regeneration, of axonal sprouting, of axonal extension and of neuronal plasticity,
d) neoplastic diseases and tumor metastasis.

11. The use of a neogenin receptor-binding domain as claimed in either of claims 1 and 2 or of a polypeptide fragment as claimed in any of claims 3 to 5 for producing a composition for the diagnosis or therapy of diseases or disease stages which are mediated by a disturbed or impaired interaction of RGM or an RGM fragment with the associated receptor.

12. The use as claimed in claim 11, where the diseases or disease stages are selected from
a) altered axonogenesis processes associated with psychotic disorders and chronic states of pain caused by excessive axon sprouting and/or pathological synaptogenesis.
b) disorders associated with an impaired iron metabolism
c) disorders associated with an impaired bone growth
d) disorders associated with degenerative cartilage changes
e) disorders associated with damage to intervertebral disks and vertebrae
f) disorders associated with deregulated, uncontrolled cell migration processes.

13. The neogenin receptor-binding domain as claimed in either of claims 1 and 2 or the polypeptide fragment as claimed in any of claims 3 to 5 for use as target for detecting or for identifying RGM-binding ligands.

14. The neogenin receptor-binding domain as claimed in either of claims 1 and 2 or the polypeptide fragment as claimed in any of claims 3 to 5 for use as immunogen for active or passive immunization.

15. A polyclonal antiserum obtainable by immunizing a mammal with an antigenic amount of a neogenin receptor-binding domain as claimed in either of claims 1 and 2 or of a polypeptide fragment as claimed in any of claims 3 to 5.

16. A monoclonal antibody against a neogenin receptor-binding domain as claimed in either of claims 1 and 2 or against a polypeptide fragment as claimed in any of claims 3 to 5, or an antigen-binding fragment thereof, where appropriate in humanized form.

17. A pharmaceutical composition comprising in a pharmaceutically acceptable carrier at least one active ingredient selected from:
a) a neogenin receptor-binding domain as claimed in either of claims 1 and 2 or a polypeptide fragment as claimed in any of claims 3 to 5,
b) monoclonal or polyclonal antibodies as claimed in either of claims 15 and 16.

18. The pharmaceutical composition as claimed in claim 17, for intrathecal, intravenous, subcutaneous, oral or parenteral, nasal and inhalational administration.

19. An expression vector comprising at least one coding nucleic acid sequence for a neogenin receptor-binding domain as claimed in either of claims 1 and 2 or a polypeptide fragment as claimed in any of claims 3 to 5, operatively linked to at least one regulatory nucleic acid sequence.

20. A recombinant microorganism which harbors at least one vector as claimed in claim 19.

21. A hybridoma cell line which produces a monoclonal antibody as claimed in claim 16.

22. A method for producing a neogenin receptor-binding domain as claimed in either of claims 1 and 2 or a polypeptide fragment as claimed in any of claims 3 to 5, where a recombinant microorganism as claimed in claim 20 is cultured, and the produced protein product is isolated from the culture.

23. A method for producing a monoclonal antibody as claimed in claim 16, where a hybridoma cell line as claimed in claim 21 is cultured, and the produced protein product is isolated from the culture.

## Revendications

1. Domaine liant le récepteur de néogénine de la Repulsive Guidance Molecule (RGM) ayant une séquence d'aminoacides
des positions d'aminoacides 200 à 350 selon SEQ ID NO:2,
des positions d'aminoacides 200 à 325 selon SEQ ID NO:2, ou
des positions d'aminoacides 200 à 300 selon SEQ ID NO:2, ou
des positions d'aminoacides 200 à 285 selon SEQ ID NO:2, ou
des positions d'aminoacides 250 à 285 selon SEQ ID NO:2, ou
des positions d'aminoacides 260 à 285 selon SEQ ID NO:2, ou
des positions d'aminoacides 215 à 340 selon SEQ ID NO:2, ou
des positions d'aminoacides 260 à 340 selon SEQ ID NO:2, ou
des positions d'aminoacides 250 à 300 selon SEQ ID NO:2, ou
des positions d'aminoacides 260 à 291 selon SEQ ID NO:2, ou
des positions d'aminoacides 210 à 260 selon SEQ ID NO:2, ou
des positions d'aminoacides 290 à 350 selon SEQ ID NO:2, ou
ou fragment liant le récepteur de néogénine fonctionnel de celui-ci ayant une longueur de 10-30 résidus d'aminoacides liés, ou un équivalent liant le récepteur de néogénine fonctionnel de celui-ci ayant une homologie de séquence d'au moins 75 %.

2. Domaine liant le récepteur de néogénine ayant une séquence d'aminoacides selon
le domaine de séquence de la position 260 à 291 ou 267 à 285 selon SEQ ID NO:2, ou
le domaine de séquence de la position 260 à 325 selon SEQ ID NO:4 ou
le domaine de séquence de la position 250 à 300 selon SEQ ID NO:6 ; ou un fragment liant le récepteur de néogénine de celui-ci, qui comprend au moins 10 résidus d'aminoacides liés de celui-ci.

3. Fragment de polypeptide antigène du domaine liant le récepteur de néogénine selon l'une des revendications précédentes, où le fragment antigène comprend au moins 10 résidus d'aminoacides liés du domaine.

4. Fragment de polypeptide antigène selon la revendication 3, qui est utilisable pour la production de molécules d'immunoglobulines qui modulent la liaison de RGM au récepteur de néogénine.

5. Fragment de polypeptide antigène selon la revendication 3, qui comprend au moins 10 résidus d'aminoacides liés d'un peptide ayant l'une des séquences selon SEQ ID NO:7, 8, 9, 10 ou 23 à 29 ou 31 à 34.

6. Utilisation d'un domaine liant le récepteur de néogénine selon l'une des revendications 1 et 2 ou d'un fragment de polypeptide selon l'une des revendications 3 à 5, pour la production d'un antisérum polyclonal ou d'un anticorps monoclonal contre RGM.

7. Utilisation selon la revendication 6, où l'antisérum ou l'anticorps module la liaison de RGM au récepteur de néogénine.

8. Antisérum polyclonal ou anticorps monoclonal contre RGM selon la définition dans l'une des revendications 6 et 7 destiné à être utilisé dans le diagnostic ou la thérapie.

9. Utilisation d'un antisérum polyclonal ou d'un anticorps monoclonal selon la revendication 8 pour la production d'un agent pharmaceutique pour le diagnostic ou la thérapie de maladies ou de stades de maladies qui sont à médiation par une interaction du récepteur de néogénine avec RGM ou un fragment de RGM.

10. Utilisation selon la revendication 9, où les maladies ou les stades de maladies sont choisis parmi
a) les lésions mécaniques du crâne, du cerveau et de la moelle épinière,
b) les maladies chroniques choisies parmi les maladies neurodégénératives, inflammatoires ou autoimmunes,
c) les troubles de la régénération neuronale, du sprouting axonal, de l'extension des neurites et de la plasticité neuronale,
d) les maladies tumorales et des métastases tumorales.

11. Utilisation d'un domaine liant le récepteur de néogénine selon l'une des revendications 1 et 2 ou d'un fragment de polypeptide selon l'une des revendications 3 à 5 pour la production d'un agent pour le diagnostic ou la thérapie de maladies ou de stades de maladies qui sont à médiation par une interaction perturbée ou dégradée de RGM ou d'un fragment de RGM avec le récepteur correspondant.

12. Utilisation selon la revendication 11, où les maladies ou stades de maladies sont choisis parmi
a) les processus de neuritogenèse modifiés dans des maladies psychotiques et des états douloureux chroniques qui sont provoqués par un bourgeonnement de neurites excessif et/ou une synaptogenèse pathologique,
b) les maladies associées avec un métabolisme du fer perturbé;
c) les maladies associées avec une croissance osseuse perturbée
d) les maladies associées avec des modifications dégénératives des cartilages
e) les maladies associées avec des lésions des disques et des corps vertébraux,
f) les maladies associées avec des processus de migration cellulaire dérégulés, incontrôlés.

13. Domaine liant le récepteur de néogénine selon l'une des revendications 1 et 2 ou fragment de polypeptide selon l'une des revendications 3 à 5 destiné à être utilisé comme cible pour la mise en évidence ou pour l'identification de ligands liant RGM.

14. Domaine liant le récepteur de néogénine selon l'une des revendications 1 et 2 ou fragment de polypeptide selon l'une des revendications 3 à 5 destiné à être utilisé comme immunogène pour l'immunisation active ou passive.

15. Antisérum polyclonal pouvant être obtenu par immunisation d'un mammifère avec une quantité antigène d'un domaine liant le récepteur de néogénine selon l'une des revendications 1 et 2 ou d'un fragment de polypeptide selon l'une des revendications 3 à 5.

16. Anticorps monoclonal contre un domaine liant le récepteur de néogénine selon l'une des revendications 1 et 2 ou contre un fragment de polypeptide selon l'une des revendications 3 à 5, ou fragment de liaison d'antigène de celui-ci, éventuellement sous forme humanisée.

17. Agent pharmaceutique comprenant, dans un vecteur pharmaceutiquement acceptable, au moins un constituant actif choisi parmi :
a) un domaine liant le récepteur de néogénine selon l'une des revendications 1 et 2 ou un fragment de polypeptide selon l'une des revendications 3 à 5,
b) des anticorps monoclonaux ou polyclonaux selon l'une des revendications 15 et 16.

18. Agent pharmaceutique selon la revendication 17, pour l'administration intrathécale, intraveineuse, sous-cutanée, orale ou parentérale, nasale et par inhalation.

19. Vecteur d'expression comprenant au moins une séquence d'acide nucléique codante pour un domaine liant le récepteur de néogénine selon l'une des revendications 1 et 2 ou un fragment de polypeptide selon l'une des revendications 3 à 5, liée de manière fonctionnelle à au moins une séquence d'acide nucléique régulatrice.

20. Microorganisme recombinant qui porte au moins un vecteur selon la revendication 19.

21. Lignée cellulaire d'hybridome qui produit un anticorps monoclonal selon la revendication 16.

22. Procédé pour produire un domaine liant le récepteur de néogénine selon l'une des revendications 1 et 2 ou un fragment de polypeptide selon l'une des revendications 3 à 5, où on cultive un microorganisme recombinant selon la revendication 20 et on isole de la culture le produit protéique produit.

23. Procédé pour produire un anticorps monoclonal selon la revendication 16, où on cultive une lignée cellulaire d'hybridome selon la revendication 21 et on isole de la culture le produit protéique produit.
